# EUROPEAN PATENT APPLICATION

(11) **EP 4 636 093 A1**
(43) Date of publication of application: **22.10.2025**
(21) Application number: 24171327.0
(22) Date of filing: 19.04.2024
(51) Int. Cl.: C12N 15/86, C12N 5/0783

(54) **TARGETING MAPK-ERK PATHWAY FOR ENHANCING NUCLEIC ACID DELIVERY**

(71) Applicant: Paul-Ehrlich-Institut, Bundesinstitut für Impfstoffe und biomedizinische Arzneimittel, 63225 Langen (DE)
(72) Inventor: ELHAM, Adabi, 61350 Bad Homburg (DE); BUCHHOLZ, Christian, 60594 Frankfurt am Main (DE)
(74) Representative: Zwicker, Jörk

(57) **Abstract**

The present invention relates to a MAPK-ERK pathway inhibitor for enhancing functional delivery of a nucleic acid into a mammalian cell. The invention further relates to a method of delivering a nucleic acid into a mammalian cell. The invention also relates to a MAPK-ERK pathway inhibitor for use in enhancing the functional delivery of a nucleic acid or a vector comprising the nucleic acid to a subject, in particular for treating or preventing a cancer disease, a genetic disease or a viral infection.

## Description

The present invention relates to a MAPK-ERK pathway inhibitor for enhancing functional delivery of a nucleic acid into a mammalian cell. The invention further relates to a method of delivering a nucleic acid into a mammalian cell. The invention also relates to a MAPK-ERK pathway inhibitor for use in enhancing the functional delivery of a nucleic acid or a vector comprising the nucleic acid to a subject, in particular for treating or preventing a cancer disease, a genetic disease or viral infection.

### BACKGROUND OF THE INVENTION

Mammalian cells, including human cells can be difficult to transduce with a vector comprising a nucleic acid of interest. Immune cells such as T cells, B cells, plasmablasts, Natural Killer (NK) cells and Natural Killer T (NKT) cells, macrophages and hematopoietic stem cells offer numerous therapeutic benefits, particularly in the field of adoptive cell therapy and genetic manipulation. Gene manipulation is an engineering technology that allows to transfer, i.e. transduce, a vector comprising a transgene into a target cell. Adoptive cell therapy, also known as cellular immunotherapy, is a form of treatment that uses immune cells to eliminate cancer. Unfortunately, immune cells are difficult to transduce, making the genetic manipulation of these cells for therapeutic benefit costly and time consuming.

A prominent example of cellular immunotherapy uses T cells engineered to express a specific transgenic T cell receptor (TCR) or a chimeric antigen receptor (CAR) on their surface, and have shown significant efficacy against some hematologic malignancies. CAR T cell therapy has shown promising results in clinical trials, and several CAR T cell products have been approved for use in certain cancers, most notably B-cell lymphoma, B-cell acute lymphoblastic leukemia, and multiple myeloma (Cappell, KM, and Kochenderfer, JN, 2023. Long-term outcomes following CAR T cell therapy: what we know so far. Nature Reviews Clinical Oncology, 20(6), 359-371; Shafer, P. et al. 2022. Cancer Therapy With TCR-engineered T Cells: Current Strategies, Challenges, and Prospects. Front Immunol,13:835762)*.* However, the therapies with TCR T cells and CAR T cells are relatively new and still need to be improved, particularly in terms of efficacy, side effects such as cytokine release syndrome, procedure length and cost. Lentiviral vectors (LVs) are the most widely used technology in CAR T cell generation due to their ability to efficiently deliver nucleic acids into T cells.

The generation of TCR T cells and of CAR T cells depends on the successful, in most cases stable transduction of T cells with nucleic acids. Stable T cell transduction is usually performed with viral vectors or with non-viral gene editing methods (Foy, S, et al., 2022. Non-viral precision T cell receptor replacement for personalized cell therapy. Nature, 615, 687-696). Viral transduction, in particular with lentiviral vectors (LVs) or with adeno-associated viral vectors (AAV) often involves a complex multi-step process initiated by receptor binding of viral vectors, in particular of LVs, followed by endocytosis for VSV-LV and fusion at the cell membrane for targeted vectors (Frank, AM, and Buchholz, CJ 2019. Surface-Engineered Lentiviral Vectors for Selective Gene Transfer into Subtypes of Lymphocytes. Molecular Therapy - Methods & Clinical Development, 12, 19-31). Inside the target cells, the transduction process further involves uncoating, evasion of cell restriction factors, transcription of the viral RNA by the reverse transcriptase to produce double-stranded DNA (dsDNA), delivery of the dsDNA to the nucleus, and integration of the transgene into the host genome via the lentiviral integrase enzymes.

Transgenic TCR T cell and of CAR T cell therapy often use T cells isolated from the patient to be treated, i.e. autologous T cells and, thus the number of T cells available for genetic modification is often limited. Usually only a small fraction of the T cells in the treated sample is genetically modified and expresses the CAR on its surface. Using current experimental protocols, only 30-40% of T cells are successfully modified into CAR T cells by viral transduction, leaving a large proportion of cells unmodified. Generating enough CAR T cells to reach the recommended dose is important for the optimal efficacy of therapy. Current strategies aimed to increase the percentage of CAR T cells in the final product involve *ex vivo* expansion (Levine et al., 2017. Global Manufacturing of CART Cell Therapy. Molecular Therapy - Methods & Clinical Development, 4, 92-101). However, prolonged culture times or excessive expansion can lead to T cell exhaustion or loss of functionality, potentially reducing efficacy (Stock et al., 2019. Optimizing Manufacturing Protocols of Chimeric Antigen Receptor T Cells for Improved Anticancer Immunotherapy. International Journal of Molecular Sciences, 20(24), 6223). Improving the delivery efficiency of nucleic acids with viral vectors will allow to reduce the therapeutic efficient vector dose and thus therapy costs.

A further adoptive cancer immunotherapy strategy uses genetically modified NK cells that have been redirected to tumor targets via the introduction of either activating or chimeric antigen receptors (Pegram HJ, et al., 2009. Genetic modification of natural killer cells for adoptive cellular immunotherapy. Immunotherapy, 1(4):623-30). Other approaches involve the introduction of genes expressing cytokines for enhancement of *in vivo* survival and cytotoxicity of adoptively transferred NK cells. In the last decade several clinical trials have started to test genetically modified NK cells for adoptive cancer immunotherapy. However, efficient gene modification of NK cells has been largely hampered by the absence of an efficient gene-transfer protocol. (Alici, et al., 2009. Retroviral gene transfer into primary human natural killer cells. Methods Mol Biol. 506:127-137), and novel methods are needed in order to improve genetic modification of NK cells and manufacture more efficient NK cell therapy products.

B cells can be engineered to secrete specific monoclonal antibodies with therapeutic benefit. Macrophages have recently emerged as prominent candidates for cancer cellular immunotherapy (Moyes, KW, et al., 2017. Genetically Engineered Macrophages: A Potential Platform for Cancer Immunotherapy. Hum Gene Ther. 28: 2, 200-215). Recent approaches have used genetic engineering to design macrophages ("genetically engineered macrophages" (GEMs)) that express proinflammatory transgenes of interest or chimeric antigen receptors.

Hematopoietic stem cells (HSCs) are an attractive target for cancer and leukemia immunotherapy because they can be genetically engineered to express TCRs or CARs directed against tumor-associated antigens. HSCs could provide a continuous source of targeted anti-cancer effector cells to sustain remissions. In addition, CAR- or TCR-engineered HSCs may produce not only T cell, but also myeloid and natural killer cells expressing the CAR or the TCR, providing broader and faster anti-tumor activity after transplantation, which does not rely solely on *de novo* thymopoiesis. A further advantage is that T cells generated *de novo* from engineered HSCs may continuously replenish anergic or exhausted anti-tumor T cells in the intratumoral microenvironment.

Unfortunately, the current protocols used for genetic engineering of immune cells have low transduction efficiencies, making the genetic engineering of these cells for therapeutic benefit costly and time consuming. Indeed, the generation of large quantities of cells for adoptive cellular immunotherapy remains a challenge, and the number of cells that can be administered to a patient for adoptive cell transfer is generally limited. Therefore, there is a need for novel methods and compounds to enhance the functional delivery of nucleic acids into mammalian target cells and thus improve efficiency of genetic engineering of said cells.

### SUMMARY OF THE INVENTION

The present inventors have discovered a negative role of the MAPK-ERK pathway in transduction and that functional delivery of a nucleic acid into a mammalian cell can be enhanced by using inhibitors of the MAPK-ERK pathway. Mammalian cells incubated with various MAPK-ERK pathway inhibitors showed both enhanced endocytosis of the vector comprising the nucleic acid and enhanced expression of the protein encoded by the vector. These findings suggest that the MAPK-ERK pathway inhibitors could affect different steps of the transduction process, such as the endocytosis of the vector, the efficient and functional delivery of the nucleic acid to the nucleus, and/or the expression of the encoded protein or polypeptide. Thus, the present invention provides MAPK-ERK pathway inhibitors suitable to improve genetic engineering of mammalian cells, and methods to improve genetic engineering of mammalian cells using said inhibitors *in vitro*, *ex vivo* or *in vivo.* The disclosed MAPK-ERK pathway inhibitors can thus be useful to improve genetic engineering and cellular immunotherapy and overcome the limitations of the current strategies.

Accordingly, in a first aspect the invention provides the use of a MAPK-ERK pathway inhibitor for enhancing functional delivery of a nucleic acid into a mammalian cell.

In a second aspect, the invention provides a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor.

In a third aspect, the invention provides the use of a MAPK-ERK pathway inhibitor in combination with another type of transduction enhancer for enhancing functional delivery of a nucleic acid into a mammalian cell.

In a fourth aspect, the invention provides a method of functionally delivering a nucleic acid into a mammalian cell, comprising the steps of contacting the mammalian cell with the nucleic acid or a vector comprising the nucleic acid and contacting the mammalian cell with a MAPK-ERK pathway inhibitor, and further comprising the step of contacting the mammalian cell with another type of transduction enhancer prior to, concomitant with or subsequent to contacting of the mammalian cell with the nucleic acid or the vector comprising the nucleic acid.

In a fifth aspect, the invention provides a MAPK-ERK pathway inhibitor for use in enhancing functional delivery of a nucleic acid to a subject.

In a sixth aspect, the invention provides MAPK-ERK pathway inhibitor for use in enhancing functional delivery of a nucleic acid to a subject, wherein the nucleic acid or a vector comprising the nucleic acid is administered to the subject to treat or prevent a cancer disease, a genetic disease, or a viral infection.

### Brief description of the drawings

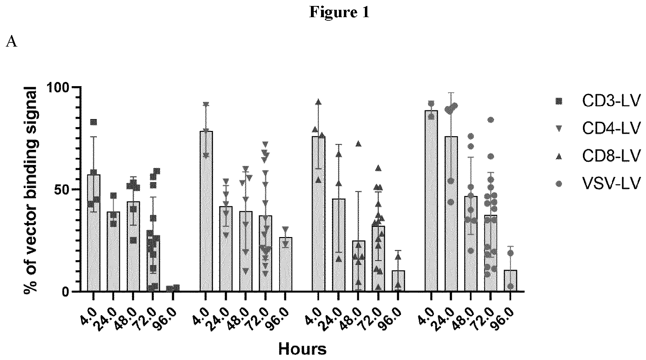

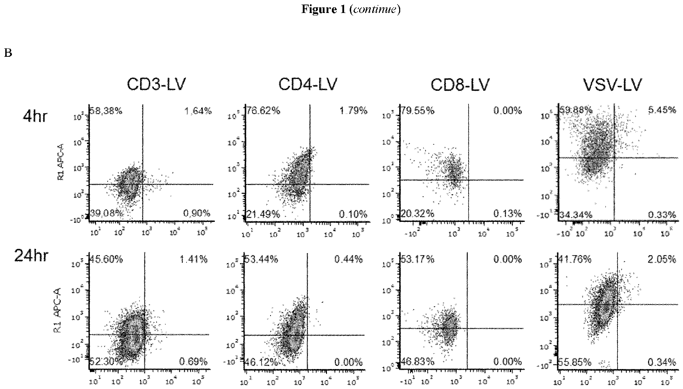

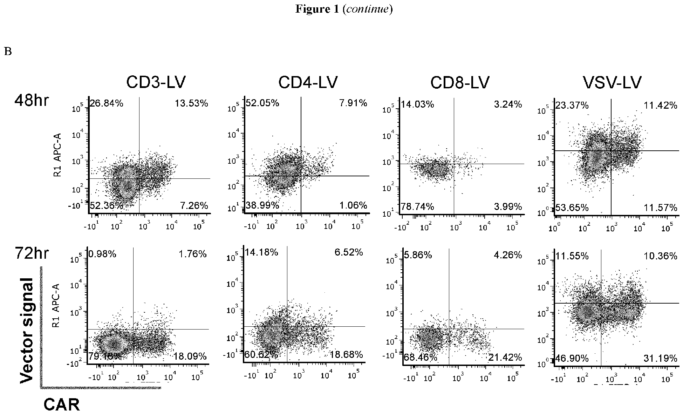

**Figure 1**: Vector binding and CAR expression kinetics. (A) Percentage of vector^{pos} T cells at different time points. Vector signal detected on CD3 T cells (for CD3-LV and VSV-LV), CD4 T cells (for CD4-LV), and CD8 T cells (for CD8-LV). For each time point, at least 2 different donors were measured. Each dot in each group represents biological or technical replicates. (B) Flow cytometry plots of stained T cells at different time points after adding VSV-G or targeted CD3-LV, CD4-LV, and CD8-LV vectors. All LVs had Anti-CD19 CAR transgene with a Myc-tag sequence. A reporter protein (VSV-G or His tag) was on the vector's envelope.

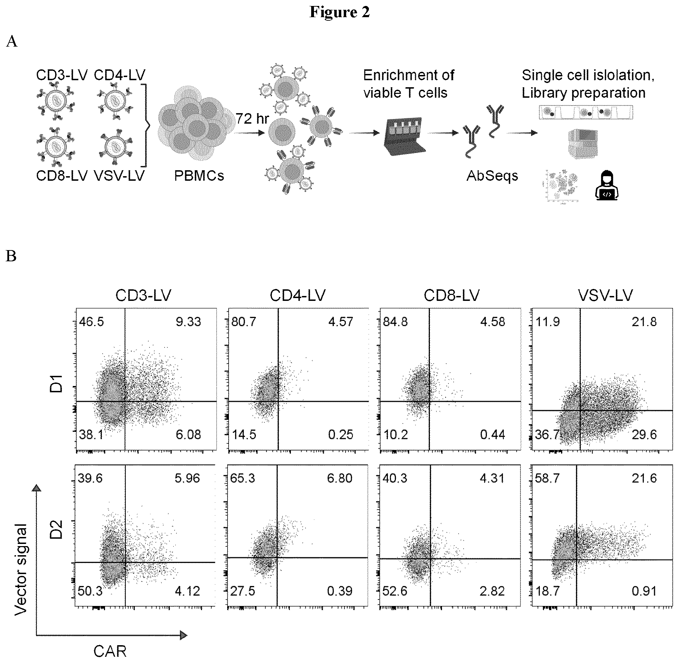

**Figure 2**: (A) Experimental layout for single-cell multi-omics analysis. Activated PBMCs from two healthy donors were incubated with different lentiviral vectors and 72 hours later T cells were enriched for viability and desired T cell subtypes. Prior to single-cell isolation, the cells were stained with different AbSeqs against CAR, vector envelope and 30 T cell immune markers as well as sample multiplexing tags. The mRNA and barcodes associated with the AbSeqs, and multiplexing tags were captured by magnetic beads in single-cell isolation wells, and the libraries generated were sequenced after reverse transcription. (B) PBMCs isolated from two healthy donors were transduced with CD3-LV, CD4-LV, CD8-LV, and VSV-LV, and CAR expression (x-axis) and vector binding (y-axis) were measured 72 hours later. Anti-His antibody was used to detect targeted vectors binding and anti-VSV-G antibody was used to detect VSV-G vector binding. Viable CD3 T cells for CD3-LV and VSV-LV, CD4 T cells for CD4-LV and CD8 T cells for CD8-LV were plotted. FACS plots show the heterogeneous T cell population with differences in vector binding and CAR expression between different vectors and donors. In all conditions, the T cell population with vector binding signal and no CAR expression was observed. D1: donor 1, D2: donor 2.

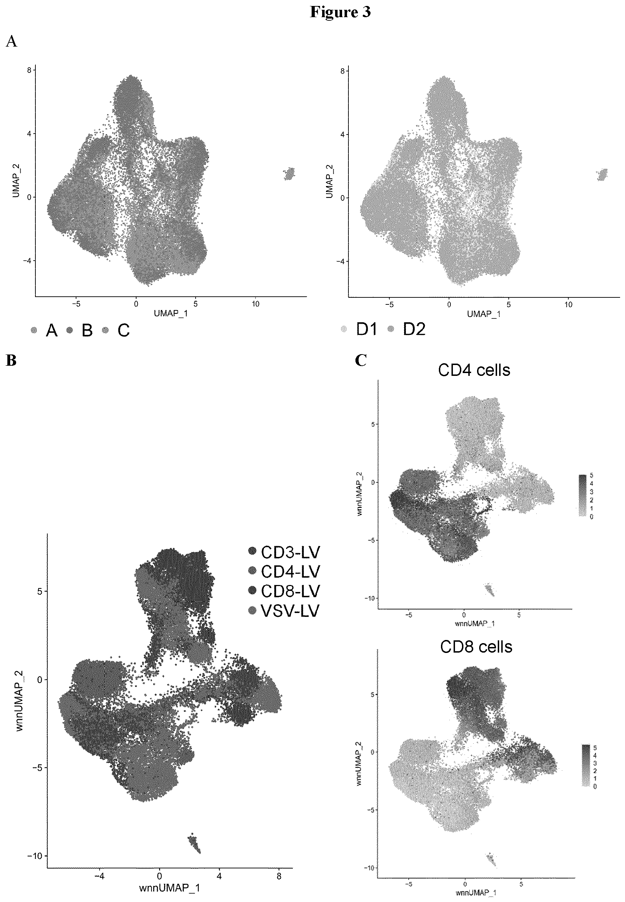

**Figure 3**: Sample integration and UMAP plots. (A) Integrated samples visualized based on cartridge (left) or donor (right). (B) UMAP plots showing the distribution of samples across different clusters. CD4 (left) and CD8 (right) positive clusters detected by AbSeq.

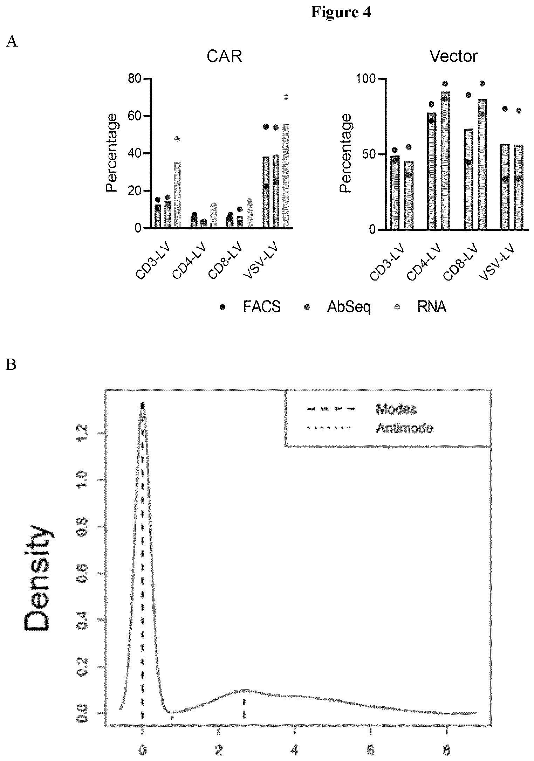

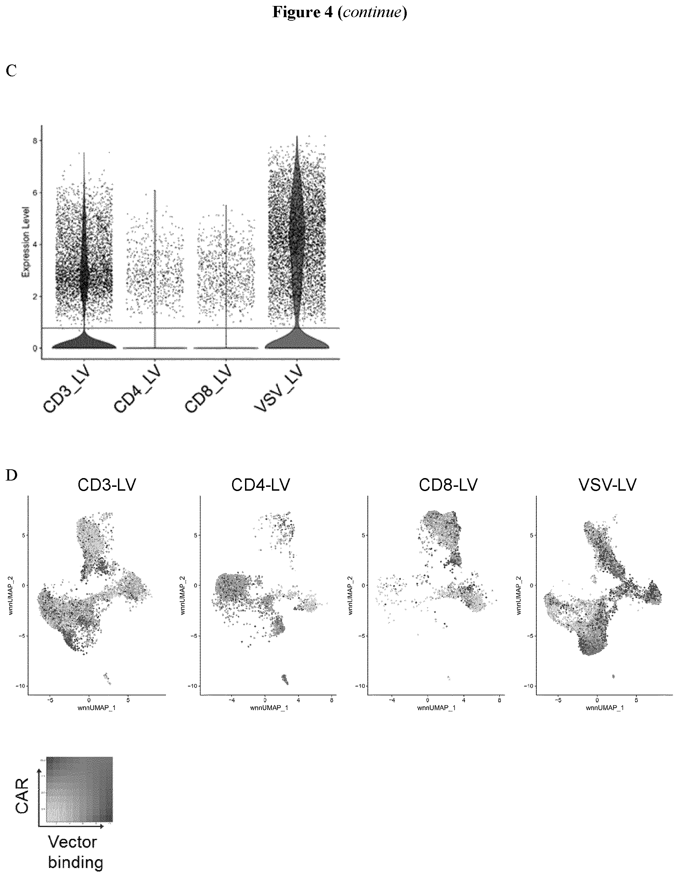

**Figure 4**: Single-cell multi-omics analysis. (A) Percentages of CAR-positive cells determined on protein levels by FACs and Abseq as well as on RNA level (left). Percentages vector-bound cells were determined by flow cytometry or AbSeq (right). Data are from two donors (each dot represents one donor). (B) Multimodal analysis and (C) violin plots for setting expression thresholds to subset *CAR*^{neg} and *CAR*^{pos} cells based on CAR mRNA detection. The horizontal line in the violin plot indicates the threshold. (D) Blended UMAP plots showing vector-bound cells (red) and CARp-positive cells (purple) and their overlays (pink).

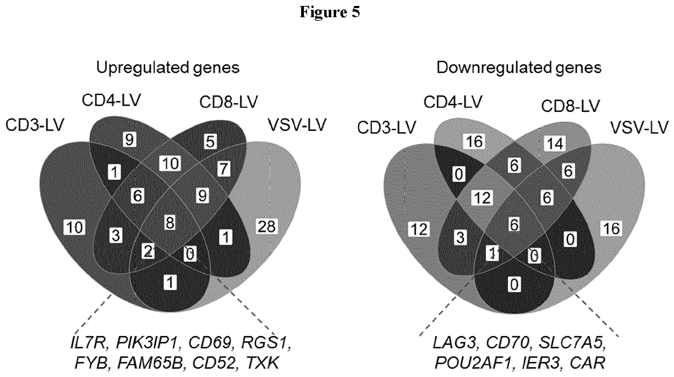

**Figure 5**: Overlaps of differentially expressed genes between *CAR*^{pos}/CARp^{neg} and *CAR*^{pos}/CARp^{pos} cells from different LVs. Venn plots showing the overlaps of DE genes (|log2FC|>0.25, FDR<0.05) identified by comparing the *CAR*^{pos}/CARp^{neg} to the *CAR*^{pos}/CAR^{pos} cells among all vector types. Venn plot of upregulated gene (left) and downregulated genes (right). The common genes from each group are listed.

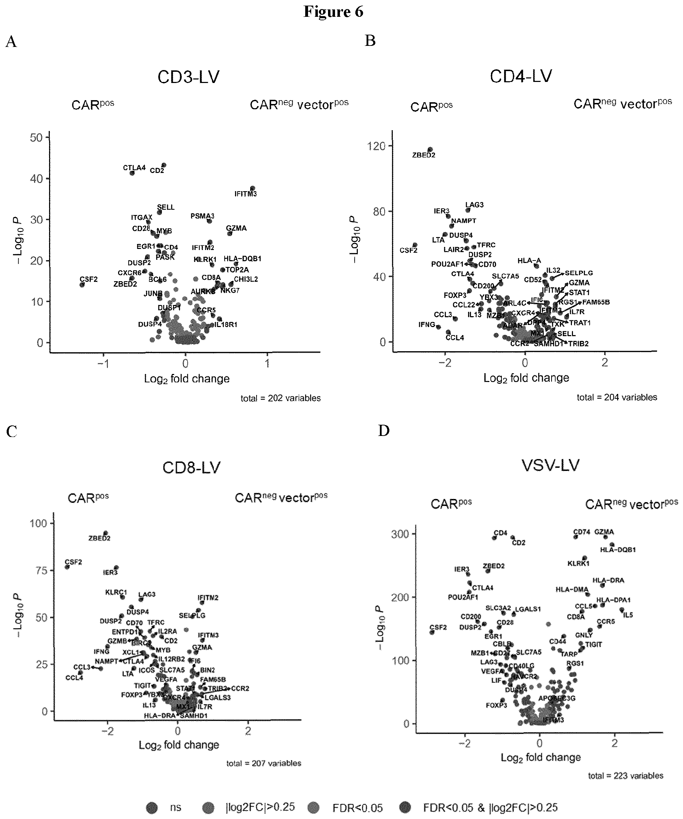

**Figure 6**: Differential gene expression analysis between *CAR*^{pos} and *CAR*^{neg} cells. Volcano plots of DEGs between *CAR*^{pos} T cells and *CAR*^{neg}/vector^{pos} T cells in the (A) CD3-LV, (B) CD4-LV, (C) CD8-LV, and (D) VSV-LV treated groups. Values for *CAR* were excluded from the diagrams for better visualization. Negative fold-change indicates genes upregulated in *CAR*^{pos} T cells. Positive fold-changes indicates genes upregulated in *CAR*^{neg}/vector^{pos} T cells. n.s: non-significant. FDR: false discovery rate.

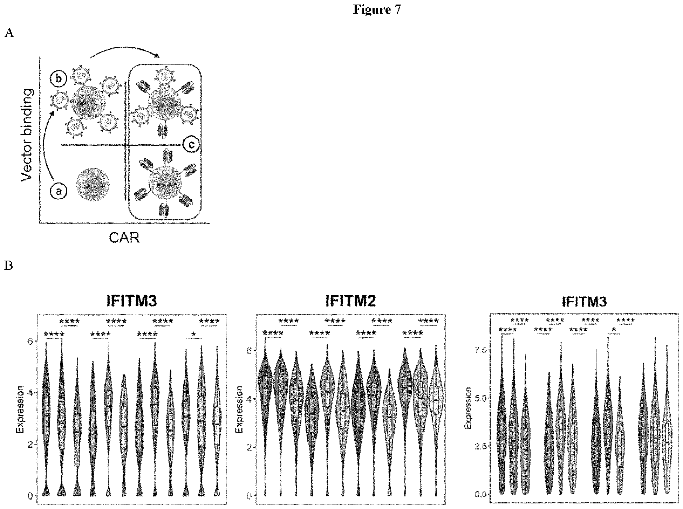

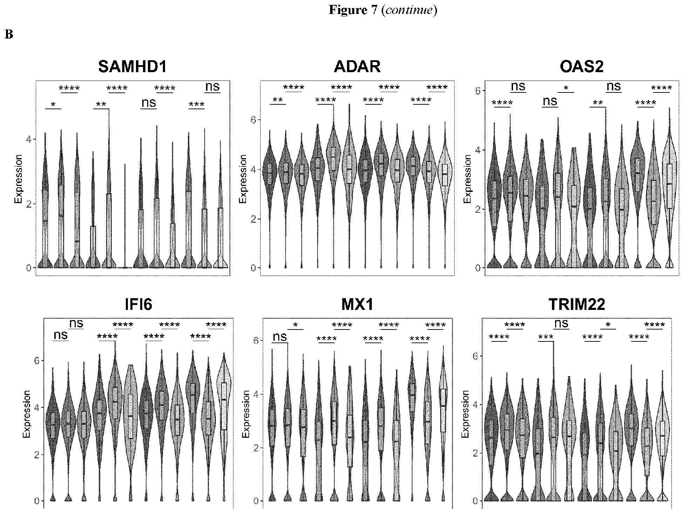

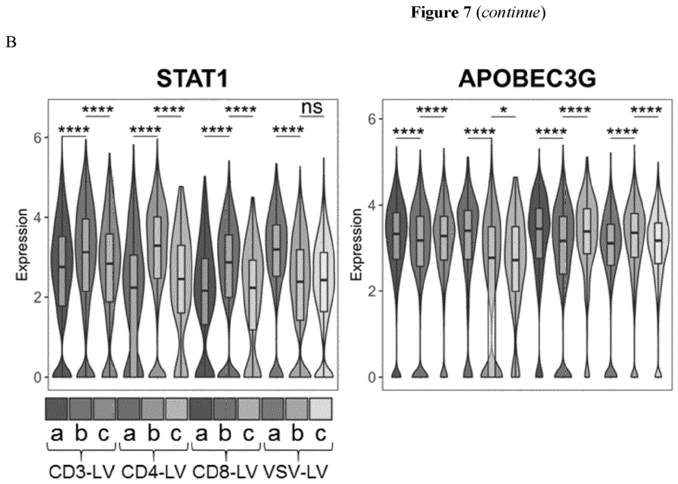

**Figure 7**: Expression pattern of interferon-stimulated genes among different T cell subsets. (A) T cells during CAR T cell generation were classified into three distinct subsets depending on the presence of bound vector and CARp. a: CARp^{neg}/vector^{neg}, b: CARp^{neg} /vector^{pos}, and c: CAR^{pos} T cells. (B) Violin plots showing the expression of interferon-stimulated genes from all vector types across the three defined cell subsets. Statistical significant differences between subsets are highlighted by arrows when samples followed the identified pattern. Dotted line arrows indicate statistically non-significant tendencies following the pattern. Statistics show FDR-corrected values from differential expression analysis (Wilcoxon rank sum test). FDR: false discovery rate, ns: non-significant, *p<0.05, **p<0.01, ***p<0.001, ****p<0.0001.

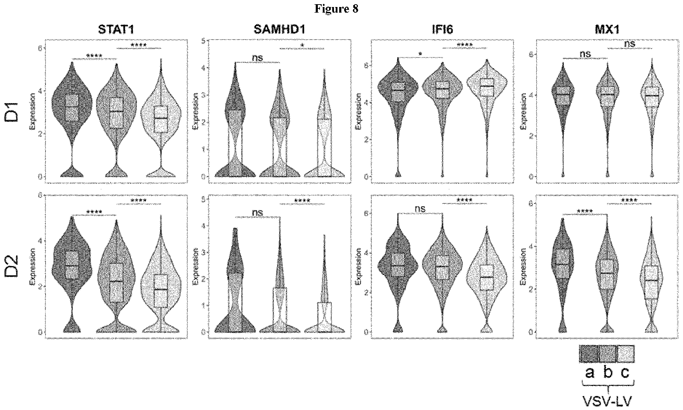

**Figure 8**: Gene expression patterns in VSV-LV treated T cells from each donor. Violin plots show the expression level of some differentially expressed genes from each donor (donor 1: top row and donor 2: bottom row) separately in the VSV-LV group in three different subsets of cells, a: CARp^{neg}/vector^{neg} T cells, b: CARp^{neg}/vector^{pos} T cells, and c: CARp^{pos} T cells. ns: non-significant, *p<0.05, ****p<0.0001.

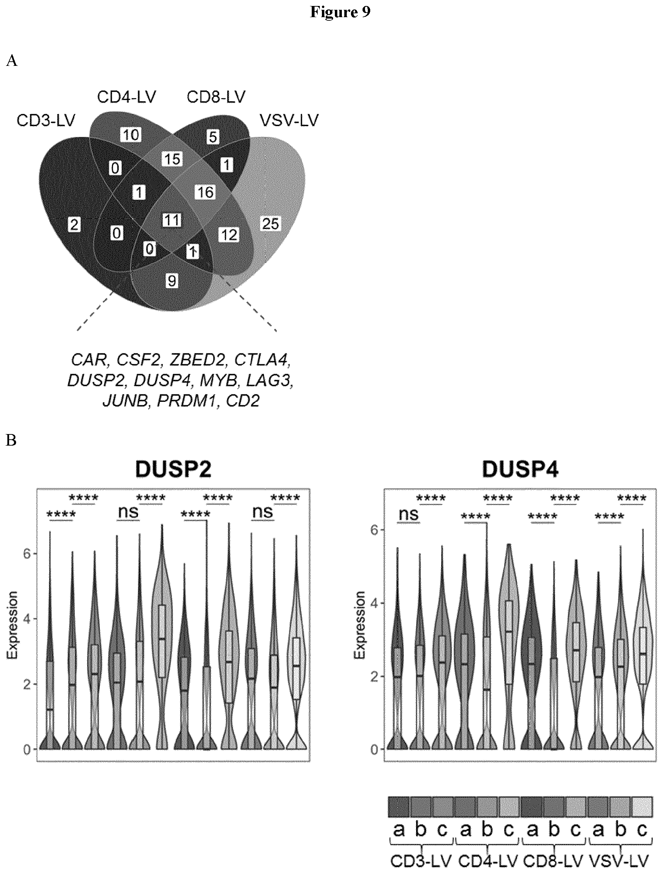

**Figure 9**: Genes upregulated in CAR^{pos} T cells. (A) Venn plots showing the overlaps of upregulated genes (log2FC>0.25, FDR<0.05) identified in *CAR*^{pos} T cells compared to *CAR*^{neg}/vector^{pos} T cells from four different vector types. The 11 common genes are listed. (B) Violin plots showing the expression of *DUSP2* and *DUSP4* in different cell subsets from each of the four vectors, a: CARp^{neg}/vector^{neg}, b: CARp^{neg}/vector^{pos}, and c: CARp^{pos} T cells. Statistics show FDR-corrected values from differential expression analysis (Wilcoxon rank sum test). ns: non-significant, ****p<0.0001.

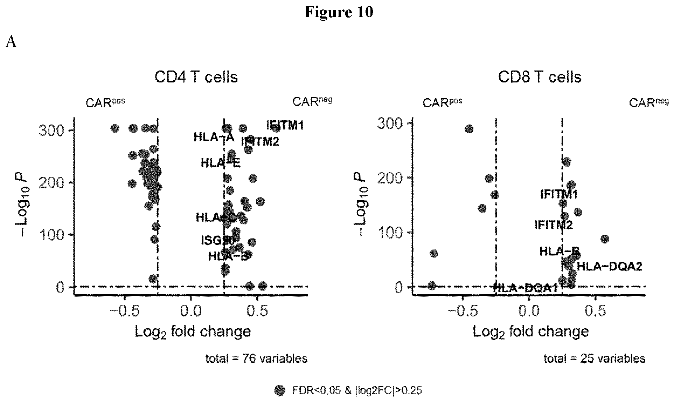

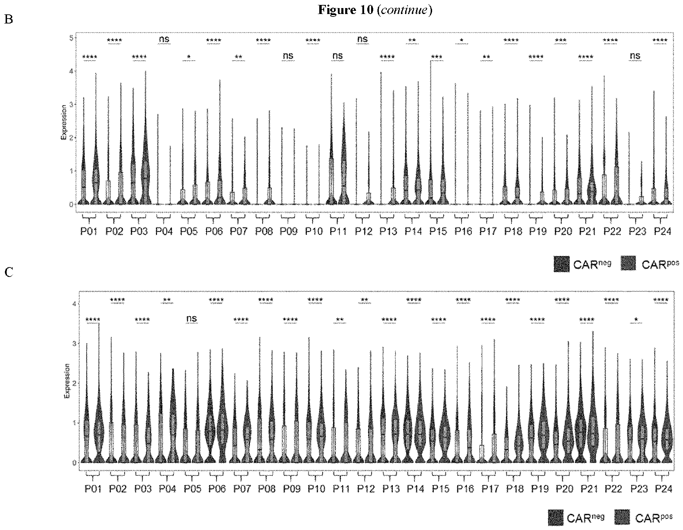

**Figure 10**: Differentially expressed genes between *CAR*^{pos} and *CAR*^{neg} cells of patient-derived CAR T cell products. (A) Volcano plots comparing CAR^{pos} T cells to CAR^{neg} T cells among 24 patients. Values for CAR were excluded from the diagrams for better visualization. Differentially expressed genes between CAR^{pos} and CAR^{neg} in CD4 (left) and CD8 (right) T cells. On the left side of volcano plots: genes upregulated in CAR^{pos} T cells, right side: genes upregulated in CAR^{neg} T cells. FDR: false discovery rate. (B) Violin plots showing the expression of the *DUSP2* gene in CAR^{pos} T cells (green) and comparison to CAR^{neg} T cells (red) for each patient separately. ns: non-significant, *p<0.05, **p<0.01, ***p<0.001, ****p<0.0001. (C) Violin plots show the expression of the *DUSP4* genes in CAR^{pos} T cells (green) and comparison to CAR^{neg} T cells (red) for each patient separately. ns: non-significant, *p<0.05, **p<0.01, ***p<0.001, ****p<0.0001.

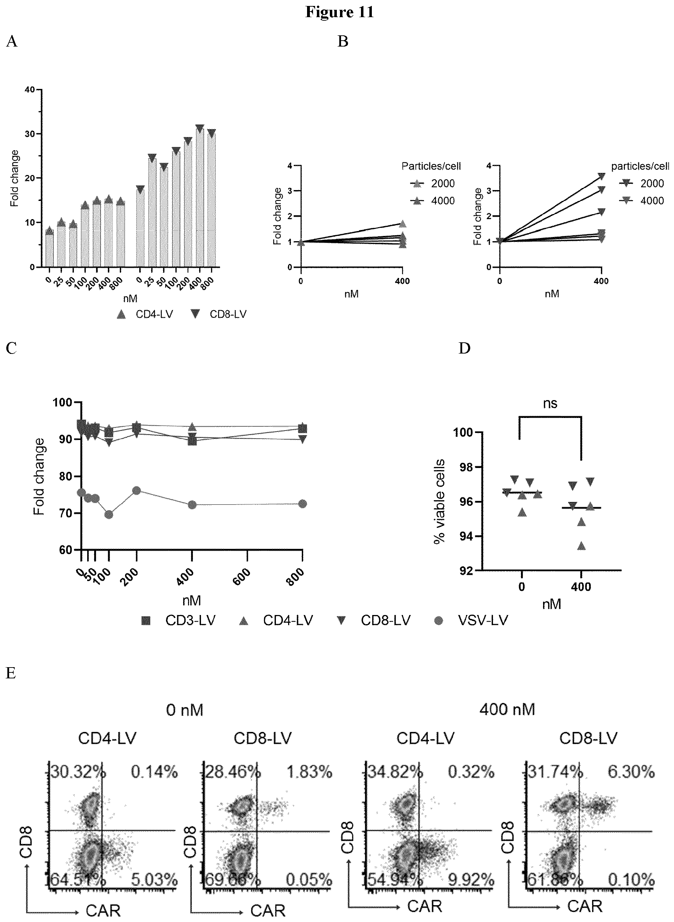

**Figure 11**: Deucravacitinib enhances T cell transduction. (A) Percentages of CAR expression upon transduction with different LVs, in the presence of different doses of deucravacitinib (one donor). Depending on the vector type, CAR expression was measured either on CD4 T cells (CD4-LV) or CD8 T cells (CD8-LV). (B) Fold change differences in CAR T cell percentages after transduction with CD4-LV and CD8-LV (three donors; each dot represents the average of two technical replicates) with or without 400 nM deucravacitinib. (C) Viability of T cells after treatment with different doses of deucravacitinib (one donor). (D) Activated PBMCs were transduced with CD4-LV and CD8-LV with or without 400nM deucravacitinib and viability of T cells were measured 3 days after transduction (three donors, each dot is the mean of two technical replicates). Statistical analysis was performed by unpaired t-test. ns: non-significant. (E) Representative FACS plots for one donor after transduction with CD4-LV and CD8-LV with or without 400nM deucravacitinib.

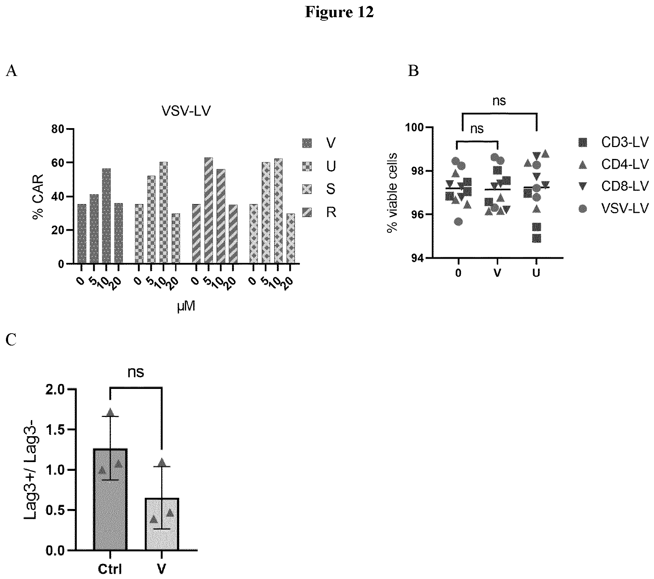

**Figure 12**: Effect of ERK inhibitors on CAR expression, viability, and T cell exhaustion. (A) Testing different doses of ERK inhibitors on T cells that were transduced with similar VSV-LV dose (one donor). The CAR expression level was measured on CD3 T cells. V: VX-11e, U: ulixertinib, R: ravoxertinib, S: selumetinib. (B) Viability of T cells transduced with CD3-LV, CD4-LV, CD8-LV and VSV-LV in the presence or absence of 5 µM ulixertinib or 20µM VX-11e (three donor, two technical replicates). (C) Ratio of CD4+CAR+LAG3+ to CD4+CAR+LAG3- cells frequencies generated in the presence or absence of 20µM VX-11e n with CD4-LV (three donors; each dot is the mean of two technical replicates). Statistical analysis was performed by unpaired t-test. ns: non-significant.

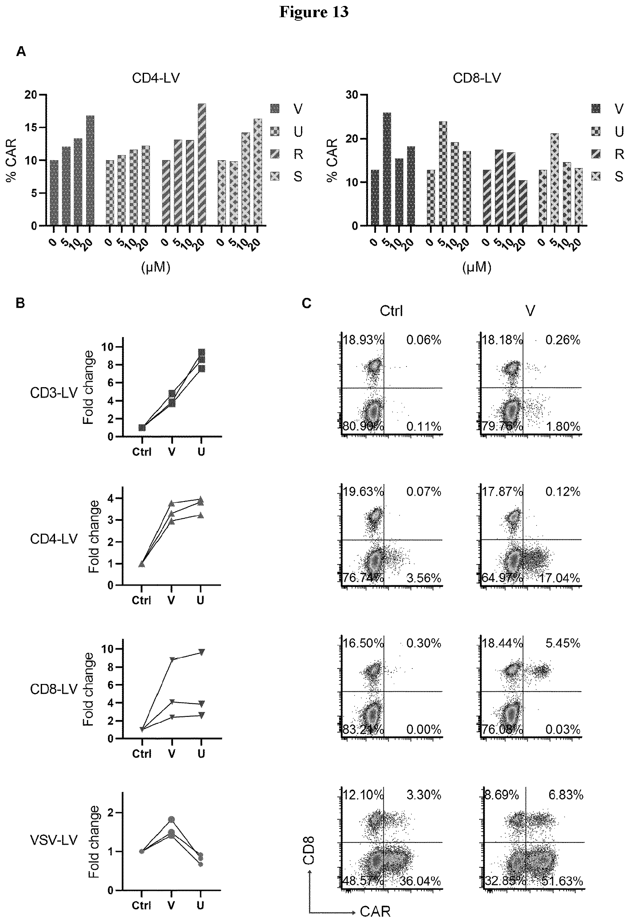

**Figure 13**: Inhibition of ERK2 promotes T cell transduction with LVs. (A) Titration of four distinct ERK inhibitors on T cells that are transduced with similar dosages of CD4-LV (left) or CD8-LV (right) (one donor). The level of CAR expression was measured in CD4 or CD8 T cells for treated groups of either CD4-LV or CD8-LV. (B) Fold change differences in CAR T cell percentages after transduction with various vectors (three donors; each dot represents the average of two technical replicates) with or without 5 µM ulixertinib or 20 µM VX-11 e. (C) Representative flow cytometry results of CAR T cells generated using CD3-LV, CD4-LV, CD8-LV, or VSV-LV, with or without 5 µM of ulixertinib for targeted vectors or 20 µM of VX-11e for VSV-LV. V: VX-11e, U: ulixertinib, R: ravoxertinib, S: selumetinib.

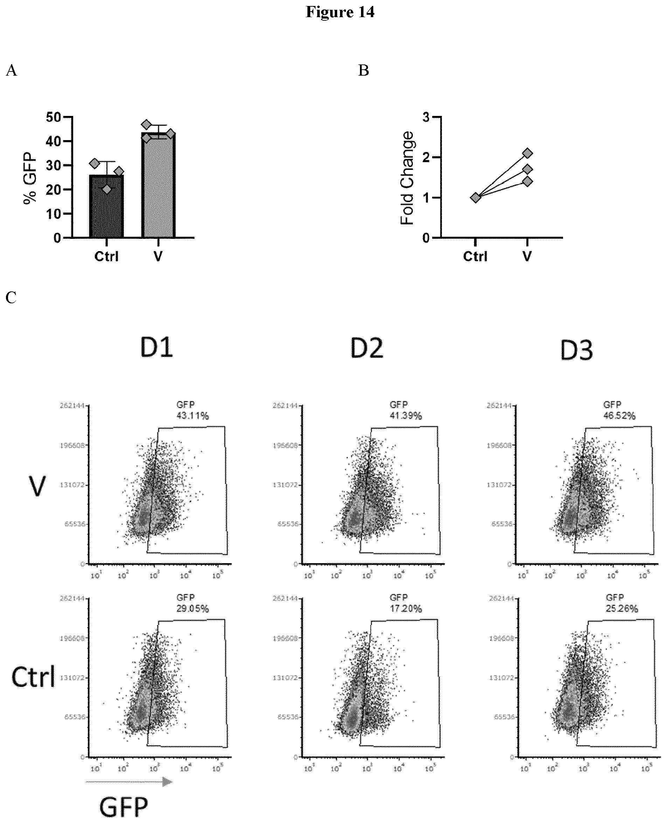

**Figure 14**: Enhanced AAV6-mediated transduction of human T lymphocytes by Vx-11e. (A) Percentages of GFP expression upon transduction with AAV6, in the presence (V) or absence (Ctrl) of 20 µM Vx-11e. (three different donors, two technical replicates) (B) Fold change differences in percentages of GFP-positive cells after transduction with AAV6 (three donors, two technical replicates) in the presence (V) or absence (Ctrl) of Vx-11e 20 µM. (C) Representative flow cytometry results of GFP expression in T cells from three different donors in the presence (V) or absence (Ctrl) of 20 µM Vx-11e. Ctrl: control, V: Vx-11e D: donor.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, HGW, Nagel, B and Klbl, H eds. (1995), Helvetica Chimica Acta, CH-4010 Basel, Switzerland) and as described in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmacopeial Convention, Inc., Rockville Md., 2001.

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, Sambrook Jet al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated feature, integer or step or group of features, integers or steps but not the exclusion of any other feature, integer or step or group of integers or steps.

As used in this specification and the appended claims, the singular forms "a", "an", and "the" include plural referents, unless the content clearly dictates otherwise. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the disclosure and does not pose a limitation on the scope of the claims. No language in the specification should be construed as indicating any non-claimed element essential to the practice of the disclosure.

In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

Several documents are cited throughout the text of this specification. Each of the documents cited herein (including all patents, patent applications, scientific publications, manufacturer's specifications, instructions, etc.), whether *supra* or *infra*, are hereby incorporated by reference in their entirety. Nothing herein is to be construed as an admission that the invention is not entitled to antedate such disclosure by virtue of prior invention.

### Aspects of the invention and particular embodiments thereof

The invention relates to several aspects as set out above in the summary of the invention. These aspects comprise alternative embodiments and preferred embodiments, which are described below.

### MAPK-ERK pathway inhibitors

The MAPK-ERK signaling pathway, also known as the RAS/RAF/MEK/ERK signaling pathway or cytoplasmic cascade, mediates cellular responses to growth signals, linking cell membrane receptor activity to cell proliferation, differentiation and survival through the regulation of cytoplasmic or nuclear targets. Activation of RAS GTPase proteins in response to growth factors, hormones, cytokines, etc. stimulates phosphorylation and activation of RAF kinases, which then phosphorylate and activate the intracellular protein kinases MEK1 and MEK2 (MAP/ERK kinases 1 and 2), which in turn phosphorylate and activate other protein kinases, ERK1 and ERK2 (extracellular signal-regulated kinases 1 and 2). The RAS family of GTPase proteins transduce signals from activated growth factor receptors to downstream intracellular targets. Prominent intracellular downstream effectors of RAS are the serine/threonine protein kinases of the RAF family, which consists of three related kinases (A-, B- and C- RAF). RAF-mediated activation of MEK1 and MEK2 in turn phosphorylates ERK1 and ERK2 at tyrosine 185 and threonine 183. MEK1 and MEK2 are members of a larger family of dual-specificity kinases (MEK1-7) that phosphorylate threonine and tyrosine residues of different MAP kinases. Activated ERK1 and ERK2 translocate and accumulate in the nucleus where they can phosphorylate and activate various substrates, including transcription factors, to control cell growth and survival. The MAPK-ERK signaling pathway is reviewed, for example in Akinleye *et al.*, 2013 (Akinleye et al., 2013. MEK and the inhibitors: from bench to bedside. JHematol Oncol. 12; 6:27).

The inventors have surprisingly found that MAPK-ERK pathway inhibitors, such as ERK1/2 inhibitors and MEK1/MEK2 inhibitors, can enhance delivery of a nucleic acid encoding a chimeric antigen receptor (CAR) to T cells. Importantly, the use of MAPK-ERK pathway inhibitors allowed for increased transduction with all types of viruses used without adversely affecting cell viability or T cell exhaustion profile.

Therefore, a first aspect the invention provides the use of a MAPK-ERK pathway inhibitor for enhancing functional delivery of a nucleic acid into a mammalian cell.

The term "MAPK-ERK pathway inhibitor" is used herein to refer to any substance that reduces the activity, expression or phosphorylation of proteins or other members of the MAPK-ERK pathway. A preferred MAPK-ERK pathway inhibitor is a direct inhibitor of the MAPK-ERK pathway. A direct inhibitor of the MAPK-ERK pathway is an inhibitor that specifically acts on a protein that is part of the MAPK-ERK pathway. Preferably the inhibitor (i) non-covalently or covalently binds to a protein, e.g. to RAF, A-RAF, B-RAF, C-RAF, ERK1, ERK2 MEK1, or MEK2, that is part of the MAPK-ERK pathway and inhibits its activity, e.g. by preventing an activating phosphorylation or by inhibiting its kinase activity or (ii) specifically inhibits the transcription or expression of a protein that is part of the MAPK-ERK pathway, e.g. with an siRNA. In contrast an indirect inhibitor of the MAPK-ERK pathway may act on a protein further up-or downstream of a protein that is part of the MAPK-ERK pathway.

The terms "nucleic acid" and "polynucleotide" are used interchangeably herein and are understood as a polymeric or oligomeric macromolecule made from nucleotide monomers. Nucleotide monomers are composed of a nucleobase, a five-carbon sugar (such as but not limited to ribose or 2'-deoxyribose), and one to three phosphate groups. Typically, a polynucleotide is formed by phosphodiester bonds between the individual nucleotide monomers. Nucleic acids can be synthesized chemically, e.g. by the phosphotriester method (see, for example, Uhlmann, E. & Peyman, A. (1990) Chemical Reviews, 90, 543-584). Preferred nucleic acids to be used in the context of the invention are RNA or DNA.

The term "functional delivery of a nucleic acid into a mammalian cell" refers to the process of targeted introduction of foreign genetic material into a mammalian cell. In particular, the term "functional delivery of a nucleic acid into a mammalian cell" refers to the process of delivering a nucleic acid, such as a DNA or RNA molecule, into the nucleus or cytoplasm of a mammalian cell, wherein "functional" refers to the fact that the DNA or RNA molecule exerts its function in the mammalian cell, as explained in more detail in the following sections. In some embodiments, the mammalian cell is an *in vitro* cultured mammalian cell, in which case the delivery of the nucleic acid occurs *in vitro.* In some embodiments, the mammalian cell is a cell isolated from a subject, in which case the delivery of the nucleic acid occurs *ex vivo.* In some embodiments, the mammalian cell is a cell of an organ or a tissue of a subject, in which case the delivery of the nucleic acid occurs *in vivo.* In some embodiments, the term "functional delivery of a nucleic acid into a mammalian cell" comprises delivery of a nucleic acid, such as ssDNA or dsDNA, into the nucleus of a mammalian cell. In some embodiments, the term "functional delivery of a nucleic acid into a mammalian cell" comprises delivery of a nucleic acid, such as mRNA or siRNA, into the cytoplasm of a mammalian cell. In some embodiments, the term "functional delivery of a nucleic acid into a mammalian cell" comprises transduction of a mammalian cell with the nucleic acid. In some embodiments, the term "functional delivery of a nucleic acid into a mammalian cell" comprises transfection of a mammalian cell with the nucleic acid. The term "transduction" refers to the introduction of a nucleic acid into a mammalian cell by a virus or viral vector. The term "transfection" relates to the introduction of nucleic acids, in particular RNA, into a mammalian cell or to the uptake of a nucleic acid by such cell. Transfection methods are known to a person skilled in the art and comprise, but are not limited to, mechanical methods, such as microinjection, electroporation, particle bombardment, sonoporation, magnetofection, and laser irradiation, and chemical methods using nanocarriers such as cationic polymer nanoparticles, cationic liposomes, cationic lipid nanoparticles, and magnetic nanoparticles. In some embodiments, the delivered nucleic acid is integrated into the genome of the mammalian cells, meaning that the mammalian cell is stably transduced or transfected with the nucleic acid. In some embodiments, the delivered nucleic acid is not integrated into the genome of the mammalian cells, meaning that the mammalian cell is transiently transduced or transfected with the nucleic acid. Functional delivery of a nucleic acid into a mammalian cell, for example, by transduction with a viral vector can be measured on the basis of the expression of a heterologous protein encoded by the viral vector in the mammalian cell. Such methods are well known to the skilled person and are also described in the Examples of this application. They comprise the determination of the amount of mRNA encoding the heterologous protein by, e.g. RT-qPCR and single-cell mRNA quantification, or the amount of the heterologous protein expressed in the mammalian cell, e.g. by Western blotting, Elisa, multiplex protein assay, or FACS. If the nucleic acid delivered to the mammalian cell is a non-coding nucleic acid, e.g. an inhibitory RNA, the amount of the inhibitory RNA produced or the amount of the mRNA targeted by the inhibitory RNA may be determined by methods known in the art. In the latter case, functional delivery of the inhibitory RNA will decrease the amount of the mRNA targeted.

The term "enhancing functional delivery of a nucleic acid into a mammalian" cell means that the functional delivery of the nucleic acid to the nucleus or to the cytoplasm in the presence of the MAPK-ERK pathway inhibitor is higher in comparison to the delivery of the nucleic acid to the nucleus or to the cytoplasm in the absence of the MAPK-ERK pathway inhibitor. In the context of the present invention the functional delivery of a nucleic acid into a mammalian cell is enhanced, if the amount of the nucleic acid delivered to the cell is increased by at least 10%, more preferably by at least 20%, 30%, 40%, 50%, 60%, 70%, 80%, 90%, 100%, 200%, 300%, 400% or 500% or more. This aspect of enhancing functional delivery is based on the amount of nucleic acid delivered to the individual cell. The term also refers to the enhancement of the percentage of mammalian cells within a population of mammalian cells to which the nucleic acid is delivered. Enhancement of functional delivery of a nucleic acid into mammalian cells is also attained, if the percentage of mammalian cells in a given population of mammalian cells, e.g. T cells, that express a protein encoded by a transgene comprised in the nucleic acid is increased by at least 10%, more preferably by at least 20%, 30%, 40%, 50%, 60%, 70%, 80%, 90%, 200%, 300%, 400% or 500% or more in comparison to mammalian cells into which the nucleic acid has been introduced in the absence of the MAPK-ERK pathway inhibitor. In some embodiments, enhancement of functional delivery of a nucleic acid into mammalian cells is also attained, if the percentage of mammalian cells in a given population of mammalian cells, e.g. T cells, that express an mRNA encoding a transgene comprised in the nucleic acid is increased by at least 10%, more preferably by at least 20%, 30%, 40%, 50%, 60%, 70%, 80%, 90% , 200%, 300%, 400% or 500% or more in comparison to mammalian cells into which the nucleic acid has been introduced in the absence of the MAPK-ERK pathway inhibitor. In some embodiments, enhancement of functional delivery of a nucleic acid into mammalian cells is also attained, if the percentage of mammalian cells in a given population of mammalian cells, e.g. T cells, that express a protein encoded by an mRNA target of an inhibitory mRNA molecule comprised in the nucleic acid is decreased by at least 10%, more preferably by at least 20%, 30%, 40%, 50%, 60%, 70%, 80%, 90%, 200%, 300%, 400% or 500% or more in comparison to mammalian cells into which the nucleic acid has been introduced in the absence of the MAPK-ERK pathway inhibitor. In some embodiments, enhancement of functional delivery of a nucleic acid into mammalian cells is also attained, if the percentage of mammalian cells in a given population of mammalian cells, e.g. T cells, that express an mRNA target of an inhibitory mRNA molecule comprised in the nucleic acid is decreased by at least 10%, more preferably by at least 20%, 30%, 40%, 50%, 60%, 70%, 80%, 90%, 200%, 300%, 400% or 500% or more in comparison to mammalian cells into which the nucleic acid has been introduced in the absence of the MAPK-ERK pathway inhibitor.

Definitions and embodiments described below, in particular under the header 'Definitions and further embodiments of the invention' apply to all aspects of the invention, in particular to the first, second, third, fourth, fifth and sixth aspect.

In a second aspect, the invention provides a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor. In a preferred embodiment of the second aspect, the invention provides a method of delivering a nucleic acid into a mammalian cell, comprising the steps of contacting the mammalian cell with the nucleic acid and contacting the mammalian cell with a MAPK-ERK pathway inhibitor. In a preferred embodiment of the second aspect, the invention provides a method of delivering a nucleic acid into a mammalian cell, comprising the steps of contacting the mammalian cell with a vector comprising the nucleic acid and contacting the mammalian cell with a MAPK-ERK pathway inhibitor.

In one embodiment of the second aspect, the present invention relates to a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor, wherein the steps of contacting the mammalian cell with the nucleic acid or the vector comprising the nucleic acid and of contacting the mammalian cell with the MAPK-ERK pathway inhibitor are *in vitro.* In one embodiment of the second aspect, the present invention relates to a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor, wherein the steps of contacting the mammalian cell with the nucleic acid or the vector comprising the nucleic acid and of contacting the mammalian cell with the MAPK-ERK pathway inhibitor are *ex vivo.*

In one embodiment of all aspects disclosed herein, the contacting the mammalian cell with a MAPK-ERK pathway inhibitor is prior to the contacting with the nucleic acid or the vector comprising the nucleic acid. In one embodiment of all aspects disclosed herein, the contacting the mammalian cell with a MAPK-ERK pathway inhibitor is concomitant with the contacting with the nucleic acid or the vector comprising the nucleic acid. In one embodiment of all aspects disclosed herein, the contacting the mammalian cell with a MAPK-ERK pathway inhibitor is subsequent to the contacting with the nucleic acid or the vector comprising the nucleic acid. In a preferred embodiment, the contacting the mammalian cell with a MAPK-ERK pathway inhibitor is prior to the contacting with the nucleic acid or the vector comprising the nucleic acid. In a more preferred embodiment, the contacting the mammalian cell with a MAPK-ERK pathway inhibitor is between one and 24 hours prior to the contacting with the nucleic acid or the vector comprising the nucleic acid. In a still more preferred embodiment, the contacting the mammalian cell with a MAPK-ERK pathway inhibitor is one hour prior to the contacting with the nucleic acid or the vector comprising the nucleic acid.

In some embodiments, the method of delivering a nucleic acid into a mammalian cell, comprises the steps of contacting the mammalian cell with the nucleic acid or the vector comprising the nucleic acid and contacting the mammalian cell with one or more MAPK-ERK pathway inhibitors, for example at least two MAPK-ERK pathway inhibitors.

In some embodiments, the method of delivering a nucleic acid into a mammalian cell, comprises the steps of contacting the mammalian cell with the nucleic acid or a vector comprising the nucleic acid and contacting the mammalian cell with a MAPK-ERK pathway inhibitor at concentration of about 0.001 µM, about 0.005 µM, about 0.01 µM, about 0.05 µM, about 0.1 µM, about 0.5 µM, about 1 µM, about 5 µM, about 10 µM, about 15 µM, about 20 µM, about 25 µM, about 30 µM, about 35 µM, about 40 µM, about 45 µM, about 50 µM, about 75 µM, or about 100 µM, preferably about 5 µM, more preferably about 20 µM. In some embodiments, the method of delivering a nucleic acid into a mammalian cell, comprises the steps of contacting the mammalian cell with the nucleic acid or a vector comprising the nucleic acid and contacting the mammalian cell with ravoxertinib at concentration of about 0.5 µM, about 1 µM, about 5 µM, about 10 µM, about 15 µM, about 20 µM, preferably about 5 µM, more preferably about 20 µM. In some embodiments, the method of delivering a nucleic acid into a mammalian cell, comprises the steps of contacting the mammalian cell with the nucleic acid or a vector comprising the nucleic acid and contacting the mammalian cell with ulixertinib at concentration of about 0.5 µM, about 1 µM, about 5 µM, about 10 µM, about 15 µM, about 20 µM, preferably about 5 µM, more preferably about 20 µM. In some embodiments, the method of delivering a nucleic acid into a mammalian cell, comprises the steps of contacting the mammalian cell with the nucleic acid or a vector comprising the nucleic acid and contacting the mammalian cell with VX-11e at concentration of about 0.5 µM, about 1 µM, about 5 µM, about 10 µM, about 15 µM, about 20 µM, preferably about 5 µM, more preferably about 20 µM. In some embodiments, the method of delivering a nucleic acid into a mammalian cell, comprises the steps of contacting the mammalian cell with the nucleic acid or a vector comprising the nucleic acid and contacting the mammalian cell with selumetinib at concentration of about 0.5 µM, about 1 µM, about 5 µM, about 10 µM, about 15 µM, about 20 µM, preferably about 5 µM, more preferably about 20 µM. In some embodiments, the method of delivering a nucleic acid into a mammalian cell, comprises the steps of contacting the mammalian cell with the nucleic acid or a vector comprising the nucleic acid and contacting the mammalian cell with a MAPK-ERK pathway inhibitor at concentration of about 0.001 µM to about 50 µM, about 0.005 µM to about 50 µM, about 0.01 µM to about 50 µM, about 0.05 µM to about 50 µM, about 0.1 µM to about 50 µM, about 0.5 µM to about 50 µM, about 1 µM to about 50 µM, about 5 µM to about 50 µM, about 10 µM to about 50 µM, of about 0.001 µM to about 20 µM, about 0.005 µM to about 20 µM, about 0.01 µM to about 20 µM, about 0.05 µM to about 20 µM, about 0.1 µM to about 20 µM, about 0.5 µM to about 20 µM, about 1 µM to about 20 µM, about 5 µM to about 20 µM, about 10 µM to about 20 µM, of about 0.001 µM to about 15 µM, about 0.005 µM to about 15 µM, about 0.01 µM to about 15 µM, about 0.05 µM to about 15 µM, about 0.1 µM to about 15 µM, about 0.5 µM to about 15 µM, about 1 µM to about 15 µM, about 5 µM to about 15 µM, about 10 µM to about 15 µM, about 0.5 µM to about 10 µM, about 1 µM to about 10 µM, about 5 µM to about 10 µM, and preferably of about 1 µM to about 20 µM. In some embodiments, the method of delivering a nucleic acid into a mammalian cell, comprises the steps of contacting the mammalian cell with the nucleic acid or a vector comprising the nucleic acid and contacting the mammalian cell with ravoxertinib at concentration of about 1 µM to about 20 µM, about 5 µM to about 20 µM, about 10 µM to about 20 µM, about 1 µM to about 15 µM, about 5 µM to about 15 µM, about 10 µM to about 15 µM, about 1 µM to about 10 µM, about 5 µM to about 10 µM, and preferably of about 5 µM to about 20 µM. In some embodiments, the method of delivering a nucleic acid into a mammalian cell, comprises the steps of contacting the mammalian cell with the nucleic acid or a vector comprising the nucleic acid and contacting the mammalian cell with ulixertinib at concentration of about 1 µM to about 20 µM, about 5 µM to about 20 µM, about 10 µM to about 20 µM, about 1 µM to about 15 µM, about 5 µM to about 15 µM, about 10 µM to about 15 µM, about 1 µM to about 10 µM, about 5 µM to about 10 µM, and preferably of about 5 µM to about 20 µM. In some embodiments, the method of delivering a nucleic acid into a mammalian cell, comprises the steps of contacting the mammalian cell with the nucleic acid or a vector comprising the nucleic acid and contacting the mammalian cell with VX-11e at concentration of about 1 µM to about 20 µM, about 5 µM to about 20 µM, about 10 µM to about 20 µM, about 1 µM to about 15 µM, about 5 µM to about 15 µM, about 10 µM to about 15 µM, about 1 µM to about 10 µM, about 5 µM to about 10 µM, and preferably of about 5 µM to about 20 µM. In some embodiments, the method of delivering a nucleic acid into a mammalian cell, comprises the steps of contacting the mammalian cell with the nucleic acid or a vector comprising the nucleic acid and contacting the mammalian cell with selumetinib at concentration of about 1 µM to about 20 µM, about 5 µM to about 20 µM, about 10 µM to about 20 µM, about 1 µM to about 15 µM, about 5 µM to about 15 µM, about 10 µM to about 15 µM, about 1 µM to about 10 µM, about 5 µM to about 10 µM, and preferably of about 5 µM to about 20 µM.

In one embodiment of all aspects disclosed herein, the MAPK-ERK pathway inhibitor is an inhibitor of RAS. In one embodiment of all aspects disclosed herein, the MAPK-ERK pathway inhibitor is an inhibitor of K-RAS. In one embodiment of all aspects disclosed herein, the MAPK-ERK pathway inhibitor is an inhibitor of RAF. In one embodiment of all aspects disclosed herein, the MAPK-ERK pathway inhibitor is an inhibitor of B-RAF. In one embodiment of all aspects disclosed herein, the MAPK-ERK pathway inhibitor is an inhibitor of ERK1/ERK2, i.e. ERK1 and/or ERK2. In one embodiment of all aspects disclosed herein, the MAPK-ERK pathway inhibitor is an inhibitor of ERK1. In one embodiment of all aspects disclosed herein, the MAPK-ERK pathway inhibitor is an inhibitor of ERK2. In one embodiment of all aspects disclosed herein, the MAPK-ERK pathway inhibitor is an inhibitor of MEK1/MEK2, i.e. MEK1 and/or MEK2. In one embodiment of all aspects disclosed herein, the MAPK-ERK pathway inhibitor is an inhibitor of MEK1. In one embodiment of all aspects disclosed herein, the MAPK-ERK pathway inhibitor is an inhibitor of MEK2. In one preferred embodiment of all aspects disclosed herein, the MAPK-ERK pathway inhibitor is an inhibitor of ERK1/ERK2 or an inhibitor of MEK1/MEK2.

In one embodiment of all aspects disclosed herein, the inhibitor of RAS is selected from the group consisting of AMG-510, MRTX 849, BI 1701963 and BBP-454; or is a hydrate, solvate, or pharmaceutically acceptable salt thereof. In one embodiment of all aspects disclosed herein the inhibitor of RAF is selected from the group consisting of dabrafenib, encorafenib, vemurafenib, FORE-8394 (PLX-8394), tinloragenib, AZ-304, agerafenib, KIN-2787, BGB- 3245, ABM-1310, TQB-3233, UB-941, AFX-1251, ARQ 736, ASN003, AVB-BRAF, BDTX-4933, CCT196969, CFT1946, HLX208, RO5212054, RO7276389, and LY3009120; or is a hydrate, solvate, or pharmaceutically acceptable salt thereof. In one embodiment of all aspects disclosed herein the inhibitor of ERK1/ERK2 is selected from the group consisting of ulixertinib, ravoxertinib, VX-11e, SCH772984, LY3214996, HH-2710, ASTX029, ATG-017, JSL1187, JRP- 890, JRF-108, and MK-8353, preferably ravoxertinib, ulixertinib, VX-11e; or is a hydrate, solvate, or pharmaceutically acceptable salt thereof. In one embodiment of all aspects disclosed herein the inhibitor of MEK1/MEK2 is selected from the group consisting of selumetinib, trametinib, cobimetinib, binimetinib, PD-325901, and CI-1040, preferably selumetinib; or is a hydrate, solvate, or pharmaceutically acceptable salt thereof. In one preferred embodiment of all aspects disclosed herein, the MAPK-ERK pathway inhibitor is selected from the group consisting of ravoxertinib, ulixertinib, VX-11e, and selumetinib; or is a hydrate, solvate, or pharmaceutically acceptable salt thereof.

The term "inhibitor of ERK1/ERK2" is used herein to refer to any substance that reduces the expression or the activity of ERK1 and/or ERK2 protein kinases. In particular, an "ERK1/ERK2 inhibitor" means a substance that (i) interacts directly with ERK1 and/or ERK2, e.g., by binding to ERK1/2; or (ii) acts upstream of ERK1/ERK2, such as RAS, RAF or MEK inhibitors.

The chemical structures of the preferred MAPK-ERK pathway inhibitors for the present invention are shown in Table 1 below.

**Table 1: Chemical structures and manufacturers of MAPK-ERK pathway inhibitors for the present invention.**

| RAS inhibitors | | |
|---|---|---|
| AMG-510 (sotorasib, LUMAKRAS^{™}), | MRTX 849 (adagrasib) | |
| 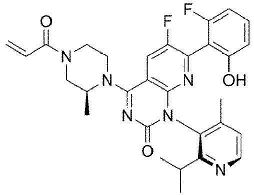 | 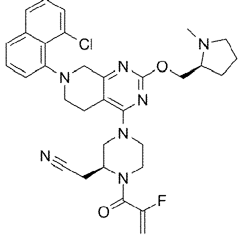 | |
| | | |

| RAF inhibitors | | |
|---|---|---|
| dabrafenib | encorafenib | vemurafenib |
| 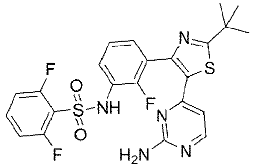 | 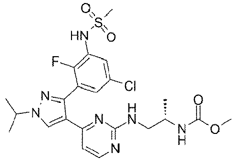 | 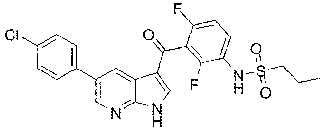 |
| FORE-8394 (PLX-8394) | tinlorafenib | AZ-304 |
| 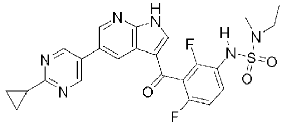 | 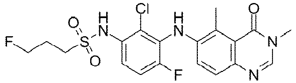 | 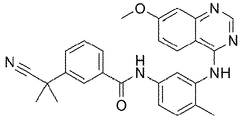 |
| agerafenib | KIN-2787 | BGB- 3245 (brimarafenib) |
| 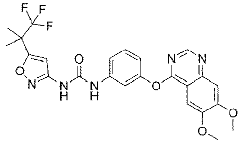 | 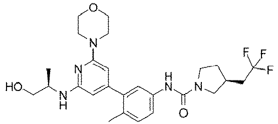 | 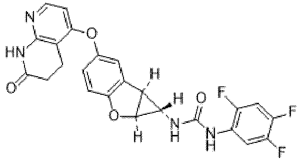 |
| ARQ 736 | CFT1946 | |
| 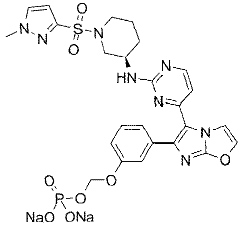 | 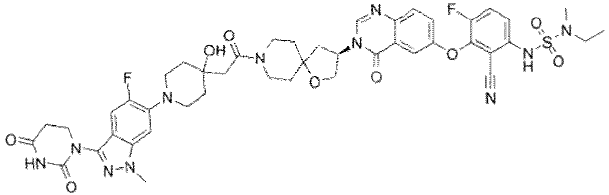 | |
| LY3009120 | CCT196969 | |
| 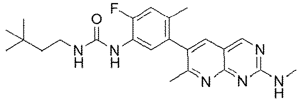 | 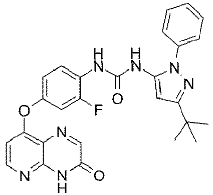 | |
| | | |

| ERK1/ERK2 inhibitors | | |
|---|---|---|
| ulixertinib | ravoxertinib | VX-11e |
| 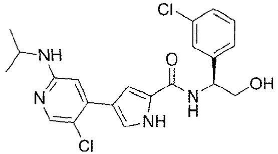 | 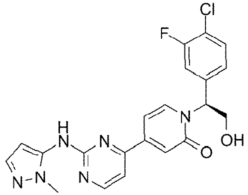 | 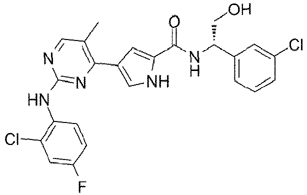 |
| LY3214996 (temuterkib) | HH-2710 (ASN007) | ASTX029 |
| 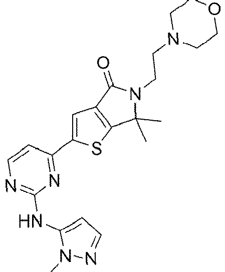 | 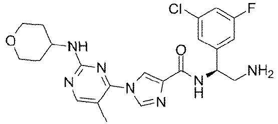 | 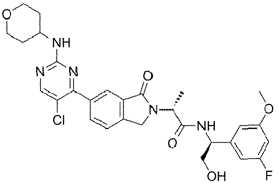 |
| ATG-017 (AZD0364) | MK-8353 | |
| 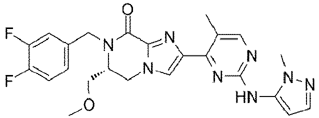 | 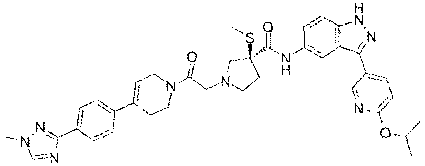 | |
| | | |

| MEK1/MEK2 inhibitors | | |
|---|---|---|
| selumetinib | trametinib | cobimetinib |
| 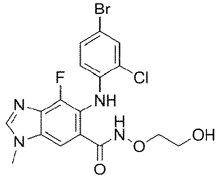 | 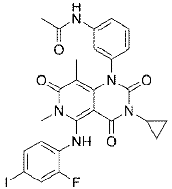 | 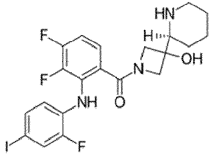 |
| binimetinib | PD-325901 | CI-1040 |
| 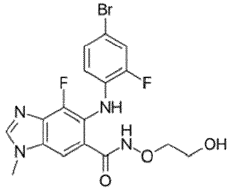 | 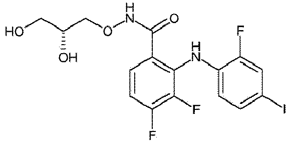 | 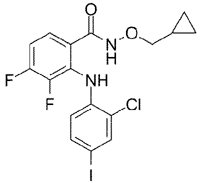 |

### Mammalian cells for nucleic acid delivery

Certain mammalian cell types can be difficult to transduce or transfect due to low efficiency of nucleic acid delivery into said cells. Different factors may contribute to the low efficiency of nucleic acid delivery, such as expression of receptors for vector entry, vector quality, cellular barriers, and host factors, e.g. cell cycle stage, differentiation state, or metabolic activity. It is therefore advantageous to enhance the efficiency of nucleic acid delivery into mammalian cells.

In some embodiments, the cell for nucleic acid delivery is a mammalian cell. In some embodiments, the mammalian cell is a non human great apes cell, a rodent cell or a human cell. In some embodiments, the mammalian cell is an embryonic stem cell, a cancer cell, a neuronal cell, an epithelial cell, an immune cell, an endocrine cell, a muscle cell, an erythrocyte, or a lymphocyte. In certain embodiments, the mammalian cell is a blood cell. In certain embodiments, the mammalian cell is an immune cell. In one embodiment of all aspects disclosed herein, the mammalian cell is selected from the group consisting of a B cell, a dendritic cell, a hematopoietic stem cell, a macrophage, a monocyte, a NKT cell, a NK cell, a plasmablast, and a T cell. In certain embodiments, the mammalian cell is a primary cell. In certain embodiments, the primary cell is human naive B cell, peripheral blood CD56⁺ NK cell, peripheral blood CD8⁺ cytotoxic T cell, peripheral blood CD4⁺ helper T cell, or peripheral blood CD14⁺ monocyte.

The term "B cells" as used in the context of the invention refers to a subgroup of lymphocytes that produce antibody molecules which may be either secreted or inserted into the plasma membrane where they serve as a part of B-cell receptors.

The term "dendritic cell" (DC) as used in the context of the invention refers to a subset of phagocytic cells belonging to the class of antigen presenting cells. In one embodiment, dendritic cells are derived from haematopoietic bone marrow progenitor cells.

The term "hematopoietic stem cells" (HSCs) as used in the context of the invention refers to multipotent primitive cells that can develop into all types of blood cells, including myeloid and lymphoid lineage cells. HSCs can be found in several organs, such as peripheral blood (PB), bone marrow (BM), and umbilical cord blood (UCB).

The term "macrophage" as used in the context of the invention refers to a subset of phagocytic cells produced by the differentiation of monocytes.

As used herein, the term "NK cell" or "natural killer cell" as used in the context of the invention refers to a subset of peripheral blood lymphocytes defined by the expression of CD56 or CD16 and the absence of the T cell receptor. As provided herein, the NK cell may also be differentiated from a stem or progenitor cell.

As used herein, the term "NKT cell" or "natural killer T cell" as used in the context of the invention refers to a subset of T cells that coexpress an αβ T cell receptor, and also express different molecular markers typically associated with NK cells, such as NK1.1. CD1d-restricted NKT cells ("type 2 NKT") recognize lipids and glycolipids presented by CD1d molecules rather than by major histocompatibility complexes (MHCs).

The term "plasmablast" as used in the context of the invention refers to a cell derived from an antigen-presenting B cell that proliferated. An antigen-presenting B cell is a B cell that encountered and internalized an antigen via a receptor-mediated endocytosis. Plasmablasts are stem cells capable of dividing to yield more cells that may differentiate later into plasma cells. Plasmablasts are capable of secreting antibodies, and may also act as antigen-presenting cell when they internalize antigens.

The terms "T cell" and "T lymphocyte" as used in the context of the invention are used interchangeably herein and include T helper cells (CD4⁺ T cells) and cytotoxic T cells (CTLs, CD8⁺ T cells), which include cytolytic T cells. The term "antigen-specific T cell" or similar terms refers to a T cell that recognises the antigen to which the T cell is directed and preferably exerts T cell effector functions. T cells are considered to be antigen-specific if they kill target cells expressing an antigen. T cell specificity can be assessed by any of a number of standard techniques, for example by proliferation assay or by measuring the synthesis of lymphokines such as interferon-γ.

In the context of the use according to the first aspect or the method according to the second aspect the mammalian cell is preferably an immune cell, in particular a NKT cell or T cell.

### Nucleic acids

In one embodiment of all aspects disclosed herein, the nucleic acid is a dsDNA, in particular a plasmid, or a cosmid, ssDNA or RNA, and preferably the dsDNA, or ssDNA comprises at least one transgene, and preferably the RNA comprises an mRNA, more preferably a mRNA encoding a transgene, miRNA, DsiRNA or a siRNA.

The term "dsDNA" as used in the context of the invention refers to double-stranded DNA consisting of two polynucleotide chains whose nitrogenous bases are connected by hydrogen bonds.

The term "plasmid" as used in the context of the invention refers to a small, extrachromosomal DNA molecule within a cell that is physically separated from chromosomal DNA and can replicate independently.

The term "cosmid" as used in the context of the invention refers to a plasmid containing one or more cohesive sites (cos sites) of the bacteriophage λ DNA.

The term "ssDNA" as used in the context of the invention refers to a linear structure that has only one DNA strand.

The term "siRNA" or "small interfering RNA (siRNA)" as used in the context of the invention refers to a class of double-stranded non-coding molecules, which are typically 20 - 24 base pairs in length, and operate within the RNA interference (RNAi) pathway. A siRNA induces cleavage and degradation of the target mRNA transcript of a protein-coding gene.

The term "miRNA" or "microRNA" as used in the context of the invention refers to small, single-stranded, non-coding RNA molecules containing 21 to 23 nucleotides, possessing the reverse complement of an mRNA transcript of a protein-coding gene. These miRNAs can inhibit the expression of that target protein-coding gene.

The term "DsiRNA" as used in the context of the invention refers to duplex RNAs having a length of 25-30 nucleotides that demonstrate increased potency in RNA interference compared to traditional siRNAs.

The term "inhibitory RNA molecule" as used in the context of the invention refers to an RNA molecule able to inhibit expression of a target mRNA such as a "siRNA", "miRNA", and a "DsiRNA".

The term "mRNA" as used in the context of the invention refers to a single-stranded RNA involved in protein synthesis. An mRNA molecule is made from a DNA template during the process of transcription. Subsequently, the mRNA may be translated into a peptide or protein by the process termed "translation" or "expression" in the ribosomes of a cell. Protein expression can be transient or stable.

A "gene" or "coding sequence" or a "sequence encoding a particular protein" is a nucleic acid molecule that is transcribed (in the case of DNA) and translated (in the case of mRNA) into a polypeptide *in vitro* or *in vivo* when placed under the control of appropriate regulatory sequences. The boundaries of the gene are defined by a start codon at the 5' (i.e., amino) terminus and a translation stop codon at the 3' (i.e., carboxy) terminus. A gene can include, but is not limited to, cDNA from prokaryotic or eukaryotic mRNA, genomic DNA sequences from prokaryotic or eukaryotic DNA, and even synthetic DNA sequences. The term "encoding" refers to the inherent property of specific sequences of nucleotides in a polynucleotide, such as a gene, cDNA, or mRNA, to serve as templates for the synthesis of other polymers and macromolecules in biological processes having either a defined sequence of nucleotides (i.e., rRNA, tRNA and mRNA) or a defined sequence of amino acids and the biological properties resulting therefrom.

The term "transgene" is used herein to refer to a nucleic acid that is intended to be or has been introduced into a mammalian cell. A transgene includes any nucleic acid, such as a heterologous polynucleotide sequence or a heterologous nucleic acid or a heterologous gene, encoding a protein or peptide. The terms transgene and heterologous gene/nucleic acid/polynucleotide sequence are used interchangeably herein. The term "mRNA encoding a transgene" refers to an mRNA transcribed from a DNA template encoding a transgene.

The nucleic acids described herein may be recombinant and/or isolated molecules.

The inventors have shown that contacting mammalian T cells with a MAPK-ERK inhibitor prior to contacting them with a vector such as CD3-LV, CD4-LV, CD8-LV, VSV-LV or AAV6 comprising a transgene encoding a CAR protein can result in increased expression of the CAR protein by up to 1.8-fold to up to 9.6-fold (Figures 12A, 13A, 14). In some embodiments, the nucleic acid comprises a transgene dsDNA, ssDNA, cDNA, or an mRNA encoding a transgene and the enhanced functional delivery of the nucleic acid results in an increased expression of the protein encoded by the transgene into the mammalian cell. In some embodiments, the term "enhancing functional delivery of a nucleic acid" means that the percentage of cells expressing the protein encoded by the transgene is increased when the transgene is delivered in the presence of the MAPK-ERK pathway inhibitor in comparison to the percentage of cells expressing that protein when the transgene is delivered in the absence of the MAPK-ERK pathway inhibitor. In some embodiments, the term "enhancing functional delivery of a nucleic acid" means that the percentage of mammalian cells expressing the protein encoded by the transgene is increased when the transgene is delivered in the presence of the MAPK-ERK pathway inhibitor in comparison to the percentage of cells expressing that protein when the transgene is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold,about 10-fold, about 12-fold, about 15-fold, about 20-fold, about 25-fold, about 30-fold. In some embodiments, the term "enhancing functional delivery of a nucleic acid" means that the percentage of mammalian cells expressing the protein encoded by the transgene is increased when the transgene is delivered in the presence of the MAPK-ERK pathway inhibitor in comparison to the percentage of cells expressing that protein when the transgene is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1.1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In some embodiments, the term "enhancing functional delivery of a nucleic acid" means that the expression of the protein encoded by the transgene is increased when the transgene is delivered in the presence of the MAPK-ERK pathway inhibitor in comparison to the expression of that protein when the transgene is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the expression of the protein encoded by the transgene is increased when the transgene is delivered in the presence of the MAPK-ERK pathway inhibitor in comparison to the expression of that protein when the transgene is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1.1 -fold to about 10-fold, about 1.2-fold to about 10-fold, about 1.3 -fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold. When the vector for delivery the nucleic acid is a viral vector, the n-fold increase of expression of the protein encoded by the transgene or of the mRNA encoding the transgene is also referred to in the contest of the invention as "transduction efficiency enhancement".

In certain embodiments, at least about 10%, about 20%, about 25%, about 30%, about 35%, about 40%, about 45%, about 50%, about 55%, about 60%, about 65%, about 70%, about 75%, about 80%, about 85%, about 90%, or about 95% of the mammalian cells express the protein encoded by the transgene when the transgene is delivered in the presence of the MAPK-ERK pathway inhibitor.

In further embodiments, the term "enhancing functional delivery of a nucleic acid" means that the percentage of mammalian cells expressing an mRNA encoding a transgene is increased when the transgene is delivered in the presence of the MAPK-ERK pathway inhibitor in comparison to the percentage of mammalian cells expressing that mRNA when the transgene is delivered in the absence of the MAPK-ERK pathway inhibitor. In some embodiments, the term "enhancing functional delivery of a nucleic acid" means that the percentage of mammalian cells expressing the mRNA encoding the transgene is increased when the transgene is delivered in the presence of the MAPK-ERK pathway inhibitor in comparison to the percentage of mammalian cells expressing that mRNA when the transgene is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of mammalian cells expressing the mRNA encoding the transgene is increased when the transgene is delivered in the presence of the MAPK-ERK pathway inhibitor in comparison to the percentage of cells expressing that mRNA when the transgene is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In alternative embodiments, the term "enhancing functional delivery of a nucleic acid" means that the expression of the mRNA encoding the transgene in the mammalian cells is increased when transgene is delivered in the presence of the MAPK-ERK pathway inhibitor in comparison to the expression of the mRNA encoding the transgene when the transgene is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In alternative embodiments, the term "enhancing functional delivery of a nucleic acid" means that the expression of the mRNA encoding the transgene in the mammalian cells is increased when transgene is delivered in the presence of the MAPK-ERK pathway inhibitor in comparison to the expression of the mRNA encoding the transgene when the transgene is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1.1 -fold to about 10-fold, about 1.2-fold to about 10-fold, about 1.3 -fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.In certain embodiments, at least about 10%, about 20%, about 25%, about 30%, about 35%, about 40%, about 45%, about 50%, about 55%, about 60%, about 65%, about 70%, about 75%, about 80%, about 85%, about 90%, or about 95% of the mammalian cells express the mRNA encoding the transgene when the transgene is delivered in the presence of the MAPK-ERK pathway inhibitor.

In some embodiments, the nucleic acid comprises an inhibitory RNA molecule inhibiting transcription of a target mRNA, and the enhanced functional delivery of the nucleic acid results in a decreased expression, i.e. downregulation, of the target protein, i.e. the protein encoded by the target mRNA, or of the target mRNA into the mammalian cell. In some embodiments, the term "enhancing functional delivery of a nucleic acid" means that the percentage of cells expressing the target protein is decreased when the inhibitory RNA molecule is delivered in the presence of the MAPK-ERK pathway inhibitor in comparison to the percentage of cells expressing that protein when the inhibitory RNA molecule is delivered in the absence of the MAPK-ERK pathway inhibitor. In some embodiments, the term "enhancing functional delivery of a nucleic acid" means that the percentage of mammalian cells expressing the target protein is decreased when the inhibitory RNA molecule is delivered in the presence of the MAPK-ERK pathway inhibitor in comparison to the percentage of cells expressing that protein when the inhibitory RNA molecule is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of mammalian cells expressing the target protein is decreased when the inhibitory RNA molecule is delivered in the presence of the MAPK-ERK pathway inhibitor in comparison to the percentage of cells expressing that protein when the inhibitory RNA molecule is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In further embodiments, the term "enhancing functional delivery of a nucleic acid" means that the expression of the target protein by the mammalian cells is decreased when the inhibitory RNA molecule is delivered in the presence of the MAPK-ERK pathway inhibitor in comparison to the expression of the target protein when the inhibitory RNA molecule is delivered in the absence of the MAPK-ERK pathway inhibitor. In some embodiments, the term "enhancing functional delivery of a nucleic acid" means that the expression of the target protein by the mammalian cells is decreased when the inhibitory RNA molecule is delivered in the presence of the MAPK-ERK pathway inhibitor in comparison to the expression of the target protein when the inhibitory RNA molecule is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the expression of the target protein by the mammalian cells is decreased when the inhibitory RNA molecule is delivered in the presence of the MAPK-ERK pathway inhibitor in comparison to the expression of the target protein when the inhibitory RNA molecule is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1. 1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1. 1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1. 1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In certain embodiments, less than about 5%, about 10%, about 20%, about 25%, about 30%, about 35%, about 40%, about 45%, about 50%, about 55%, about 60%, about 65%, about 70%, about 75%, about 80%, about 85%, or about 90% of the mammalian cells express the target protein when the inhibitory RNA molecule is delivered in the presence of the MAPK-ERK pathway inhibitor.

In further embodiments, the term "enhancing functional delivery of a nucleic acid" means that the percentage of mammalian cells expressing the target mRNA is decreased when the inhibitory RNA molecule is delivered in the presence of the MAPK-ERK pathway inhibitor in comparison to the percentage of mammalian cells expressing that mRNA when the inhibitory RNA molecule is delivered in the absence of the MAPK-ERK pathway inhibitor. In some embodiments, the term "enhancing functional delivery of a nucleic acid" means that the percentage of mammalian cells expressing the target mRNA is decreased when the inhibitory RNA molecule is delivered in the presence of the MAPK-ERK pathway inhibitor in comparison to the percentage of mammalian cells expressing that mRNA when the inhibitory RNA molecule is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of mammalian cells expressing the target mRNA is decreased when the inhibitory RNA molecule is delivered in the presence of the MAPK-ERK pathway inhibitor in comparison to the percentage of mammalian cells expressing that mRNA when the inhibitory RNA molecule is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In alternative embodiments, the term "enhancing functional delivery of a nucleic acid" means that the target mRNA expression in the mammalian cells is decreased when the inhibitory RNA molecule is delivered in the presence of the MAPK-ERK pathway inhibitor in comparison to the target mRNA expression when the inhibitory RNA molecule is delivered in the absence of the MAPK-ERK pathway inhibitor. In alternative embodiments, the term "enhancing functional delivery of a nucleic acid" means that the target mRNA expression in the mammalian cells is decreased when the inhibitory RNA molecule is delivered in the presence of the MAPK-ERK pathway inhibitor in comparison to the target mRNA expression when the inhibitory RNA molecule is delivered in the absence of the MAPK-ERK pathway inhibitor of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In alternative embodiments, the term "enhancing functional delivery of a nucleic acid" means that the target mRNA expression in the mammalian cells is decreased when the inhibitory RNA molecule is delivered in the presence of the MAPK-ERK pathway inhibitor in comparison to the target mRNA expression when the inhibitory RNA molecule is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1. 1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1. 1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1. 1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In certain embodiments, less than about 5%, about 10%, about 20%, about 25%, about 30%, about 35%, about 40%, about 45%, about 50%, about 55%, about 60%, about 65%, about 70%, about 75%, about 80%, about 85%, or about 90% of the mammalian cells express the target mRNA when the inhibitory RNA molecule is delivered in the presence of the MAPK-ERK pathway inhibitor.

Methods to calculate expression of an "mRNA encoding a transgene" are known to a person skilled in the art and comprise, but are not limited to, the comparative Ct method, wherein ΔΔ Ct = Δ Ct of sample- Δ Ct of reference gene, and the relative gene expression or fold change can be calculated by taking -ΔΔCt as the power of 2 (Rao X., et al., 2013. An improvement of the 2^(-delta delta CT) method for quantitative real-time polymerase chain reaction data analysis. Biostat Bioinforma Biomath. 3(3): 71-85). Target "mRNA expression" usually refers to the expression of a target mRNA, e.g. a mRNA encoding the transgene or the mRNA target of an inhibitory molecule, in comparison to the expression of one or more housekeeping genes (i.e. a gene constitutively expressed) in the same sample.

In one embodiment of all aspects disclosed herein, the transgene encodes for a CAR, a TCR or subunit thereof, or a therapeutic protein, preferably a vaccine antigen, an antibody, an enzyme, a growth factor or a cytokine. In one preferred embodiment of all aspects disclosed herein, the transgene encodes for a CAR or a TCR, or a subunit thereof, more preferably for a CAR.

### CAR and TCR

According to the invention, the term "CAR" or "chimeric antigen receptor" is synonymous with the terms "chimeric T cell receptor" and "artificial T cell receptor" and relates to an artificial receptor comprising a single molecule or a complex of molecules that recognizes, i.e. binds to, a target structure such as an antigen on a target cell such as a cancer cell, for example by binding of an antigen binding domain to the antigen expressed on the surface of the target cell, and which may confer specificity to an immune effector cell such as a T cell expressing said CAR on the cell surface. Such cells do not necessarily require processing and presentation of an antigen for recognition of the target cell, but may preferably recognize with specificity any antigen present on a target cell. Preferably, recognition of the target structure by a CAR results in activation of the immune effector cell expressing said CAR. A CAR may comprise one or more protein subunits said protein subunits comprising one or more domains as described herein.

Generally, a CAR molecule comprises:
i) a target antigen binding domain;
ii) a hinge domain, e.g. CD8α hinge domain;
iii) a transmembrane domain; and
iv) an intracellular domain, for example comprising a 4-1BB costimulatory domain, and a CD3-zeta signaling domain.

The target antigen binding domain is generally part of the extracellular domain of the CAR. In one embodiment of the present invention, the antigen binding domain of the CAR binds to an antigen selected from the group consisting of AG72 (Tumor-associated glycoprotein 72), B7-H3 (B7 homolog 3 protein), BAFF (B-cell activating factor), BCMA (B-cell maturation antigen), CD4, CD5, CD7, CD19, CD22, CD123, CD20, CD30, BCMA and CD19, CD33, CD38, CD56, CD79b, CD138, CD207, CEA (Carcinoembryonic Antigen), CLDN6/Claudin18.2, CLL-1 (C-type lectin-like molecule-1), EGFR (Epidermal Growth Factor Receptor), FAP (Fibroblast Activation Protein Alpha), FLT3 (FMS-like tyrosine kinase 3), GD2 (Disialoganglioside), GPC3 (Glypican-3), GPRC5D (G protein-coupled receptor, class C, group 5, member D), GUCY2C (Guanylate Cyclase 2C), HER-2 (Human Epidermal Growth Factor Receptor-2), MMP-2 (Matrix Metalloproteinase-2), MUC-1 (Mucin 1), NKG2D (Natural Killer Group 2 membrane D), PSMA (Prostate-Specific Membrane Antigen), ROR-1 (Receptor Tyrosine Kinase Like Orphan Receptor 1), SLAMF7 (Signaling Lymphocytic Activation Molecule Family Member 7), T4 (Thyroxin 4), TTACI (Transmembrane activator and Calcium modulating ligand interactor), and TRBC1 (T cell Receptor Constant β Chain-1), or a functional variant thereof, or a combination thereof.

In one embodiment of the present invention, the antigen binding domain of the CAR comprises a mono, bi or multispecific scFv, a single domain antibody, or a single chain antibody-like protein scaffold, in particular an anticalin, Ankyrin repeat protein, DARPin, affibody, atrimer, avimer, neutrophil gelatinase-associated lipocalin (NGAL) or evasin. Preferably, the antigen binding domain of the CAR comprises a scFv.

A CAR comprises a transmembrane domain that is fused to the extracellular domain of the CAR either directly or by a hinge domain. The transmembrane domain may be derived either from a natural or from a synthetic source. In one embodiment, the transmembrane domain comprises a transmembrane domain of a protein selected from the group consisting of the alpha, beta or zeta chain of the T cell receptor, CD28, CD3 epsilon, CD45, CD4, CD5, CD8, CD9, CD16, CD22, CD33, CD37, CD64, CD80, CD86, CD134, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7Ra, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CDllb, ITGAX, CD11c, ITGBl, CD29, ITGB2, CD18, LFA-1, ITGB7, TNFR2, DNAMl (CD226), SLAMF4 (CD244, 2B4), CD84, CD96 (Tactile), CEACAM1, CRT AM, Ly9 (CD229), CD160 (BY55), PSGLl, CDIOO (SEMA4D), SLAMF6 (NTB-A, Lyl08), SLAM (SLAMF1, CD150, IPO-3), BLAME (SLAMF8), SELPLG (CD162), LTBR, PAG/Cbp, NKp44, NKp30, NKp46, NKG2D, and NKG2C, or a functional variant thereof.

The term "antibody" as used in the context of the invention refers to an immunoglobulin comprising at least two heavy (H) chains and two light (L) chains interconnected by disulfide bonds. Each heavy chain comprises a heavy chain variable region (abbreviated herein as VH) and a heavy chain constant region. Each light chain comprises a light chain variable region (abbreviated herein as VL) and a light chain constant region. Antibodies may exist in a variety of forms including, for example, polyclonal antibodies, monoclonal antibodies, antibody fragments, as well as single-chain antibodies and humanized antibodies (Harlow et al., 1999, In: Using Antibodies: A Laboratory Manual, Cold Spring Harbor Laboratory Press, NY; Harlow et al., 1989, in: Antibodies: A Laboratory Manual, Cold Spring Harbor, New York; Houston et al., 1988, Proc Natl Acad Sci USA 85: 5879-5883; Bird et al., 1988, Science 242: 423-426).

The term "antibody fragment" as used in the context of the invention refers to a portion of an intact antibody and typically comprises the antigenic determining variable regions of an intact antibody. Examples of antibody fragments include, but are not limited to, Fab, Fab', F(ab')2, and Fv fragments, linear antibodies, scFv antibodies, and multi-specific antibodies formed from antibody fragments.

An "antibody heavy chain", as used in the context of the invention, refers to the larger of the two types of polypeptide chains present in antibody molecules in their naturally occurring conformations. An "antibody light chain", as used in the context of the invention, refers to the smaller of the two types of polypeptide chains present in antibody molecules in their naturally occurring conformations, κ and λ light chains referring to the two major antibody light chain isotypes.

The term "TCR" (T cell receptor) as used in the context of the invention refers to a heterodimeric cell surface protein of the immunoglobulin super-family that associates with the invariant CD3 dimers CD3εγ, CD3εδ, and CD3ζζ to form a TCR-CD3 complex. The TCR mediates recognition of antigenic peptides bound to MHC molecules (pMHC), while the CD3 molecules transduce activation signals to the T cell. TCRs consists of two main subunits α- and β- chains (αβ-TCR) or γ- and δ-chains (γδ-TCR), which are twisted together and have quite distinct anatomical locations and functions.

T cells expressing an engineered T cell receptor (TCR-T cells or TCR-engineered T cells) represent a promising tool for cancer immunotherapy. It allows to generate of sufficient numbers of T cells specific for a given tumor antigen in a short period of time, avoiding their exhaustion. The TCR is usually transduced into central memory T cells or T cells with stem cell characteristics, which may ensure better persistence and function upon transfer. TCR-engineered T cells are usually infused into cancer patients who have been rendered lymphopenic by chemotherapy or irradiation, which allows for efficient engraftment but inhibits immune suppression.

Exemplary TCRs for the present invention bind to an antigen selected from the group consisting of CEA (carcinoembryonic antigen), gp100 (glycoprotein 100), HBV, HPV16-E6, HPV16-E7, KRASG12D, MAGE-A3, A4, A10 (melanoma-associated antigen 3, 4, 10), MART-1 (melanoma antigen recognized by T cells 1), MCPyV (Merkel cell polyomavirus), NY-ESO-1, and TP53 (tumor protein p53).

### Therapeutic proteins

The term "therapeutic protein" as used in the context of the invention refers to a protein with a beneficial or advantageous effect on a condition or disease state of a subject when expressed by the subject in a therapeutically effective amount. In one embodiment, a therapeutic protein has curative or palliative properties. A therapeutic protein may have prophylactic properties and may be used to delay the onset of a disease or to reduce the severity of such a disease or pathological condition. The term "therapeutic protein" includes entire proteins or peptides, and can also refer to therapeutically active fragments thereof. It can also include therapeutically active variants of a protein. Examples of therapeutically active proteins include, but are not limited to, vaccine antigens, antibodies, enzymes, growth factors, and cytokines.

The term "antigen" as used in the context of the invention refers to any protein or peptide capable of eliciting an immune response in a mammal. An antigen preferably comprises at least 8 amino acids and most preferably comprises between 8 and 12 amino acids. The term "antigen" as used in the context of the invention may refer to a "bacterial antigen", "viral antigen", "fungal antigen","tumor-associated antigen", and "tumor-specific antigen". In this context, the term "tumor-associated antigen" means a structure that is predominantly presented by tumor cells and thereby allows a differentiation from non-malignant tissue. Preferably, such a tumor-associated antigen is located on or in the cell membrane of a tumor cell. Examples of tumor-associated antigens are described, e.g., in DeVita et al. (Eds., "Biological Therapy of Cancer", 2. Edition, Chapter 3: Biology of Tumor Antigens, Lippincott Company, ISBN 0-397-51416-6 (1995)). A "tumor-specific antigen" (TSA) or "neoantigen" is an antigen that is expressed exclusively by tumour cells and is associated with the tumourigenesis process (mutations and viral induction). Targeting these neoantigens with immunotherapy has a very limited risk of toxicity because they are not expressed by normal tissues.

The term "cytokine" as used in the context of the invention includes naturally occurring cytokines and functional variants thereof, as well as fragments of the naturally occurring cytokines and variants thereof.

Cytokines are a category of small proteins (~5-20 kDa) that are important in cell signaling. Their release affects the behaviour of surrounding cells. Cytokines are involved in autocrine signalling, paracrine signalling and endocrine signalling as immunomodulatory agents. Cytokines include chemokines, interferons, interleukins, lymphokines and tumour necrosis factors, but generally not hormones or growth factors, although there is some overlap in terminology. Cytokines are produced by a wide range of cells, including immune cells such as macrophages, B lymphocytes, T lymphocytes and mast cells, as well as endothelial cells, fibroblasts and various stromal cells. A given cytokine may be produced by more than one cell type. Cytokines act through receptors and are particularly important in the immune system; cytokines modulate the balance between humoral and cellular immune responses and regulate the maturation, growth and responsiveness of specific cell populations. Some cytokines enhance or inhibit the action of other cytokines in complex ways. The cytokines described herein may be prepared as fusion or chimeric polypeptides containing a cytokine portion and a heterologous polypeptide, e.g. albumin, to prolong the circulation half-life relative to free cytokine.

In certain embodiments, the cytokine is selected from the group consisting of IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IFN-γ, IFN-α, IFN-β, IFN-ω, TNF- α, and TNF- β, 4-1BB ligand, B-cell activating factor (BAFF), FAS ligand (FASL), lymphotoxin, OX40L, RANKL, and TRAIL, or a functional variant thereof.

In certain embodiments, the cytokine is a chemokine, such as the C type chemokines XCL1 and XCL2, C-C type chemokines, including CCL1-CCL28, and CXC type chemokines, including CXCL1-CXCL17, or a functional variant thereof. Chemokine can be useful for treatment of immune disorders.

In certain embodiments, the therapeutic protein is an enzyme selected from the group consisting of alpha galactosidase, alpha-1-anti-trypsin, alpha-L-iduronidase , alpha-N-acetylglucosaminidase, apolipoproteins, ASS 1 (arginosuccinate synthase), ATP7B (copper transporting ATPase2), ABCA4 (ATP-binding cassette, sub-family A (ABC1), member 4), beta-glucocerebrosidase, beta-hexosaminidase A, b-globin, blood coagulation factor (e.g. Factor XIII, Factor IX, Factor VIII, Factor X, Factor VII, Factor VIla, protein C), CFTR (cystic fibrosis transmembrane regulator protein), CLN2p/TPP-1 (CLN2/Tripeptidyl Peptidase 1), cystathionine beta-synthase, connexin26, elosulfase alfa, GAA (acid alpha-glucosidase), galsulfase, glucokinase, glucose-6-phosphatase, GM2-AP, GUCY2D (guanylate cyclase 2D), iduronidase (IDUA), idursulfase, LCA 5 (LCA-Lebercilin), lecithin-cholesterol acyltransferase (LCAT), lipoprotein lipase (LPL), low-density lipoprotein receptor (LDL-R), lysosomal β-hexosaminidase, mertk, NPC1 , ornithine ketoacid aminotransferase, ornithine transcarbamoylase (OTC), phenylalanine hydroxylase (PAH) or phenylalanine ammonia-lyase (PAL), Rab escort protein 1, RPE65 (retinal pigment epithelium-specific 65 kDa protein), SERPING1 (C1 protease inhibitor or C1 esterase inhibitor), sphingomyelinase, and suicide gene product (e.g. human deoxycytidine kinase (dCK)), or a functional variant thereof.

In certain embodiments, the therapeutic protein is a growth factor selected from the group consisting of acidic fibroblast growth factor (aFGF), angiopoietin, basic fibroblast growth factor (bFGF), bone morphogenic protein (BMP), brain-derived neurotrophic factor (BDNF), ciliary neurotrophic factor (CNTF), connective tissue growth factor (CTGF), epidermal growth factor (EGF), ephrins, erythropoietin (EPO), follicle stimulating hormone (FSH), glial cell line derived neurotrophic factor (GDNF), granulocyte colony stimulating factor (GCSF), growth hormone (GH), growth hormone releasing factor (GRF), hepatocyte growth factor (HGF), human chorionic gonadotropin (hCG), insulin growth factors I and II (IGF-I and IGF-II), keratinocyte growth factor (KGF), nerve growth factor (NGF), neurturin (NRTN), noggin, platelet-derived growth factor (PDGF), transforming growth factor (TGF) family member, and vascular endothelial growth factor (VEGF), or a functional variant thereof.

Examples of therapeutic proteins and the corresponding treatable disease are listed in the Table 2 below.

**Table 2: Therapeutic proteins**

| **Therapeutic protein** | **Disease** |
|---|---|
| albumin | Analbuminaemia |
| alpha galactosidase | Fabry's disease |
| alpha-1-anti-trypsin | Alpha-1-anti-trypsin deficiency; emphysema or chronic obstructive pulmonary disease (COPD) |
| alpha-L-iduronidase | mucopolysaccharidosis I (MPS I) |
| alpha-N-acetylglucosaminidase | mucopolysaccharidosis type IIIB (MPS IIIB) |
| apolipoproteins | apo lipoprotein (Apo) A-I deficiency |
| ASS 1 (arginosuccinate synthase) | Citrullinemia Type 1 |
| ATP7B (copper transporting ATPase2) | Wilson's disease |
| ABCA4 (ATP-binding cassette, sub-family A (ABC1), member 4) | Stargardt disease |
| beta-glucocerebrosidase | Gaucher disease Type 1 |
| beta-hexosaminidase A | Tay Sachs disease |
| b-globin | β-thalassemia |
| blood coagulation factor (e.g. Factor XIII, Factor IX, Factor VIII, Factor X, Factor VII, Factor VIla, protein C) | hemophilia |
| CFTR (cystic fibrosis transmembrane regulator protein) | cystic fibrosis |
| CLN2p/TPP-1 (CLN2/Tripeptidyl Peptidase 1) | Batten disease, Neuronal Ceroid Lipofuscinosis Type 2 (CLN2) |
| cystathionine beta-synthase | homocystinuria |
| Connexin26 | DFNB1 (Connexin 26 deafness) |
| elosulfase alfa | mucopolysaccharidosis type IVA (MPS IVA) |
| GAA (acid alpha-glucosidase) | Pompe disease |
| galsulfase | mucopolysaccharidosis type VI (MPS VI) |
| glucokinase | diabetes |
| glucose-6-phosphatase | glycogen storage disease type I (GSDI) |
| GM2-AP | GM2 Gangliosidoses |
| GUCY2D (guanylate cyclase 2D) | Leber congenital amaurosis (LCA) |
| iduronidase (IDUA) | mucopolysaccharidosis type I (MPS I) |
| idursulfase | mucopolysaccharidosis type II (MPS II) |
| keratinocyte growth factor (KGF) | epithelial tissue damage |
| LCA 5 (LCA-Lebercilin) | Leber congenital amaurosis (LCA) |
| lecithin-cholesterol acyltransferase (LCAT) | LCAT deficiency and atherosclerosis |
| lipoprotein lipase (LPL) | LPL deficiency |
| low-density lipoprotein receptor (LDL-R) | familial hypercholesterolemia (FH) |
| lysosomal β-hexosaminidase | Lysosomal Storage Disorders |
| mertk | autosomal recessive retinitis pigmentosa |
| NPC1 | Niemann-Pick C1 disease |
| ornithine ketoacid aminotransferase | Gyrate Atrophy |
| ornithine transcarbamoylase (OTC) | OTC deficiency (OTCD) |
| phenylalanine hydroxylase (PAH) or phenylalanine ammonia-lyase (PAL) | phenylketonuria (PKU) |
| Rab escort protein 1 | Choroideremia |
| RPE65 (retinal pigment epithelium-specific 65 kDa protein) | Leber congenital amaurosis (LCA), retinitis pigmentosa (RP) |
| SERPING1 (C1 protease inhibitor or C1 esterase inhibitor) | hereditary angioedema (HAE) |
| sphingomyelinase | Niemann-Pick disease |
| suicide gene product (e.g. human deoxycytidine kinase (dCK)) | Cancer |
| tumor suppressor protein (e.g., p53, Rb, Wt-l, NF1, Von Hippel-Findau (VHF)) | Cancer |

### Vectors for nucleic acid delivery

In one embodiment of all aspects disclosed herein, the vector comprising the nucleic acid is selected from the group comprising a viral vector and a nanocarrier, preferably a lipid nanoparticle. In one embodiment, the viral vector is selected from the group comprising a lentiviral vector, an adeno-associated viral vector, in particular AAV6 or AAV2, an adenoviral vector, a retroviral vector, and a herpes simplex virus, and wherein the viral vector is preferably a pseudotyped and/or targeted viral vector, more preferably a CD3-, CD4-, CD62L- or CD8-targeted lentiviral vector. In a preferred embodiment of all aspects disclosed herein, the vector is preferably a pseudotyped and/or targeted lentiviral vector, more preferably a CD3-, CD4-, CD62L- or CD8-targeted lentiviral vector.

In some embodiments, the term "vector" as used in the context of the invention refers to any vectors known to a skilled person in the art, including plasmid vectors, cosmid vectors, phage vectors such as lambda phage, viral vectors such as retroviral vectors, adenoviral (Ad) vectors (e.g., non-replicating Ad5, Ad11, Ad26, Ad35, Ad49, ChAd3, ChAd4, ChAd5, ChAd7, ChAd8, ChAd9, ChAd10, ChAd11, ChAd16, ChAd17, ChAd19, ChAd20, ChAd22, ChAd24, ChAd26, ChAd30, ChAd31, ChAd37, ChAd38, ChAd44, ChAd63 and ChAd82 vectors or replication-competent Ad4 and Ad7 vectors known from the prior art, e.g. WO 2005/071093 A2), adeno-associated virus (AAV) vectors (e.g., AAV type 6 (AAV6) and AAV type 2 (AAV2)), alphaviral vectors (e.g., Venezuelan equine encephalitis virus (VEE), sindbis virus (SIN), semliki forest virus (SFV), and VEE-SIN chimeras), baculoviral vectors, herpes virus vectors, measles virus vectors, pox virus vectors (e.g., vaccinia virus, modified vaccinia virus Ankara (MVA), NYVAC (derived from the Copenhagen strain of vaccinia), avipox vectors (canarypox (ALVAC) and fowlpox (FPV) vectors), vesicular stomatitis virus vectors, virus-like particles (VLP), and artificial chromosome vectors such as bacterial artificial chromosomes (BAC), yeast artificial chromosomes (YAC), or P1 artificial chromosomes (PAC). The term "vector" includes expression vectors, cloning vectors and vectors that are useful to generate recombinant viruses in host cells. Expression vectors comprise plasmids as well as viral vectors and generally contain a desired coding sequence and appropriate DNA sequences necessary for the expression of the operably linked coding sequence in a particular host organism (e.g., bacterial, yeast, plant, insect, or mammalian) or in *in vitro* expression systems.

The term "adeno-associated viral vector or AAV vector" as used in the context of the present invention refers to a complete virus particle, such as a wild-type ("wt") AAV virus particle, i.e., including a linear, single-stranded AAV nucleic acid genome associated with an AAV capsid protein coat. In this regard, single-stranded AAV nucleic acid molecules of either complementary sense (i.e., "sense" or "antisense" strands) can be packaged into any one AAV virion; both strands are equally infectious. The AAV vector of the present invention may also be an infectious and replication-defective virus composed of an AAV protein shell encapsidating a heterologous DNA molecule of interest flanked on both sides by an AAV ITR.

The term "adenovirus" (Ad) as used in the context of the invention refers to a non-enveloped, icosahedral virus that has been identified in several avian and mammalian hosts. Human adenoviruses (hAds) belong to the *Mastadenovirus* genus which includes all known human Ads and many Ads of animal origin (e. g., bovine, porcine, canine, murine, equine, monkey and sheep). Human adenoviruses are generally classified into six subgroups (A-F) based on a number of biological, chemical, immunological and structural criteria, including hemagglutination properties of rat and rhesus monkey erythrocytes, DNA homology, restriction enzyme cleavage patterns, percentage G+C content and oncogenicity (Straus, 1984, in The Adenoviruses, ed. H. Ginsberg, pp. 451-498, New York: Plenus Press, and Horwitz, 1990; in Virology, eds. B. N. Fields and D. M. Knipe, pp. 1679-1721).

The term "retrovirus" (RV) as used in the context of the invention refers to an enveloped RNA virus that is capable of infecting animal cells, and that utilizes the enzyme reverse transcriptase in the early stages of infection to generate a DNA copy of their RNA genome, which is then typically integrated into the host genome. Non-limiting examples of retroviral vectors comprise Moloney murine leukemia virus (MLV)-derived vectors, retroviral vectors based on a murine stem cell virus, and complex retrovirus-derived vectors, such as lentiviral vectors.

The term "lentivirus" (LV) as used in the context of the invention refers to a genus of the family *Retroviridae.* Lentiviruses are unique among the retroviruses in that they can infect non-dividing cells; they can deliver a significant amount of genetic information into the DNA of the host cell, making them one of the most efficient methods of a gene delivery vectors. Non-limiting examples of lentiviruses include HIV (human immunodeficiency virus; including HIV type 1 and HIV type 2), Visna-Maedi virus (VMV), the caprine arthritis-encephalitis (CAE) virus, equine infectious anemia (EIA) virus, feline immunodeficiency virus (FIV), bovine immune deficiency virus (BIV), and simian immunodeficiency virus (SIV).

The term "herpes simplex virus (HSV)" as used in the context of the invention refers to a human neurotropic dsDNA virus with a large envelope and with the characteristics of lifelong latent infection of neurons, and of allowing for long-term transgene expression. An HSV vector has a double-stranded linear genome of 150kb and is particularly suitable for gene delivery to the nervous system and tumour cells due to its neurotrophic nature. An HSV vector does not integrate into the host genome, resulting in transient expression in infected cells. Most HSV vectors have been developed from strains of HSV-1. HSV-1 infection can be lytic or latent.

The term "pseudotyping" generally refers to the incorporation of one or more heterologous viral glycoproteins onto the cell surface of a virus particle, allowing the virus particle to infect a selected cell different from its normal target cells. Typically, the glycoprotein coding regions of the viral vector have been genetically modified, for example by deletion, to prevent expression of its own glycoprotein.

In certain embodiments, the viral vector (e.g., retroviral, lentiviral) is pseudotyped with one or more viral glycoproteins or envelope proteins to target selected cell types. In certain embodiments, the viral vector is pseudotyped with a heterologous viral glycoprotein that targets immune cells, such as B cells, plasmablasts, NK cells, or T cells. In certain embodiments, the heterologous viral glycoprotein is derived from a glycoprotein of a virus selected from the group consisting of baboon endogenous retrovirus (e.g., BaEV), cocal virus (COCV), feline endogenous retrovirus (e.g., RD114), measles virus (e.g., hemagglutinin (H), fusion protein (F)), nipah virus (e.g., G/F), and vesicular stomatitis virus (e.g., VSV-G). In preferred embodiments of all aspects disclosed herein, the viral vector is pseudotyped with a vesicular stomatitis virus G-protein (VSV-G) envelope protein.

T cell-targeted viral vectors using CD3, CD62L, CD4, and CD8 as entry receptors enable selective transduction of the corresponding T cell subtypes. It has been shown that transduction with these targeted vectors overcomes the risk of off-target modifications, allows for simpler manufacturing processes, and can be used for direct *in vivo* gene transfer applications (Frank, AM, and Buchholz, CJ, 2019).

The term "nanocarrier" as used in the context of the invention refers to a carrier used as a transport module for a nucleic acid. Nanocarriers may have a diameter of 5-1000 nm, in particular from about 5 to about 500 nm, more particularly from about 5 to about 300 nm. In particular, the size of the nanocarrier is such that it can be taken up by a mammalian cell by endocytosis and subsequently transported to endo-lysosomal organelles. Examples of nanocarriers include microspheres, core-shell microparticles, polyelectrolyte microparticles, metallic microparticles, metal-organic framework (MOF) materials, emulsions and microparticulate powders (e.g. obtained by spray drying), drug conjugates, polymer conjugates, polymeric nanoparticles, polymeric micelles, emulsions, lipid-based nanocarriers, extracellular vesicles, dendrimers, core-shell nanoparticles, carbon nanotubes and metallic nanoparticles. The nanocarriers may be formed from any suitable biocompatible materials, which may be biodegradable or non-biodegradable. Examples of suitable biodegradable materials include collagen, fibrin, chitosan, hyaluronic acid, dextran, poly(anhydrides), degradable polyesters, poly(hydroxy acids), poly(ortho esters), poly(propylfumerates), poly(caprolactones), polyamides, polyamino acids, polyacetals, biodegradable polycyanoacrylates, biodegradable polyurethanes, poly(glycerol sebacates), and polysaccharides. Non-biodegradable, yet biocompatible, materials include polypyrrole, polyanilines, polythiophene, polystyrene, polyesters, non-biodegradable polyurethanes, polyureas, polyethylene vinyl acetate, polypropylene, polymethacrylate, polyethylene, polycarbonates, and polyethylene oxide). Lipid-based nanocarriers include solid lipid nanoparticles, liposomes and micelles. Preferred "nanocarriers" for the present invention are "lipid nanoparticles" (LNP).

The term "lipid nanoparticle" as used in the context of the invention refers to any lipid composition that can be used to deliver a nucleic acid, preferably a RNA nucleic acid, including, but not limited to, liposomes or vesicles, wherein an aqueous volume is encapsulated by amphipathic lipid bilayers (i.e. single; unilamellar or multiple; multilamellar), or wherein the lipids coat an interior comprising the therapeutic nucleic acid, or lipid aggregates or micelles.

In one embodiment, the lipid-nanoparticle comprises at least one lipid selected from, but not limited to, 9A1P9, A2-Iso5-2DC18, ALC-0159, ALC-0315, BAME-016B, BHEM-Cholesterol, Cholesterol, C12-200, cKK-E12, 3D6ODC-Cholesterol, DLin-DMA, DLin-K-DMA, DLin-MC3-DMA, DLin-KC2-DMA, DODMA, DOPE, DOSPA, DOTMA, DOTAP, DSPC, ePC, FTT5, Lipid H (SM-102), 98N12-5, OF-Deg-Lin, 306Oᵢ₁₀, PLGA, PEG, PEG-DMG and PEGylated lipid, such as PEG₂₀₀₀-DMG, β-sitosterol, TT3 (Hou, X, et al. 2021. Lipid nanoparticles for mRNA delivery. Nat Rev Mater 6, 1078-1094).

Definitions given and embodiments described with respect to the first aspect apply also to the second aspect, in as far as they are applicable. Also, definitions and embodiments described below, in particular under the header 'Definitions and further embodiments of the invention' apply to the method of the second aspect.

### Other transduction enhancers

The MAPK-ERK pathway inhibitors used in the present invention target a previously unknown pathway to enhance transduction. Because they have completely different and independent modes of action, they can be combined with other substances that act as transduction enhancers, resulting in a synergistic increase in transduction efficiency.

In a third aspect, the invention provides an use of a MAPK-ERK pathway inhibitor in combination with another type of transduction enhancer for enhancing functional delivery of a nucleic acid into a mammalian cell.

The term "transduction enhancer" refers to a substance capable of enhancing the transduction efficiency of a nucleic acid into a mammalian cell, and thus enhancing the functional delivery of a nucleic acid into said mammalian cell.

When using a MAPK-ERK pathway inhibitor in combination with another type of transduction enhancer for enhancing functional delivery of a nucleic acid into a mammalian cell, the term "enhancing functional delivery of a nucleic acid" into a mammalian cell means that the delivery of the nucleic acid to the nucleus or to the cytoplasm in the presence of the MAPK-ERK pathway inhibitor and of the another type of transduction enhancer is higher in comparison to the delivery of the nucleic acid to the nucleus or to the cytoplasm in the absence of the MAPK-ERK pathway inhibitor and of the another type of transduction enhancer.

In some embodiments, the nucleic acid comprises a transgene dsDNA, ssDNA, cDNA, or an mRNA encoding a transgene and the enhanced functional delivery of the nucleic acid results in an increased expression of the protein encoded by the transgene into the mammalian cell. In these embodiments, the term "enhancing functional delivery of a nucleic acid" means that the percentage of cells expressing the protein encoded by the transgene is increased when the transgene is delivered in the presence of the MAPK-ERK pathway inhibitor and of the another type of transduction enhancer in comparison to the percentage of cells expressing that protein when the transgene is delivered in the absence of the MAPK-ERK pathway inhibitor and of the another type of transduction enhancer. In some embodiments, the term "enhancing functional delivery of a nucleic acid" means that the percentage of cells expressing the protein encoded by the transgene is increased when the transgene is delivered in the presence of the MAPK-ERK pathway inhibitor and of the another type of transduction enhancer in comparison to the percentage of cells expressing that protein when the transgene is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of cells expressing the protein encoded by the transgene is increased when the transgene is delivered in the presence of the MAPK-ERK pathway inhibitor and of the another type of transduction enhancer in comparison to the percentage of cells expressing that protein when the transgene is delivered in the absence of the MAPK-ERK pathway inhibitor and of the another type of transduction enhancer of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1.1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In some embodiments, the term "enhancing functional delivery of a nucleic acid" means that the expression of the protein encoded by the transgene is increased when the transgene is delivered in the presence of the MAPK-ERK pathway inhibitor and of the another type of transduction enhancer in comparison to the expression of that protein when the transgene is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the expression of the protein encoded by the transgene is increased when the transgene is delivered in the presence of the MAPK-ERK pathway inhibitor and of the another type of transduction enhancer in comparison to the expression of that protein when the transgene is delivered in the absence of the MAPK-ERK pathway inhibitor and of the another type of transduction enhancer of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1. 1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1. 1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In certain embodiments, atleast about 10%, about 20%, about 25%, about 30%, about 35%, about 40%, about 45%, about 50%, about 55%, about 60%, about 65%, about 70%, about 75%, about 80%, about 85%, about 90%, or about 95% of the mammalian cells express the protein encoded by the transgene when the transgene is delivered in the presence of the MAPK-ERK pathway inhibitor and of the another type of transduction enhancer.

In further embodiments, the term "enhancing functional delivery of a nucleic acid" means that the percentage of mammalian cells expressing an mRNA encoding a transgene is increased when the transgene is delivered in the presence of the MAPK-ERK pathway inhibitor and of the another type of transduction enhancer in comparison to the percentage of mammalian cells expressing that mRNA when the transgene is delivered in the absence of the MAPK-ERK pathway inhibitor and of the another type of transduction enhancer. In some embodiments, the term "enhancing functional delivery of a nucleic acid" means that the percentage of mammalian cells expressing the mRNA encoding the transgene is increased when the transgene is delivered in the presence of the MAPK-ERK pathway inhibitor and of the another type of transduction enhancer in comparison to the percentage of mammalian cells expressing that mRNA when the transgene is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of mammalian cells expressing the mRNA encoding the transgene is increased when the transgene is delivered in the presence of the MAPK-ERK pathway inhibitor and of the another type of transduction enhancer in comparison to the percentage of cells expressing that mRNA when the transgene is delivered in the absence of the MAPK-ERK pathway inhibitor and of the another type of transduction enhancer of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1.1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In alternative embodiments, the term "enhancing functional delivery of a nucleic acid" means that the expression of the mRNA encoding the transgene in the mammalian cells is increased when the transgene is delivered in the presence of the MAPK-ERK pathway inhibitor and of the another type of transduction enhancer in comparison to the expression of the mRNA encoding the transgene when the transgene is delivered in the absence of the MAPK-ERK pathway inhibitor and of the another type of transduction enhancer of about 1.1 -fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In alternative embodiments, the term "enhancing functional delivery of a nucleic acid" means that the expression of the mRNA encoding the transgene in the mammalian cells is increased when the transgene is delivered in the presence of the MAPK-ERK pathway inhibitor and of the another type of transduction enhancer in comparison to the expression of the mRNA encoding the transgene when the transgene is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1. 1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In certain embodiments, atleast about 10%, about 20%, about 25%, about 30%, about 35%, about 40%, about 45%, about 50%, about 55%, about 60%, about 65%, about 70%, about 75%, about 80%, about 85%, about 90%, or about 95% of the mammalian cells express the mRNA encoding the transgene when the transgene is delivered in the presence of the MAPK-ERK pathway inhibitor and of the another type of transduction enhancer.

In some embodiments, the nucleic acid comprises an inhibitory RNA molecule and the enhanced functional delivery of the nucleic acid results in a decreased expression, i.e. downregulation, of the target protein, i.e. the protein encoded by the target mRNA, into the mammalian cell. In some embodiments, the term "enhancing functional delivery of a nucleic acid" means that the percentage of cells expressing the target protein is decreased when the inhibitory RNA molecule is delivered in the presence of the MAPK-ERK pathway inhibitor and of the another type of transduction enhancer in comparison to the percentage of cells expressing that protein when the inhibitory RNA molecule is delivered in the absence of the MAPK-ERK pathway inhibitor and of the another type of transduction enhancer. In some embodiments, the term "enhancing functional delivery of a nucleic acid" means that the percentage of cells expressing the target protein is decreased when the inhibitory RNA molecule is delivered in the presence of the MAPK-ERK pathway inhibitor and of the another type of transduction enhancer in comparison to the percentage of cells expressing that protein when the inhibitory RNA molecule is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of mammalian cells expressing the target protein is decreased when the inhibitory RNA molecule is delivered in the presence of the MAPK-ERK pathway inhibitor and of the another type of transduction enhancer in comparison to the percentage of cells expressing that protein when the inhibitory RNA molecule is delivered in the absence of the MAPK-ERK pathway inhibitor and of the another type of transduction enhancer of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1.1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In further embodiments, the term "enhancing functional delivery of a nucleic acid" means that the expression of the target protein by the mammalian cells is decreased when the inhibitory RNA molecule is delivered in the presence of the MAPK-ERK pathway inhibitor and of the another type of transduction enhancer in comparison to the expression of the target protein when the inhibitory RNA molecule is delivered in the absence of the MAPK-ERK pathway inhibitor and of the another type of transduction enhancer. In some embodiments, the term "enhancing functional delivery of a nucleic acid" means that the expression of the target protein by the mammalian cells is decreased when the inhibitory RNA molecule is delivered in the presence of the MAPK-ERK pathway inhibitor and of the another type of transduction enhancer in comparison to the expression of the target protein when the inhibitory RNA molecule is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the expression of the target protein by the mammalian cells is decreased when the inhibitory RNA molecule is delivered in the presence of the MAPK-ERK pathway inhibitor and of the another type of transduction enhancer in comparison to the expression of the target protein when the inhibitory RNA molecule is delivered in the absence of the MAPK-ERK pathway inhibitor and of the another type of transduction enhancer of about 1.1 -fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1. 1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In certain embodiments, less than about 5%, about 10%, about 20%, about 25%, about 30%, about 35%, about 40%, about 45%, about 50%, about 55%, about 60%, about 65%, about 70%, about 75%, about 80%, about 85%, or about 90% of the mammalian cells express the target protein when the inhibitory RNA molecule is delivered in the presence of the MAPK-ERK pathway inhibitor and of the another type of transduction enhancer.

In further embodiments, the term "enhancing functional delivery of a nucleic acid" means that the percentage of mammalian cells expressing the target mRNA is decreased when the inhibitory RNA molecule is delivered in the presence of the MAPK-ERK pathway inhibitor and of the another type of transduction enhancer in comparison to the percentage of mammalian cells expressing that mRNA when the inhibitory RNA molecule is delivered in the absence of the MAPK-ERK pathway inhibitor and of the another type of transduction enhancer. In some embodiments, the term "enhancing functional delivery of a nucleic acid" means that the percentage of mammalian cells expressing the target mRNA is decreased when the inhibitory RNA molecule is delivered in the presence of the MAPK-ERK pathway inhibitor and of the another type of transduction enhancer in comparison to the percentage of mammalian cells expressing that mRNA when the inhibitory RNA molecule is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In some embodiments, the term "enhancing functional delivery of a nucleic acid" means that the percentage of mammalian cells expressing the target mRNA is decreased when the inhibitory RNA molecule is delivered in the presence of the MAPK-ERK pathway inhibitor in comparison to the percentage of mammalian cells expressing that mRNA when the inhibitory RNA molecule is delivered in the absence of the MAPK-ERK pathway inhibitor of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1. 1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1. 1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In alternative embodiments, the term "enhancing functional delivery of a nucleic acid" means that the target mRNA expression in the mammalian cells is decreased when the inhibitory RNA molecule is delivered in the presence of the MAPK-ERK pathway inhibitor and of the another type of transduction enhancer in comparison to the target mRNA expression when the inhibitory RNA molecule is delivered in the absence of the MAPK-ERK pathway inhibitor and of the another type of transduction enhancer. In alternative embodiments, the term "enhancing functional delivery of a nucleic acid" means that the target mRNA expression in the mammalian cells is decreased when the inhibitory RNA molecule is delivered in the presence of the MAPK-ERK pathway inhibitor and of the another type of transduction enhancer in comparison to the target mRNA expression when the inhibitory RNA molecule is delivered in the absence of the MAPK-ERK pathway inhibitor and of the another type of transduction enhancer of about 1.1-fold, about 1.2-fold, about 1.3-fold, about 1.4-fold, about 1.5-fold, about 1.8-fold, about 2-fold, about 2.1-fold, about 4-fold, about 5-fold, about 9.6-fold, about 10-fold, about 12-fold, about 15-fold, about 20-fold, about 25-fold, about 30-fold. In alternative embodiments, the term "enhancing functional delivery of a nucleic acid" means that the target mRNA expression in the mammalian cells is decreased when the inhibitory RNA molecule and of the another type of transduction enhancer is delivered in the presence of the MAPK-ERK pathway inhibitor in comparison to the target mRNA expression when the inhibitory RNA molecule is delivered in the absence of the MAPK-ERK pathway inhibitor and of the another type of transduction enhancer of about 1.1-fold to about 30-fold, about 1.2-fold to about 30-fold, about 1.3-fold to about 30-fold, about 1.4-fold to about 30-fold, about 1.5-fold to about 30-fold, about 1.8-fold to about 30-fold, about 2-fold to about 30-fold, about 5-fold to about 30-fold, about 10-fold to about 30-fold, of about 1.1-fold to about 20-fold, about 1.2-fold to about 20-fold, about 1.3-fold to about 20-fold, about 1.4-fold to about 20-fold, about 1.5-fold to about 20-fold, about 1.8-fold to about 20-fold, about 2-fold to about 20-fold, about 5-fold to about 20-fold, about 10-fold to about 20-fold, of about 1.1-fold to about 15-fold, about 1.2-fold to about 15-fold, about 1.3-fold to about 15-fold, about 1.4-fold to about 15-fold, about 1.5-fold to about 15-fold, about 1.8-fold to about 15-fold, about 2-fold to about 15-fold, about 5-fold to about 15-fold, about 10-fold to about 15-fold, of about 1.1-fold to about 12-fold, about 1.2-fold to about 12-fold, about 1.3-fold to about 12-fold, about 1.4-fold to about 12-fold, about 1.5-fold to about 12-fold, about 1.8-fold to about 12-fold, about 2-fold to about 12-fold, about 5-fold to about 12-fold, of about 1. 1-fold to about 10-fold, about 1.2-fold to about 10-fold, about 1.3-fold to about 10-fold, about 1.4-fold to about 10-fold, about 1.5-fold to about 10-fold, about 1.8-fold to about 10-fold, about 2-fold to about 10-fold, about 5-fold to about 10-fold, and preferably of about 1.5-fold to about 20-fold, more preferably of about 1.5-fold to about 10-fold.

In certain embodiments, less than about 5%, about 10%, about 20%, about 25%, about 30%, about 35%, about 40%, about 45%, about 50%, about 55%, about 60%, about 65%, about 70%, about 75%, about 80%, about 85%, or about 90% of the mammalian cells express the target mRNA when the inhibitory RNA molecule is delivered in the presence of the MAPK-ERK pathway inhibitor and of the another type of transduction enhancer.

Definitions given and embodiments described with respect to the first and second aspect apply also to the third aspect, in as far as they are applicable. Also, definitions and embodiments described below, in particular under the header 'Definitions and further embodiments of the invention' apply to the oligonucleotide of the third aspect.

In a fourth aspect, the invention provides a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor, and further comprising the step of contacting the mammalian cell with another type of transduction enhancer prior to, concomitant with or subsequent to contacting of the mammalian cell with the nucleic acid or the vector comprising the nucleic acid. In some embodiments, the invention provides a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor, and further comprising the step of contacting the mammalian cell with another type of transduction enhancer prior to contacting of the mammalian cell with the nucleic acid or the vector comprising the nucleic acid. In some embodiments, the invention provides a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor, and further comprising the step of contacting the mammalian cell with another type of transduction enhancer concomitant with contacting of the mammalian cell with the nucleic acid or the vector comprising the nucleic acid. In some embodiments, the invention provides a method of delivering a nucleic acid into a mammalian cell, comprising the steps of contacting the mammalian cell with the nucleic acid or a vector comprising the nucleic acid and contacting the mammalian cell with a MAPK-ERK pathway inhibitor, and further comprising the step of contacting the mammalian cell with another type of transduction enhancer subsequent to contacting of the mammalian cell with the nucleic acid or the vector comprising the nucleic acid. In preferred embodiments, the invention provides a method of delivering a nucleic acid into a mammalian cell, comprising the steps of contacting the mammalian cell with the nucleic acid or a vector comprising the nucleic acid, contacting the mammalian cell with a MAPK-ERK pathway inhibitor, and further comprising the step of contacting the mammalian cell with another type of transduction enhancer, wherein the steps of contacting with the MAPK-ERK pathway inhibitor and of contacting with another type of transduction enhancer are prior to contacting with the nucleic acid or the vector comprising the nucleic acid.

Exemplary transduction enhancers that may be used in combination with the MAPK-ERK pathway inhibitors disclosed herein are listed in Table 3 below.

Fibronectin and cationic peptides, proteins or polymers are capable of increasing transduction efficiency by physically reducing electrostatic repulsion between the negatively charged target cell and the virion, thereby increasing cell-virion interaction.

Cationic peptides (e.g. LAH4) acting as transduction enhancers have been described by Kichler A, et al., 2003. Histidine-rich amphipathic peptide antibiotics promote efficient DNA delivery into mammalian cells. Proc Natl Acad Sci USA, 100(4):1564-8.

**Table 3: Other transduction enhancers**

| **Substance name** | **Description** |
|---|---|
| 16,16-Dimethyl Prostaglandin E2 | Enhances lentiviral transduction; synthetic prostaglandin E2 derivative |
| Akti-1/2 | Enhances CAR and TCR retroviral transduction of human T cells |
| BX 795 | Enhances lentiviral transduction of NK cells; also PDPK1 and TBK inhibitor |
| Cell penetrating peptide | Enhances delivery of protein, nucleic acids, viral particles and nanocarriers into a mammalian cell |
| Chloroquine diphosphate | Enhances AAV transduction |
| Cyclosporin A | Enhances lentiviral transduction; calcineurin inhibitor |
| Cyclosporin H | Enhances lentiviral transduction |
| Daunorubicin hydrochloride | Enhances adenoviral transduction; RNA synthesis inhibitor |
| Dexamethasone | Enhances retroviral transduction; also anti-inflammatory glucocorticoid |
| Eeyarestatin I | Enhances AAV transduction |
| Etoposide | Enhances adenoviral transduction; topoisomerase II inhibitor |
| Fibronectin | Enhances transduction of hematopoietic stem cells by retroviral vectors |
| Hydroxychloroquine sulfate | Enhances AAV transduction |
| MG 132 | Enhances AAV transduction efficiency of human cell lines |
| Peptide nanofibrils (PNF), e.g. enhancing factor C (EF-C) or Protransduzin^{®} (SEQ ID NO: 1 of WO2014177635A1); vectofusin-1 ^{®} | Enhance transduction by retroviral vectors |
| Polybrene (cationic polymer) | Viral transduction enhancer |
| Prostaglandin E2 | Enhances lentiviral transduction; endogenous prostanoid |
| Protamine sulfate (polycationic protein) | Enhances transduction by retroviral vectors |
| Rapamycin | Enhances lentiviral transduction; mTOR inhibitor and immunosuppressant |
| Recombinant fibronectin fragment, e.g. Retronectin^{®} | Enhances transduction by retroviral vectors |
| Rosuvastatin calcium | Enhances lentiviral transduction of NK cells |
| SAHA | Enhances plasmid transduction; class I and II HDAC inhibitor |
| Semen-derived enhancer of viral infection (SEVI) peptides | Enhances transduction by retroviral vectors |
| Staurosporine | Enhances lentiviral transduction; non-selective protein kinase inhibitor |
| Synthetic soluble short histidine rich cationic peptide (e.g. LAH4) | Enhances transduction by retroviral vectors |
| Teniposide | Viral transduction and DNA transfection enhancer |

| Src/Abl tyrosine kinase inhibitors (TKI) | |
|---|---|
| Bosutinib | 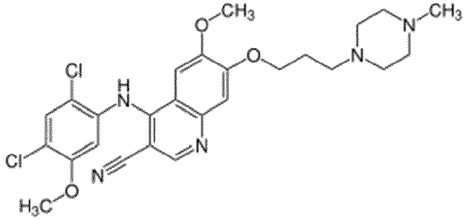 |
| Dasatinib | Enhances lentiviral-mediated gene transfer; potent pan-Src/Bcr-Abl inhibitor |
| KX2-391 (KX01) | 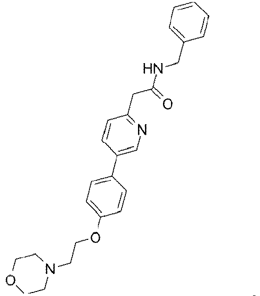 |
| MNS (3,4-Methylenedioxy-β-nitrostyrene, MDBN) | 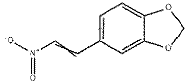 |
| MRL-1023 | 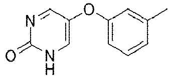 |
| NVP-BHG712 | 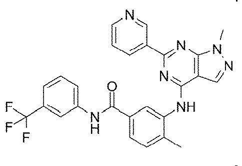 |
| PP1 | 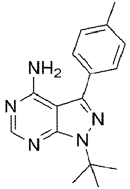 |
| PP121 | 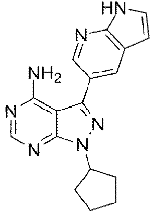 |
| PP2 | 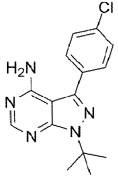 |
| saracatinib | 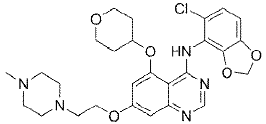 |
| TPX-0005 | 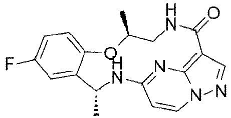 |
| WH-4-023 | 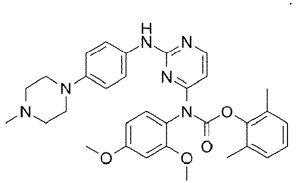 |

Thus, in one embodiment of all aspects disclosed herein, the another type of transduction enhancer is selected from the group consisting of 16,16-Dimethyl Prostaglandin E2, Akti-1/2, BX 795, cell penetrating peptide, chloroquine diphosphate, cyclosporin A, cyclosporin H, daunorubicin hydrochloride, dexamethasone, eeyarestatin I, etoposide, fibronectin, hydroxychloroquine sulfate, MG 132, peptide nanofibrils (PNF), e.g. enhancing factor C (EF-C) or protransduzin^{®} (SEQ ID NO: 1 of WO2014177635A1) and vectofusin-1^{®}; polybrene, prostaglandin E2, protamine sulfate (polycationic protein), rapamycin, recombinant fibronectin fragment, e.g. retronectin^{®}, rosuvastatin calcium, SAHA, semen-derived enhancer of viral infection (SEVI) peptides, sstaurosporine, synthetic soluble short histidine rich cationic peptide (e.g. LAH4), teniposide, a Src/Abl tyrosine kinase inhibitor (TKI). In one embodiment, the TKI is selected from the group consisting of bosutinib, dasatinib, KX2-391 (KX01), MNS (3,4-Methylenedioxy-β-nitrostyrene, MDBN), MRL-1023, NVP-BHG712, PP1, PP121, PP2, saracatinib, TPX-0005, and WH-4-023, preferably dasatinib; or is a hydrate, solvate, or pharmaceutically acceptable salt thereof.

Definitions given and embodiments described with respect to the first, second and third aspect apply also to the fourth aspect, in as far as they are applicable. Also, definitions and embodiments described below, in particular under the header 'Definitions and further embodiments of the invention' apply to the kit of the fourth aspect.

The MAPK-ERK pathway inhibitors described herein are useful for enhancing functional delivery of a nucleic acid not only *ex vivo* or *in vitro* into a mammalian cell but also *ex vivo* or *in vivo* to a subject, to produce cells comprising that nucleic acid to induce, increase or inhibit expression of a protein or peptide.

In a fifth aspect, the invention provides a MAPK-ERK pathway inhibitor for use in enhancing delivering a nucleic acid to a subject.

In one embodiment, enhancing delivering a nucleic acid to a subject comprises delivering a nucleic acid to a mammalian cell *ex vivo* or *in vivo.* In one embodiment, a combination *of ex vivo* and *in vivo* transduction may be used. In one embodiment, enhancing delivering a nucleic acid to a subject comprises delivering a nucleic acid to a mammalian cell *ex vivo,* wherein the mammalian cell is from the same subject, i.e. is autologous to the subject. In one embodiment, enhancing delivering a nucleic acid to a subject comprises delivering a nucleic acid to a mammalian cell *ex vivo,* wherein the mammalian cell is from a different subject, e.g. is allogeneic or syngeneic to the subject. In one embodiment, enhancing delivering a nucleic acid to a subject comprises removal of endogenous cells from a subject, delivering the nucleic acid to that cells, and subsequent transfer of the cells comprising the nucleic acid to the subject. In one embodiment, enhancing delivering a nucleic acid to a subject does not envision removal of cells from the subject. In the latter case all steps of delivery of the nucleic acid into the cells are intended *in vivo.*

The term "autologous" is used herein to refer to cells that are derived from the same subject or individual. The term "allogeneic" is used herein to describe cells that are derived from a different subject of the same species. Two or more subjects are said to be allogeneic to one another if the genes at one or more loci are not identical. The term "syngeneic" is used herein to describe cells derived from a subject or tissue having identical genotype, i.e., identical twins or animals of the same inbred strain, or their tissues.

In one embodiment of the fifth aspect, the invention provides a MAPK-ERK pathway inhibitor for use in combination with another type of transduction enhancer in enhancing delivering a nucleic acid to a subject.

In some embodiments of the fifth aspect, the present invention relates to a method of delivering a nucleic acid to a subject, comprising the steps of (A) contacting a mammalian cell with the nucleic acid or a vector comprising the nucleic acid, (B) contacting the mammalian cell with a MAPK-ERK pathway inhibitor, and (C) transferring the mammalian cell to the subject, wherein the mammalian cell is autologous or allogeneic to the subject. In some embodiments, step (A) is prior to, concomitant with or subsequent to step (B).

In some embodiments of the fifth aspect, the present invention relates to a method of delivering a nucleic acid to a subject, comprising the steps of (A) contacting a mammalian cell with the nucleic acid or a vector comprising the nucleic acid, (B) contacting the mammalian cell with a MAPK-ERK pathway inhibitor at concentration of about 0.001 µM, about 0.005 µM, about 0.01 µM, 0.5 µM, about 1 µM, about 5 µM, about 10 µM, about 15 µM, about 20 µM, about 25 µM, about 30 µM, about 35 µM, about 40 µM, about 45 µM, about 50 µM, about 75 µM, or about 100 µM, preferably about 5 µM, more preferably about 20 µM, and (C) transferring the mammalian cell to the subject, wherein the mammalian cell is autologous or allogeneic to the subject.

In some embodiments of the fifth aspect, the present invention relates to a method of delivering a nucleic acid to a subject, comprising the steps of (A) contacting a mammalian cell with the nucleic acid or a vector comprising the nucleic acid, (B) contacting the mammalian cell with a MAPK-ERK pathway inhibitor at concentration of about 0.5 µM, about 1 µM, about 5 µM, about 10 µM, about 15 µM, about 20 µM, preferably about 5 µM, more preferably about 20 µM, and (C) transferring the mammalian cell to the subject, wherein the mammalian cell is autologous or allogeneic to the subject, and wherein the MAPK-ERK pathway inhibitor is selected from the group consisting of ravoxertinib, ulixertinib, VX-11e, and selumetinib.

In some embodiments of the fifth aspect, the present invention relates to a method of delivering a nucleic acid to a subject, comprising the steps of (A) contacting a mammalian cell with the nucleic acid or a vector comprising the nucleic acid, (B) contacting the mammalian cell with a MAPK-ERK pathway inhibitor at concentration of about 0.001 µM to about 50 µM, about 0.005 µM to about 50 µM, about 0.01 µM to about 50 µM, about 0.05 µM to about 50 µM, about 0.1 µM to about 50 µM, about 0.5 µM to about 50 µM, about 1 µM to about 50 µM, about 5 µM to about 50 µM, about 10 µM to about 50 µM, of about 0.001 µM to about 20 µM, about 0.005 µM to about 20 µM, about 0.01 µM to about 20 µM, about 0.05 µM to about 20 µM, about 0.1 µM to about 20 µM, about 0.5 µM to about 20 µM, about 1 µM to about 20 µM, about 5 µM to about 20 µM, about 10 µM to about 20 µM, of about 0.001 µM to about 15 µM, about 0.005 µM to about 15 µM, about 0.01 µM to about 15 µM, about 0.05 µM to about 15 µM, about 0.1 µM to about 15 µM, of about 0.5 µM to about 15 µM, about 1 µM to about 15 µM, about 5 µM to about 15 µM, about 10 µM to about 15 µM, about 0.5 µM to about 10 µM, about 1 µM to about 10 µM, about 5 µM to about 10 µM, and preferably of about 5 µM to about 20 µM, and (C) transferring the mammalian cell to the subject, wherein the mammalian cell is autologous or allogeneic to the subject

In some embodiments of the fifth aspect, the present invention relates to a method of delivering a nucleic acid to a subject, comprising the steps of (A) contacting a mammalian cell with the nucleic acid or a vector comprising the nucleic acid, (B) contacting the mammalian cell with a MAPK-ERK pathway inhibitor at concentration of about 1 µM to about 20 µM, about 5 µM to about 20 µM, about 10 µM to about 20 µM, about 1 µM to about 15 µM, about 5 µM to about 15 µM, about 10 µM to about 15 µM, about 1 µM to about 10 µM, about 5 µM to about 10 µM, and preferably of about 5 µM to about 20 µM, and (C) transferring the mammalian cell to the subject, wherein the mammalian cell is autologous or allogeneic to the subject, and wherein the MAPK-ERK pathway inhibitor is selected from the group consisting of ravoxertinib, ulixertinib, VX-11e, and selumetinib.

In some embodiments of the fifth aspect, the present invention relates to a method of delivering a nucleic acid to a subject, comprising the steps of (a) administering a nucleic acid or a vector comprising the nucleic acid to the subject and of (b) administering a MAPK-ERK pathway inhibitor to the subject In some embodiments, steps (a) is prior to, concomitant with or subsequent to step (b).

In some embodiments, the present invention relates to a method of delivering a nucleic acid to a subject, comprising the steps of (a) administering a nucleic acid or a vector comprising the nucleic acid to the subject, of (b) administering a MAPK-ERK pathway inhibitor to the subject, and of (c) administering another type of transduction enhancer to the subject. In some embodiments, step (c) is prior to, concomitant with or subsequent to step (b).

Definitions given and embodiments described with respect to the first, second, third and fourth aspect apply also to the fifth aspect, in as far as they are applicable. Also, definitions and embodiments described below, in particular under the header 'Definitions and further embodiments of the invention' apply to the use of the fifth aspect.

In a sixth aspect, the invention provides MAPK-ERK pathway inhibitor for use in enhancing delivering a nucleic acid to a subject, wherein the nucleic acid or a vector comprising the nucleic acid is administered to the subject to treat or prevent a cancer disease, a genetic disease, or a viral infection.

The term "disease" refers to an abnormal condition that affects the body of an individual. A disease may be caused by factors from an external source, such as an infectious disease, or it may be caused by internal dysfunctions, such as genetic diseases. In humans, "disease" is often used more broadly to refer to any condition that causes pain, dysfunction, distress, social problems, or death to the individual, or similar problems to those in contact with the individual. In this broader sense, it sometimes includes injuries, disabilities, disorders, syndromes, infections, isolated symptoms, deviant behaviour, and atypical variations in structure and function, while in other contexts and for other purposes these may be considered distinct categories.

The terms "treat", "treatment", "treating", "therapeutic intervention" or "therapeutic treatment" refer to any treatment that improves the health status and/or prolongs (extends) the life span of an individual. Such treatment may eliminate a disease in an individual, halt, inhibit or slow the development of a disease in an individual, reduce the frequency or severity of symptoms in an individual and/or reduce the recurrence of a disease in an individual who currently has or who previously had a disease.

The terms "prevent", "prevention", "prophylaxis", "prophylactic treatment" or "preventive treatment" are used interchangeably herein and refer to any treatment intended to prevent a disease from occurring in an individual.

In one embodiment of the sixth aspect, the cancer disease is selected from the group consisting of adrenocortical carcinoma, anal cancer, bladder cancer, bone cancer, brain cancer, breast cancer, carcinoid cancer, carcinoma, cervical cancer, colon cancer, endometrial cancer, esophageal cancer, extrahepatic bile duct cancer, extracranial germ cell cancer, eye cancer, gallbladder cancer, gastric cancer, germ cell tumor, gestational trophoblastic tumor, head and neck cancer, hypopharyngeal cancer, islet cell carcinoma, kidney cancer, large intestine cancer, laryngeal cancer, leukemia, lip and oral cavity cancer, liver cancer, lung cancer, lymphoma, malignant mesothelioma, Merkel cell carcinoma, mycosis fungoides, myelodysplastic syndrome, myeloproliferative disorders, nasopharyngeal cancer, neuroblastoma, oral cancer, oropharyngeal cancer, osteosarcoma, ovarian epithelial cancer, ovarian germ cell cancer, pancreatic cancer, paranasal sinus and nasal cavity cancer, parathyroid cancer, penile cancer, pituitary cancer, plasma cell neoplasm, prostate cancer, rhabdomyosarcoma, rectal cancer, renal cell cancer, transitional cell cancer of the renal pelvis and ureter, salivary gland cancer, Sezary syndrome, skin cancers, small intestine cancer, soft tissue sarcoma, stomach cancer, testicular cancer, thymoma, thyroid cancer, urethral cancer, uterine cancer, vaginal cancer, vulvar cancer, and Wilms' tumor.

In one embodiment of the sixth aspect, the cancer disease is selected from the group consisting of acute lymphoblastic leukemia (ALL) (including non T cell ALL), acute lymphoid leukemia (ALL), and hemophagocytic lymphohistocytosis (HLH)), B cell prolymphocytic leukemia, B-cell acute lymphoid leukemia ("BALL"), blastic plasmacytoid dendritic cell neoplasm, Burkitt's lymphoma, chronic lymphocytic leukemia (CLL), chronic myelogenous leukemia (CML), chronic myeloid leukemia (CML), chronic or acute granulomatous disease, chronic or acute leukemia, diffuse large B cell lymphoma, diffuse large B cell lymphoma (DLBCL), follicular lymphoma, follicular lymphoma (FL), hairy cell leukemia, hemophagocytic syndrome (Macrophage Activating Syndrome (MAS), Hodgkin's Disease, large cell granuloma, leukocyte adhesion deficiency, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome (MDS), myeloid diseases including but not limited to acute myeloid leukemia (AML), non- Hodgkin's lymphoma (NHL), plasma cell proliferative disorders (e.g., asymptomatic myeloma (smoldering multiple myeloma or indolent myeloma), plasmablastic lymphoma, plasmacytoid dendritic cell neoplasm, plasmacytomas (e.g., plasma cell dyscrasia; solitary myeloma; solitary plasmacytoma; extramedullary plasmacytoma; and multiple plasmacytoma), POEMS syndrome (Crow-Fukase syndrome; Takatsuki disease; PEP syndrome), primary mediastinal large B cell lymphoma (PMBCL), small cell- or a large cell- follicular lymphoma, splenic marginal zone lymphoma (SMZL), systemic amyloid light chain amyloidosis, T cell acute lymphoid leukemia (TALL), T cell lymphoma, transformed follicular lymphoma, and Waldenstrom macroglobulinemia, or a combination thereof.

In one embodiment of the sixth aspect, the genetic disease is selected from the group consisting of achondroplasia, achromatopsia, acute intermittent porphyria, adenosine deaminase deficiency, adenylosuccinate lyase deficiency, Adrenoleukodystrophy, Alagille syndrome, Alexander disease, alpha-1 antitrypsin deficiency, Alstrom syndrome, Alzheimer's disease, Amelogenesis imperfecta, amyotrophic lateral sclerosis, analbuminaemia, atherosclerosis, Barth syndrome, Batten disease, Becker muscular dystrophies, biotinidase deficiency, cataract, chronic granulomatous disease (CGD), Citrullinemia Type 1, Di George's syndrome, Gaucher's disease, glycogen storage disease (GSD), Sickle Cell Anemia, alpha-1 antitrypsin deficiency, Alzheimer's disease, Barth syndrome, Becker muscular dystrophies, cataract, cystic fibrosis, diabetes, Di George's syndrome, Duchenne Muscular Dystrophy, epilepsy, Epstein-Barr virus associated malignancies, Fabry's disease, familial hypercholesterolemia, fanconi anemia, glycogen storage disease (GSD), G6PD deficiency, hemophilia A, hemophilia B, hereditary angioedema (HAE), hereditary tyrosinemia, 21-hydroxylase deficiency HIV-1, homocystinuria, Huntington's disease, Hurler's disease, late infantile neuronal ceroid lipofuscinosis type 2 (CLN2), Leber Congenital Amaurosis (LCA), lecithin-cholesterol acyltransferase (LCAT) deficiency, lipoprotein lipase deficiency, lysosomal storage disease, in particular aspartylglucosaminuria, Tay-Sachs disease, I-cell disease, Sphingolipidoses such as Krabbe disease, Gaucher, Niemann-Pick disease, Metachromatic leukodystrophy, Lysosomal acid lipase deficiency, Multiple sulfatase deficiency, mucolipidosis types I (sialidosis type I and II), II (I-cell disease), III (pseudo-Hurler disease) and IV, Galactosialidosis, mucopolysaccharide storage diseases (Hurler disease and variants, Hunter, Sanfilippo Types A,B,C,D, Morquio Types A and B, Maroteaux-Lamy and Sly diseases), Niemann-Pick disease, SAP deficiency, and Salla disease; Niemann-Pick disease, ophthalmic or ocular disease, in particular Primary Open-Angle Glaucoma (POAG), lipoprotein lipase deficiency, Menkes disease, metachromatic leukodystrophy, , mucopolysaccharidoses, muscular dystrophy, Neuronal Ceroid Lipofuscinosis Type 2 (CLN2), ophthalmic or ocular disease, in particular Primary Open-Angle Glaucoma (POAG), OTC deficiency (OTCD), retinal degenerative diseases; Parkinson's disease, phenylketonuria, polycystic kidney disease, polycythaemia vera, Pompe disease, Sandhoff disease (GM2-gangliosidosis type II), Schindler disease types I and II, severe combined immunodeficiency (SCID), Sickle cell disease (SCD), Siderius X-linked mental retardation syndrome caused by mutations in the PHF8 gene, Spinal muscular atrophy (SMA), Tay-Sachs disease (GM2-gangliosidosis type I), thalassemia, Usher Syndrome, X-linked adrenoleukodystrophy (X-ALD), X-linked severe combined immunodeficiency (X-SCID), or X-linked sideroblastic anemia (XLSA), and Wilson's disease.

Definitions given and embodiments described with respect to the first, second, third, fourth and fifth aspect apply also to the sixth aspect, in as far as they are applicable. Also, definitions and embodiments described below, in particular under the header 'Definitions and further embodiments of the invention' apply to the method of the sixth aspect.

### Definitions and further embodiments of the invention

The specification uses a variety of terms and phrases, which have certain meanings as defined below. Preferred meanings are to be construed as preferred embodiments of the aspects of the invention described herein. As such, they and also further embodiments described in the following can be combined with any embodiment of the aspects of the invention and in particular any preferred embodiment of the aspects of the invention described above.

The term "about" means approximately or nearly, and in the context of a numerical value or range set forth herein in one embodiment means ± 20%, ± 10%, ± 5%, or ± 3% of the numerical value or range recited or claimed.

The term "plurality" with reference to an object refers to a population of a certain number of said object. In certain embodiments, the term refers to a population of more than 10, 10², 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, 10¹⁷, 10¹⁸, 10¹⁹, 10²⁰, 10²¹, 10²², or 10²³ or more.

The term "heterologous" is used herein to describe a nucleic acid, a gene, a transgene or a protein derived from a source other than the subject.

The term "fragment" with reference to an amino acid sequence (peptide or protein), relates to a part of an amino acid sequence, i.e. a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 50 %, at least 60 %, at least 70 %, at least 80%, at least 90% of the amino acid residues from an amino acid sequence. A fragment of an amino acid sequence preferably comprises at least 6, in particular at least 8, at least 12, at least 15, at least 20, at least 30, at least 50, or at least 100 consecutive amino acids from an amino acid sequence.

By "parent polypeptide", "parent protein", "precursor polypeptide", or "precursor protein" as used in the context of the invention is meant an unmodified polypeptide that is subsequently modified to generate a variant. A parent polypeptide may be a wild type polypeptide, or a variant or engineered version of a wild type polypeptide.

By "wild type" or "WT" or "native" in the context of the invention is meant an amino acid sequence that is found in nature, including allelic variations. A wild type protein or polypeptide has an amino acid sequence that has not been intentionally modified.

By "variant" or "variant protein" or "variant polypeptide" in the context of the invention is meant a protein that differs from a wild type protein by virtue of at least one amino acid modification. The parent polypeptide may be a naturally occurring or wild type (WT) polypeptide, or may be a modified version of a wild type polypeptide. Preferably, the variant polypeptide has at least one amino acid modification compared to the parent polypeptide, e.g. from 1 to about 20 amino acid modifications, and preferably from 1 to about 10 or from 1 to about 5 amino acid modifications compared to the parent.

For the purposes of the present disclosure, "variant" of an amino acid sequence (peptide, protein or polypeptide) comprises amino acid insertion variants, amino acid addition variants, amino acid deletion variants and/or amino acid substitution variants. The term "variant" includes all splice variants, posttranslationally modified variants, conformations, isoforms and species homologs, in particular those which are naturally expressed by cells. The term "variant" includes, in particular, fragments of an amino acid sequence.

Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in peptide and protein variants are conservative amino acid changes, i.e., substitutions of similarly charged or uncharged amino acids. A conservative amino acid change involves substitution of one of a family of amino acids which are related in their side chains. In one embodiment, conservative amino acid substitutions include substitutions within the following groups:
glycine, alanine;
valine, isoleucine, leucine;
aspartic acid, glutamic acid;
asparagine, glutamine;
serine, threonine;
lysine, arginine; and
phenylalanine, tyrosine.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

The term "sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.). The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit according to the disclosure at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook *et al.* (1989), for example. Furthermore, the peptides and amino acid variants described herein may be readily prepared with the aid of known peptide synthesis techniques such as, for example, by solid phase synthesis and similar methods.

In one embodiment, a fragment or variant of an amino acid sequence (peptide or protein) is preferably a "functional fragment" or "functional variant". The term "functional fragment" or "functional variant" of an amino acid sequence relates to any fragment or variant exhibiting one or more functional properties identical or similar to those of the amino acid sequence from which it is derived, i.e., it is functionally equivalent.

An amino acid sequence (peptide, protein or polypeptide) "derived from" a designated amino acid sequence (peptide, protein or polypeptide) refers to the origin of the first amino acid sequence. Preferably, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof.

The term "isolated" means altered or removed from the natural state. For example, a nucleic acid or a peptide naturally present in a living animal is not "isolated", but the same nucleic acid or peptide partially or completely separated from the coexisting materials of its natural state is "isolated". An isolated nucleic acid or protein can exist in substantially purified form, or can exist in a non-native environment such as, for example, a host cell.

The term "recombinant" in the context of the present invention means "made through genetic engineering". Preferably, a "recombinant nucleic acid or protein or cell" in the context of the present invention is not occurring naturally.

The term "naturally occurring" as used in the context of the invention refers to the fact that an object can be found in nature. For example, a peptide or nucleic acid that is present in an organism (including viruses) and can be isolated from a source in nature and which has not been intentionally modified by man in the laboratory is naturally occurring.

As used in the context of the invention, the term "endogenous" refers to any material derived from or produced within an organism, cell, tissue or system. As used herein, the term "exogenous" refers to any material introduced from or produced outside an organism, cell, tissue or system.

As used in the context of the invention, the terms "linked", "fused", or "fusion" are used interchangeably. These terms refer to the joining together of two or more elements or components or domains.

As used in the context of the invention, the terms "protein", "peptide", "polypeptide", "peptides" and "polypeptides" are used interchangeably throughout. These terms refer to both naturally occurring peptides, e.g. naturally occurring proteins and synthesized peptides that may include naturally or non-naturally occurring amino acids. Peptides can be also chemically modified by modifying a side chain or a free amino or carboxy-terminus of a natural or non-naturally occurring amino acid. This chemical modification includes the addition of further chemical moieties as well as the modification of functional groups in side chains of the amino acids, such as a glycosylation. A peptide is a polymer preferably having at least 3, 4, 5, 6, 7, 8, 9, 10, 15, 20, 25, 30, 35, 40, 45, 50, 55, 60, 65, 70, 75, 80, 85, 90, 95, or at least 100 amino acids, most preferably at least 8 or at least 30 amino acids.

An "effective amount" or "therapeutically effective amount" is an amount of a therapeutic agent, such as a therapeutic protein, that is sufficient to achieve the intended purpose. The effective amount of a particular therapeutic agent will vary depending on factors such as the nature of the agent, the route of administration, the size and species of the animal receiving the therapeutic agent, and the purpose of the administration. The effective amount in each individual case may be determined empirically by a skilled artisan in accordance with established methods of the art.

The terms "individual" and "subject" are used interchangeably herein to refer to a human or other mammal (e.g., mouse, rat, rabbit, dog, cat, cattle, swine, sheep, horse, or primate) that may be afflicted with or susceptible to a disease or disorder, but may or may not have the disease or disorder. In preferred embodiments, the subject is a human. Unless otherwise noted, the terms "individual" and "subject" do not denote a specific age and thus include adults, elderly, children, and neonates. In embodiments of the present disclosure, the "individual" or "subject" is a "patient". The term "patient" means an individual or subject to be treated, in particular a diseased individual or subject.

The term "immunotherapy" refers to the treatment of a disease or condition by inducing, or enhancing an immune response. The term "immunotherapy" includes antigen immunization or antigen vaccination. The terms "immunization" or "vaccination" describe the process of administering an antigen to an individual with the purpose of inducing an immune response, for example, for therapeutic or prophylactic reasons.

The invention is described by way of the following examples which are to be construed as merely illustrative and not limiting the scope of the invention, which is limited only by the appended claims.

### EXAMPLES

### Example 1: Materials and Methods

### Cell lines

HEK-293T cells (ATCC CRL-11268) were cultured in Dulbecco's modified Eagle's medium (DMEM) (Sigma-Aldrich, Munich, Germany) supplemented with 10% fetal calf serum (FCS) (Biochrom, Berlin, Germany) and 2 mM glutamine (Sigma-Aldrich). MOLT-4.8, A301, and Jurkat E6.1 cell lines were cultured in RPMI 1640 (Biowest, Nuaillé, France) supplemented with 10% FCS and 2 mM L-glutamine. Cells were incubated at 37°C with 5% CO₂ and 90% humidity.

### Vector production

Lentiviral vectors were generated according to the previously described protocol (Weidner, T et al., 2021. Genetic in vivo engineering of human T lymphocytes in mouse models. Nature Protocols, 16(7), 3210-3240). Briefly, HEK-293T cells were co-transfected with different plasmid cassettes, including a packaging plasmid (pCMVΔR8.9, SEQ ID NO: 1), a transfer plasmid encoding CD19-CAR (pSEW-mycCD19-CAR-28z, PMID: 30224381), and plasmids encoding the corresponding glycoproteins derived from vesicular stomatitis virus (pMD2.G, Addgene Cat No.: 12259), Nipah targeting the CD3z receptor (pCG-NiV-Gmut-L3-hCD3. opt.scFv (Frank, et al., 2020. Combining T cell-specific activation and in vivo gene delivery through CD3-targeted lentiviral vectors, Blood Adv 4 (22): 5702-5715), Nipah targeting the CD8α receptor (pCAGGS-NiV-Gd34-CD8-scFv, pCAGGS-NiV-Fd22 (Bender, RR, et al., 2016. Receptor-targeted nipah virus glycoproteins improve cell-type selective gene delivery and reveal a preference for membrane-proximal cell attachment. PLoS pathogens 12, e1005641)), or measles targeting the CD4 receptor (pCG-Hmut-CD4-DARPin, pCG-FcΔ30 (Zhou, Q, et al., 2015. Exclusive Transduction of Human CD4+ T Cells upon Systemic Delivery of CD4-Targeted Lentiviral Vectors. J Immunol 195: 2493-2501). LVs were harvested and concentrated by centrifugation on a 20% sucrose cushion at 4,500×g (4°C), resuspended in Dulbecco's phosphate buffer saline (DPBS, Mg²⁺ and Ca²⁺ free), aliquoted, and stored at -80°C. Vector particle counts were measured using the NanoSight NS300 (Malvern Panalytical, Malvern, United Kingdom) according to the manufacturer's instructions. Vector titers were determined in transducing units per milliliter (TU/mL) on PBMCs or cell lines. MOLT-4.8 cells were used for titration of CD8-LV and VSV-LV, CD4-LV was titrated on A301 cells, and CD3-LV was titrated on Jurkat E6.1. Vector characteristics used in this study are listed in Table 4 below. Additionally, AAV6 vectors having a GFP coding sequence were used to transduce the donor PBMC. For AAV6 vector production HEK-293 cells were transiently co-transfected with helper plasmids pXX6-80 (SEQ ID NO: 2), packaging pRC26 for AAV6 (SEQ ID NO: 4), and the transfer vector pscAAV-SFFV-GFP (SEQ ID NO: 3).

**Table 4: Characteristics of LVs**

| Sample | VCN* | TU/ml |
|---|---|---|
| CD3-LV | D1: 0.94 D2: 0.62 | 3.49E+06 |
| CD4-LV | D1: 0.74 D2: 0.90 | 4.66E+06 |
| CD8-LV | D1: 0.92 D2: 0.72 | 4.42E+06 |
| VSV-LV | D1: 3.16 D2: 1.59 | 1.67E+09 |

| | | |
|---|---|---|
| VCN: Vector copy number; TU: transducing units T cell isolation, in vitro transduction | | |

Human PBMCs were isolated from peripheral blood buffy coats of anonymous donors obtained from the German Red Cross (DRK-Blutspendedienst Baden-Württemberg-Hessen, Frankfurt, Germany) by density gradient centrifugation with Pancoll (PAN-Biotech, Aidenbach, Germany). Purified PBMCs were cryopreserved in 90% FCS and 10% dimethyl sulfoxide (DMSO, AppliChem GmbH, Darmstadt, Germany) and cultured in T cell medium (RPMI 1640, 10% FCS, 2 mM L-glutamine, 25 mM HEPES, 0.4% penicillin/streptomycin) or in 4Cell^{®} Nutri-T medium (Sartorius AG, Göttingen, Germany) *plus* 0.4% penicillin/streptomycin. For transduction, PBMCs were activated for 3 days in plates precoated with recombinant anti-CD3 (1 µg/mL, clone OKT3, Miltenyi Biotec, Bergisch Gladbach, Germany) and soluble anti-CD28 (3 µg/mL, clone 15E8, Miltenyi Biotec) supplemented with human cytokines IL-7 (25 IU/mL, Miltenyi Biotec) and IL-15 (50 IU/mL, Miltenyi Biotec). Activated T cells were seeded into flat-bottomed 96-well plates at a density of 6-8×10⁴ cells per well. After addition of vector particles, the cells were centrifuged at 850×g, 32°C, for 90 min. Inoculation with CD3-LV vector for single cell analysis was performed in the presence of 50 nM dasatinib as previously described (Braun et al., 2023. Dasatinib is a potent enhancer for CAR T cell generation by CD3-targeted lentiviral vectors. Molecular Therapy. Methods & Clinical Development, 28, 90-98). For transduction enhancement experiments, different concentrations of deucravacitinib (BMS-986165), ulixertinib, ravoxertinib, selumetinib, VX-11e (all from MedChemExpress) were used. Activated cells were incubated for 1 hour with each of the compounds and added of CD3-LV (2000 particles per cell), CD4-LV (2000 particles per cell), CD8-LV (2000 particles per cell), VSV-LV (500 particles per cell), or AAV6 (incorporating a GFP coding sequence, 16000 genome copies per cell). Thereafter, the cells were centrifuged at 850×g, 32°C, for 90 min and incubated for 24 hours before changed the media to fresh T cell medium. All compounds were reconstituted in DMSO, and DMSO concentration reached below 0.4% in cell culture. 72 hours after transduction, cells were analyzed by flow cytometry.

### Flow cytometry analysis

Cells for flow cytometry analysis were washed twice with wash buffer (PBS, 2% FBS, 2 mM NaN₃), and stained for 30 min at 4°C with the appropriate antibody mix. Following antibodies were used for the determination of CAR expression: anti-myc [FITC]clone SH1-26e7.1.3 (Miltenyi), targeted vector particle binding: anti-His [PE] clone GG11-8F3.5.1 (Miltenyi), VSV-LV particles binding: anti-VSV-G [pure] clone 8G5F11 (Absolute Antibody) and goat anti-mouse IgG [Alexa Fluor 647] polyclonal (Jackson ImmunoResearch), and T cell characterization: anti-CD4 [PE-Cy7] clone SK3; anti-CD3 [PerCP-Cy5.5] clone UCHT1; anti-CD8 [BV510] clone SK1; anti-CD8 [VioBlue] clone REA734, anti-CD3 [PerCP] clone BW264/56 (Miltenyi Biotec). T cell viability was determined by the addition of fixable viability dye eFluor780 (Thermo Fisher Scientific). After staining, cells were washed twice with wash buffer and fixed with PBS containing 1% paraformaldehyde. The samples were measured with MACSQuant Analyzer 10 (Miltenyi Biotec) or FACSymphony^{™} A3 Cell Analyzer (BD Biosciences), and the results were analyzed using FlowJo v.10.1 (BD Biosciences) or FCS Express 6 (De Novo Software).

### AbSeqs and multiplexing sample tags

Antibody-oligonucleotide conjugates (AbSeqs) were used to detect surface proteins in single-cell sequencing. The antibodies to detect CAR, targeted vector particles, and VSV-LV vector particles listed in the flow cytometry section above were purchased from the respective companies and sent to BD Biosciences for custom barcoding. In addition, the BD AbSeq immune discovery panel (Cat. No. 625970) was used. The BD Single-Cell Multiplexing Kit (Cat. No. 633781) was used to combine multiple samples on a single BD Rhapsody^{™} Cartridge for simultaneous processing. Staining of cells with AbSeqs before single-cell isolation was performed according to the protocols provided by BD Biosciences.

### Single-cell multi-omics sequencing

T cells from two healthy donors were transduced with four different vector types, harvested 3 days after vector addition, and processed for single-cell isolation using the BD Rhapsody microwell-based system. Prior to single-cell analysis, all samples were enriched for viability using the Dead Cell Removal Kit (Miltenyi Biotec). In addition, the Pan T cell isolation kit (Miltenyi Biotec) was used for negative magnetic selection of CD3 T cells in the CD3-LV and VSV-LV treated groups, and the CD4 or CD8 isolation kits (Miltenyi Biotec) were used to enrich for CD4 or CD8 T cells in the CD4-LV and CD8-LV treated groups, respectively. Cells were labeled with AbSeqs and multiplexing sample tags and were mixed and loaded into three cartridges. Cellular mRNA, AbSeqs, and sample tag barcodes captured on poly(dT)-coated magnetic beads were reverse transcribed according to the manufacturer's protocol. The cDNA was amplified using the immune response panel (Cat. No. 633750, BD Biosciences) and a panel of complementary custom primers to detect the CAR gene and several other genes of interest as listed in the Table 4 below. The custom primers were designed and supplied by BD Biosciences. Libraries generated for different samples in a multiplex approach were sequenced on Illumina NextSeq^{™} 2000.

**Table 4: Supplementary genes added to the targeted panel**

| Gene | Annotation |
|---|---|
| IFI6 | Type I interferon signaling |
| LCP2 | T cell receptor (TCR) signaling |
| LDHA | Glycolysis |
| MX1 | Type I interferon signaling |
| MYB | Regulation of proliferation, differentiation, apoptosis |
| NOTCH1 | Notch signaling |
| OAS2 | Type I & II interferon signaling |
| PSAT1 | Glutamine metabolism |
| PSMA3 | Cell cycle, cell signaling, DNA repair |
| RASA2 | Cellular proliferation and differentiation |
| SAMHD1 | Type I interferon signaling |
| SLC1A5 | Amino acid transport |
| SLC3A2 | Amino acid transport |
| SLC7A5 | Amino acid transport |
| TFRC | Iron uptake by the process of receptor-mediated endocytosis |
| TRIM22 | Type I & II interferon signaling |
| TRIM25 | Type I & II interferon signaling |
| UQCRQ | Oxidative phosphorylation |
| XCL1 | Chemokine signaling |
| LDLR | Receptor-mediated endocytosis |

### Data analysis

The raw reads were aligned to the reference sequences using the Seven Bridges Genomics platform and the RSEC corrected count matrices were used for further data analysis. The SeqGeq^{™} v1.8 software (BD Life Sciences) and the Lex Plugin tool v1.1 were used to demultiplex the data into representative samples based on sample tag calls.

Bioinformatic analysis was performed in R Studio (R 4.2.1) using the Seurat package (Hao Y et al., 2021. Integrated analysis of multimodal single-cell data. Cell, 184(13), 3573-3587.e29). Low quality cells were filtered based on UMI counts per cell. Reads were normalized using the NormalizeData function. Log Normalize and CLR methods were used to normalize RNA and AbSeq reads, respectively. Both RNA and AbSeq data were used to generate wnnUMAP plots. Multimode analysis was performed to define the positive and negative cells based on the signal for CAR at the RNA level, or CARp (referred to as CAR detection with AbSeq), VSV-G, his-tag, CD4 and CD8 on the AbSeq level (Figure 2B) (Charitidis FT et al., 2021. Monitoring CAR T cell generation with a CD8-targeted lentiviral vector by single-cell transcriptomics. Molecular Therapy - Methods and Clinical Development, 23). Analysis of differentially expressed genes between the populations of interest was performed using the FindMarkers function with the Wilcoxon Rank Sum test. False discovery rate (FDR) was calculated by Benjamini and Hochberg procedure (Benjamini, Y and Hochberg Y, 1995a. Controlling the False Discovery Rate: A Practical and Powerful Approach to Multiple Testing. Journal of the Royal Statistical Society: Series B (Methodological), 57(1), 289-300). RNA or AbSeqs with log2(fold-change) greater than 0.25 or lower than -0.25 and FDR < 0.05 were considered to be differentially expressed.

### Analysis of single-cell RNA sequencing dataset from patient samples

Publicly available single-cell RNA sequencing data of autologous axicabtagene ciloleucel (axi-cel) anti-CD19 CAR T cell infusion products for 24 patients with LBCL were analyzed. Filtering and pre-processing of data were done according to the criteria defined in the original study (Deng et al., 2020. Characteristics of anti-CD19 CAR T cell infusion products associated with efficacy and toxicity in patients with large B cell lymphomas. Nature Medicine, 26(12), 1878-1887).

Positive and negative population of CD4 or CD8 CAR expressing T cells were defined based on the expression of CD19-scFv, with the multimode package. FindMarkers function from Seurat package was used to find differentially expressed genes. False discovery rate (FDR) by Benjamini and Hochberg (Benjamini Y and Hochberg Y, 1995b. Controlling the False Discovery Rate: A Practical and Powerful Approach to Multiple Testing. Journal of the Royal Statistical Society: Series B (Methodological), 57(1), 289-300) was calculated, and genes with absolute log2 (fold-change) greater than 0.25 and FDR < 0.05 were considered as differentially expressed. Volcano plots were constructed with the EnhancedVolcano package. Genes of interest are shown in violin plots, for which Kruskal-Wallis and/or Wilcoxon tests were performed, and p-values were adjusted according to Benjamini and Hochberg (FDR).

### Statistical analysis

Numeric values obtained from expression experiments were analyzed and plotted with GraphPad Prism 9 (GraphPad Software Inc., CA, USA). Mean values for technical replicates of each condition were calculated and included in the analysis. Statistical differences were assessed with unpaired t-test.

### Example 2: Detection and separation of vector-bound CAR^{neg} cells

Using either conventional or targeted LVs, the inventors tested whether all T cells that became bound by vector particles get transduced or whether a significant cell population exists that despite being attached to LVs does not express the CAR. To investigate this, the amounts of vector particles detectable at the cell surface were analysed at different time points after vector addition. Analysis of several donors and the four vector types VSV-LV, CD3-LV, CD4-LV, and CD8-LV revealed for all settings the highest values at four hours and then a gradual decrease over the next days with no detectable vector particles after 96 hours (Figure 1A). Signals for CAR expression came up after 24 hours and reached maximum levels between 48 and 72 hours (Figure 1B). When quantifying both signals at these time points, three cell populations could be clearly distinguished, i.e. CAR^{pas} T cells (still with or already without detectable vector), cells negative for both signals, and a significant fraction of cells ranging between 9% and 85% that had vector particles bound but showed no CAR signal (Table 5 below and Figure 1B). This picture was consistent for several donors and all vector types with some quantitative differences. Furthermore, significant variation in the percentages of these populations was observed between donors, suggesting inherent differences between them. These data demonstrated that only a fraction of T cells bound to vector particles will convert to CAR T cells.

**Table 5: Percentages of different T cell subsets 3 days after transduction**

| | CD3-LV | | | CD4-LV | | | CD8-LV | | | VSV-LV | | |
|---|---|---|---|---|---|---|---|---|---|---|---|---|
| Donors | D1 | D2 | D3 | D1 | D2 | D3 | D1 | D2 | D3 | D1 | D2 | D3 |
| CAR^{neg}Vector^{neg} | 38.1 | 50.3 | 34.5 | 14.5 | 27.5 | 39.9 | 10.2 | 52.6 | 40.7 | 36.7 | 18.7 | 14.5 |
| CAR^{neg}Vector^{pos} | 46.5 | 39.6 | 36.6 | 80.7 | 65.3 | 26.4 | 84.8 | 40.3 | 10.6 | 11.9 | 58.7 | 8.5 |
| CAR^{pos}Vector^{pos} | 9.3 | 6.0 | 15.6 | 4.6 | 6.8 | 16.8 | 4.6 | 4.3 | 16.2 | 21.8 | 21.6 | 35.3 |
| CAR^{pos}Veotor^{neg} | 6.1 | 4.1 | 13.4 | 0.3 | 0.4 | 17.0 | 0.4 | 2.8 | 32.5 | 29.6 | 0.9 | 41.7 |

### Example 3 Single-cell multi-omics analysis

The observed heterogeneity of T cell populations during CAR T cell generation prompted the inventors to determine the gene and protein expression profiles of these different T cell subsets. A targeted multi-omics single-cell (sc) analysis was performed on activated PBMCs from two healthy donors incubated with VSV-LV or each of the targeted CD3-LV, CD4-LV, and CD8-LV encoding the anti-CD19 CD28/CD3 zeta chimeric antigen receptor for 72 hours before being processed for sc analysis (Figure 2A and 2B). The Seurat integration workflow was used to combine all samples, and after integration, no significant donor or batch effects were observed in the dataset (Figure 3). Cells were evenly distributed across all clusters, regardless of vector type and donor (Figure 3B). CD4⁺ and CD8⁺ T cells were accurately annotated using the CD4 and CD8 AbSeq signals (Figure 3C). Notably, these cell populations showed a clear and pronounced separation within the two primary clusters, with CD4 T cells predominantly located in the left clusters and CD8 T cells in the right clusters of the UMAP plot. CD3-LV and VSV-LV samples were present in both CD4 and CD8 T cell clusters. As expected, CD8-LV samples were predominantly present in clusters characterized by CD8 T cells, whereas CD4-LV samples showed a predominant presence in clusters associated with CD4 T cells (Figure 3B).

CAR expressing and LV-bound T cells were identified at single-cell resolution by detecting *CAR* mRNA and AbSeqs specific for the CAR and the vector envelope proteins (Figure 4A, 4B, 4C). Comparison of the percentages of cells positive for CAR protein (referred to as CARp) and LV binding showed perfect agreement between AbSeq and flow cytometry data (Figure 4A). This demonstrated the validity of the approach to separate CAR expressing and LV-bound T cells. The percentages of *CAR*^{pos} cells determined on mRNA level were throughout all samples higher than those determined on protein level by AbSeq or flow cytometry (Figure 4A). The further experiments aimed to analyze the differentially expressed genes between *CAR*^{pos}/CARp^{neg} T cells (positive at the RNA level and negative for protein) and *CAR*^{pos}/CARp^{pos} T cells (positive for both RNA and protein) from each vector type and look into the commonly upregulated and downregulated genes (Figure 5). Genes upregulated in the *CAR*^{pos}/CARp^{pos} T cells included T cell exhaustion markers LAG3, IER3, and CD70. These differential expression patterns in CARp^{pos} cells were likely due to the tonic signaling activity of the CAR molecules, defined as ligand-independent activation and signaling. Besides exhaustion markers, POU2AF1, encoding a positive regulator of transcription by RNA polymerase II and SLC7A5, encoding for a high-affinity transporter responsible for the uptake of large neutral amino acids, were both upregulated in *CAR*^{pos}/CARp^{pos} cells (Figure 5).

Further analysis of the distribution of cells being CARp and/or LV-positive showed a strong vector binding signal distributed over several clusters (Figure 4D). In contrast, CARp was more restricted to a few clusters, revealing a high number of cells exhibiting vector binding signals without a concomitant CARp signal. This pattern was consistently observed in all samples (Figure 4D).

### Example 4: CAR^{neg} vector bound T cells show upregulation of genes with antiviral activity

To investigate potential factors influencing the transduction of T cells and to understand why some T cells despite being attached to LVs fail to become CAR^{pos}, the inventors compared the expression profiles of T cells positive for vector particles but negative for CAR (*CAR*^{neg}/vector^{pos}) with *CAR*^{pos} T cells for all LV-treated samples (Figure 6). *CAR*^{neg}/vector^{pos} T cells showed upregulation of several ISGs in each of the vector-treated groups. The CD3-LV group showed upregulation of interferon-induced transmembrane proteins, *IFITM2* and *IFITM3* (Figure 6A), while the VSV-LV group showed upregulation of *IFITM3* and *APOBEC3G* (Figure 6D). The CD4-LV and CD8-LV groups showed upregulation of *IFITM2*, *IFITM3*, *STAT1*, *SAMHD1*, *MX1, IFI6,* and *ADAR* (Figures 6B and C). Among other upregulated genes, there were genes involved in antigen processing and presentation, specifically HLA molecules (Figure 6). The VSV-LV group had the highest number of these genes being upregulated, including *HLA-DQB1*, *HLA-DRA*, *HLA-DMA*, *HLA-DPA1*, as well as *CD74,* which functions as a chaperone protein that assists in the assembly and trafficking of major histocompatibility complex class II (MHC II) (Figure 6D).

The gene expression patterns of these genes were then compared in three different cell subsets defined by CARp expression and vector binding detected by AbSeqs (Figure 7A). This analysis revealed a prominent pattern of up- and downmodulation of most of these genes between the three subsets. In CARp^{neg}/vector^{pos} T cells (population b) ISGs usually reached the highest level (Figure 7A-B). *IFITM2*, *IFITM3*, *and ADAR* were among the upregulated genes in population b across all vector types. Upregulation of *STAT1*, *SAMHD1* and *MX1* was also observed in population b in all target LVs. The genes *OAS2, IFI6* and *TRIM22* were also upregulated in population b, but with some exceptions. Specifically, *OAS2* was upregulated by CD4-LV, *IFI6* by CD4-LV and CD8-LV, and *TRIM22* by CD3-LV and CD8-LV. In addition, *APOBEC3G* was upregulated in population b of the VSV-LV group. When analyzing the expression patterns of these genes among the three different subsets defined in this study, a trend was observed in which the expression of these genes was low in subsets a (CARp^{neg}/vector^{neg}) and c (CARp^{pos}), but high in subset b (CARp^{neg}/vector^{pos}). This expression pattern was particularly evident in the CD4-LV and CD8-LV where it manifested for all ISGs except *APOBEC3G* (Figure 7B).

In the CD3-LV group, the genes *STAT1*, *SAMHD1, ADAR,* and *TRIM22* adhered to this pattern, while in the VSV-LV group only the *APOBEC3G* gene followed this pattern (Figure 7). These observations suggest that the expression of these genes is stimulated in T cells upon binding to LVs, and that this upregulation is somehow negatively regulated in CAR T cells. Further investigation of the genes that did not show significant upregulation in the CARp^{neg}/vector^{pos} T cells of the VSV-LV group, such as *STAT1*, *SAMHD1, IFI6,* and *MX1*, revealed that the lack of upregulation was primarily influenced by the individual donor (Figure 8). *STAT1* upregulation was masked when two donors were pooled, whereas upregulation of this gene was seen in population b of each donor separately (Figure 8). In contrast to donor 1, *MX1, IFI6* and *SAMHD1* were found to be upregulated in donor 2 in response to VSV-LV treatment (Figure 8). This difference between the two donors correlated with a higher CAR expression level in donor 1 compared to donor 2 (Figure 2). Taken together, these results suggest that antiviral ISGs are stimulated upon attachment of viral vector particles to T cells and then negatively affect successful transduction.

### Example 5: Consistent upregulation of DUSP2 and DUSP4 in CAR T cells

Next, the inventors investigated the genes that were consistently upregulated in *CAR*^{pos} T cells (subset c) across all vector treated groups. A comprehensive overlap analysis identified 11 common genes (Figure 9A). Notably, *CAR*^{pos} T cells generated with VSV-LV exhibited the highest number of unique upregulated genes, 25 in total, compared to the targeted vectors. Some of the genes that were universally upregulated in *CAR*^{pos} T cells are known as T cell activation and exhaustion markers, including *CD2, PRDM1, JUNB, LAG3, MYB, CTLA4, ZBED2,* and *CSF2* (GM-CSF), which exhibited the highest log2 fold-change after the *CAR* gene. Two genes which have not yet been studied in context of CAR T cells are *DUSP2* and *DUSP4,* which both were highly upregulated in subset c.

### Example 6: DEG from patient CAR T cells are consistent with healthy donors

To validate the initial observations of differentially expressed genes in cell subsets during the generation of CAR T cells from healthy donors, data from patient-derived CAR T cells were analyzed next. To this aim whole transcriptome single-cell RNA sequencing data from axicabtagene ciloleucel (axi-cel) anti-CD19 CAR T cell infusion products originating from 24 patients diagnosed with large B cell lymphoma were used; the transcriptome data of these CAR T cells were obtained almost two weeks after exposure to LV particles (Deng *et al.*, 2020). In total, 76 and 25 genes were differentially expressed between *CAR*^{pos} and *CAR*^{neg} cells in CD4 and CD8 T cells, respectively (Figure 10A). In CD4 and CD8 *CAR*^{neg} T cells, upregulation of *IFITM1* and *IFITM2* was observed. *ISG20,* another interferon-stimulated gene, was upregulated in the CD4 T cells (Figure 10A). In agreement with the healthy donor data, HLA molecules were also among the upregulated genes in both CD4 and CD8 *CAR*^{neg} T cells from patients (Figure 10A). In the CD4 *CAR*^{neg} T cells, there was an upregulation of *HLA-A, HLA-B, HLA-C,* and *HLA-E* (Figure 10A), and *HLA-B, HLA-DQA1,* and *HLA DQA2* were upregulated in the CD8 *CAR*^{neg} cells (Figure 10A). Importantly, *DUSP2* and *DUSP4* were up in *CAR*^{pos} T cells compared to *CAR*^{neg} T cells of most patients (Figure 10B and 10C). Overall, this analysis supported the findings of differentially expressed genes in the two healthy donors and demonstrated that these are relevant also for CAR T cell products used in clinics.

### Example 7: Enhancing lentiviral vector transduction by down-modulating interferon signalling

The findings of Example 4 suggest that the inhibition of interferon signaling pathways could improve CAR T cell generation. TYK2 (tyrosine kinase 2) is a protein kinase that plays a key role in the type-I interferon signaling pathway, and can be targeted to block the interferon signaling pathway and inhibit the expression of ISGs. Deucravacitinib (BMS-986165) is a highly selective, orally bioavailable allosteric TYK2 inhibitor, and was used in these experiments to inhibit TYK2 activation. Activated PBMCs were treated with different concentrations of the compound one hour prior to incubation with CD3-LV, CD4-LV, CD8-LV, and VSV-LV. Upon treatment of cells with deucravacitinib for 24 hours, 400 nM was found to be the optimal dose (Figure 11A). Notably, deucravacitinib at various doses had no negative effect on T cell viability in all vector-treated groups compared to untreated samples (Figure 11C, 11D). Treatment with deucravacitinib resulted in an increase in CAR expression for an average of up to 1.7-fold for CD4-LV, and 3.6-fold for CD8-LV (Figure 11A &11E) without compromising viability (Figure 11D). In general, these results show that TYK2 inhibition has a beneficial though moderate effect on transduction without compromising T cell viability. This provides further support for interferon signaling having an inhibitory effect on lentiviral-mediated transduction in T cells.

### Example 8: Enhancing transduction through modulation of ERKs

In the previous examples, CAR expressing T cells showed upregulation of *DUSP2* and *DUSP4* genes in single-cell analysis for all samples (Figure 9A, 9B, 10B and 10C). Both DUSP2 and DUSP4 function as phosphatases that are negative regulators of MAPK signaling, specifically dephosphorylating and inactivating extracellular signal-regulated kinases (ERKs), including ERK1 and ERK2. Highly selective inhibitors of ERK2 were used to mimic the role of DUSP2 and DUSP4 in cells and to evaluate their potential beneficial effects on transduction. The tested inhibitors were ravoxertinib, ulixertinib, VX-11e, which directly inhibit ERK2, and selumetinib, which inhibits the kinases (MEK1 and 2) upstream of ERK2. Different doses of all compounds increased transduction and CAR expression in CD4-LV, CD8-LV, and VSV-LV. However, there were variations in the extent of enhancement among the different LVs in response to each compound (Figure 13A and 12A). 20 µM of VX-11e and 5 µM of ulixertinib were identified as optimal doses and were subsequently tested in additional transduction experiments. Transduction efficiency enhancement refers to the n-fold increase of CAR expression by T cells when transduction occurs in the presence of the ERK2 inhibitor in comparison to CAR expression by T cells when transduction occurs in the absence of that ERK2 inhibitor. Both compounds enhanced the transduction efficiency of targeted vectors resulting in an average increase of up to 9.4-fold, 4-fold, and 9.6-fold for CD3-LV, CD4-LV, and CD8-LV respectively (Figure 13B and 13C). VX-11e also increased the VSV-LV mediated transduction efficiency by an average of up to 1.8-fold (Figure 13B and 13C), and the AAV6 mediated transduction efficiency by an average of up to 2.1-fold compared to untreated cells (Figure 14). Overall, the use of these compounds increased transduction with all types of LVs without adversely affecting cellular viability (Figure 12B) nor the exhaustion profile of T cells as evidenced by the expression level of LAG3 (Figure 12C). The observed results provide evidence for the role of *DUSP2* and *DUSP4* genes in enhancing transduction and the negative role of ERK2 activation in T cell transduction.

## Claims

1. Use of a MAPK-ERK pathway inhibitor for enhancing functional delivery of a nucleic acid into a mammalian cell.

2. Method of delivering a nucleic acid into a mammalian cell, comprising the steps of contacting the mammalian cell with the nucleic acid or a vector comprising the nucleic acid and contacting the mammalian cell with a MAPK-ERK pathway inhibitor.

3. The method according to claim 2, wherein the contacting the mammalian cell with a MAPK-ERK pathway inhibitor is prior to, concomitant with or subsequent to the contacting with the nucleic acid or the vector comprising the nucleic acid.

4. The use according to claim 1 or the method according to claims 2 or 3, wherein the MAPK-ERK pathway inhibitor is an inhibitor of RAS, in particular K-RAS; RAF, in particular B-RAF; ERK1/ERK2; or MEK1/MEK2.

5. The use or the method according to claim 4, wherein the:
(i) inhibitor of RAS is selected from the group consisting of AMG-510, MRTX 849, BI 1701963 and BBP-454; or is a hydrate, solvate, or pharmaceutically acceptable salt thereof;
(ii) inhibitor of RAF is selected from the group consisting of dabrafenib, encorafenib, vemurafenib, FORE-8394 (PLX-8394), tinloragenib, AZ-304, agerafenib, KIN-2787, BGB-3245, ABM-1310, TQB-3233, UB-941, AFX-1251, ARQ 736, ASN003, AVB-BRAF, BDTX-4933, CCT196969, CFT1946, HLX208, RO5212054, RO7276389, and LY3009120; or is a hydrate, solvate, or pharmaceutically acceptable salt thereof;
(iii) inhibitor of ERK1/ERK2 is selected from the group consisting of ulixertinib, ravoxertinib, VX-11e, SCH772984, LY3214996, HH-2710, ASTX029, ATG-017, JSI-1187, JRP-890, JRF-108, and MK-8353, preferably ravoxertinib, ulixertinib, VX-11e; or is a hydrate, solvate, or pharmaceutically acceptable salt thereof; and
(iv) inhibitor of MEK1/MEK2 is selected from the group consisting of selumetinib, trametinib, cobimetinib, binimetinib, PD-325901, and CI-1040, preferably selumetinib; or is a hydrate, solvate, or pharmaceutically acceptable salt thereof.

6. The use according to any one of claims 1, 4 or 5 or the method according to any one of claims 2 to 5, wherein the mammalian cell is a blood cell, preferably selected from the group consisting of a B cell, a dendritic cell, a hematopoietic stem cell, a macrophage, a monocyte, a NKT cell, a NK cell, a plasmablast, and a T cell.

7. The use according to any one of claims 1, or 4 to 6 or the method according to any one of claims 2 to 6, wherein the nucleic acid is a dsDNA, in particular a plasmid, or a cosmid, ssDNA or RNA, and preferably the dsDNA, or ssDNA comprises at least one transgene, and preferably the RNA comprises an mRNA, more preferably an mRNA encoding a transgene, miRNA, DsiRNA or a siRNA.

8. The use or method according to claim 7, wherein the transgene encodes for a CAR, a TCR or subunit thereof, or a therapeutic protein, preferably a vaccine antigen, an antibody, an enzyme, a growth factor, or a cytokine.

9. The use according to any one of claims 4 to 8 or the method according to any one of claims 2 to 8, wherein the vector comprising the nucleic acid is selected from the group comprising a viral vector and a nanocarrier, preferably a lipid nanoparticle, and wherein the viral vector is preferably selected from the group comprising a lentiviral vector, an adeno-associated viral vector, in particular AAV6 or AAV2, an adenoviral vector, a retroviral vector, and a herpes simplex virus, and wherein the viral vector is preferably a pseudotyped and/or targeted viral vector, more preferably a CD3-, CD4-, CD62L-, or CD8-targeted lentiviral vector.

10. The use according to any one of claims 1, 4 to 9, wherein the MAPK-ERK pathway inhibitor is used in combination with another type of transduction enhancer.

11. The method according to any of claims 2 to 9, comprising the further step of contacting the mammalian cell with another type of transduction enhancer prior to, concomitant with or subsequent to contacting of the mammalian cell with the nucleic acid or the vector comprising the nucleic acid.

12. The use according to claim 10 or the method according to claim 11, wherein the another type of transduction enhancer is selected from the group consisting of16,16-Dimethyl Prostaglandin E2, Akti-1/2, BX 795, cell penetrating peptide, chloroquine diphosphate, cyclosporin A, cyclosporin H, daunorubicin hydrochloride, dexamethasone, eeyarestatin I, etoposide, fibronectin, hydroxychloroquine sulfate, MG 132, peptide nanofibrils (PNF), e.g. enhancing factor C (EF-C) or protransduzin^{®} and vectofusin-1^{®}; polybrene, prostaglandin E2, protamine sulfate (polycationic protein), rapamycin, recombinant fibronectin fragment, e.g. retronectin^{®}, rosuvastatin calcium, SAHA, semen-derived enhancer of viral infection (SEVI) peptides, sstaurosporine, synthetic soluble short histidine rich cationic peptide (e.g. LAH4), teniposide, and a Src/Abl tyrosine kinase inhibitor (TKI), and wherein the TKI is preferably selected from the group consisting of bosutinib, dasatinib, KX2-391 (KX01), MNS (3,4-Methylenedioxy-β-nitrostyrene, MDBN), MRL-1023, NVP-BHG712, PP1, PP121, PP2, saracatinib, TPX-0005, and WH-4-023, preferably dasatinib; or is a hydrate, solvate, or pharmaceutically acceptable salt thereof.

13. A MAPK-ERK pathway inhibitor for use in enhancing delivering a nucleic acid to a subject.

14. The MAPK-ERK pathway inhibitor for use according to claim 13, wherein the nucleic acid or a vector comprising the nucleic acid is administered to the subject to treat or prevent a cancer disease, a genetic disease, or a viral infection.

15. The MAPK-ERK pathway inhibitor for use according to claim 14, wherein the cancer disease is selected from the group consisting of adrenocortical carcinoma, anal cancer, bladder cancer, bone cancer, brain cancer, breast cancer, carcinoid cancer, carcinoma, cervical cancer, colon cancer, endometrial cancer, esophageal cancer, extrahepatic bile duct cancer, extracranial germ cell cancer, eye cancer, gallbladder cancer, gastric cancer, germ cell tumor, gestational trophoblastic tumor, head and neck cancer, hypopharyngeal cancer, islet cell carcinoma, kidney cancer, large intestine cancer, laryngeal cancer, leukemia, lip and oral cavity cancer, liver cancer, lung cancer, lymphoma, malignant mesothelioma, Merkel cell carcinoma, mycosis fungoides, myelodysplastic syndrome, myeloproliferative disorders, nasopharyngeal cancer, neuroblastoma, oral cancer, oropharyngeal cancer, osteosarcoma, ovarian epithelial cancer, ovarian germ cell cancer, pancreatic cancer, paranasal sinus and nasal cavity cancer, parathyroid cancer, penile cancer, pituitary cancer, plasma cell neoplasm, prostate cancer, rhabdomyosarcoma, rectal cancer, renal cell cancer, transitional cell cancer of the renal pelvis and ureter, salivary gland cancer, Sezary syndrome, skin cancers, small intestine cancer, soft tissue sarcoma, stomach cancer, testicular cancer, thymoma, thyroid cancer, urethral cancer, uterine cancer, vaginal cancer, vulvar cancer, and Wilms' tumor, or wherein the cancer disease is selected from the group consisting of acute lymphoblastic leukemia (ALL) (including non T cell ALL), acute lymphoid leukemia (ALL), and hemophagocytic lymphohistocytosis (HLH)), B cell prolymphocytic leukemia, B-cell acute lymphoid leukemia ("BALL"), blastic plasmacytoid dendritic cell neoplasm, Burkitt's lymphoma, chronic lymphocytic leukemia (CLL), chronic myelogenous leukemia (CML), chronic myeloid leukemia (CML), chronic or acute granulomatous disease, chronic or acute leukemia, diffuse large B cell lymphoma, diffuse large B cell lymphoma (DLBCL), follicular lymphoma, follicular lymphoma (FL), hairy cell leukemia, hemophagocytic syndrome (Macrophage Activating Syndrome (MAS), Hodgkin's Disease, large cell granuloma, leukocyte adhesion deficiency, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome (MDS), myeloid diseases including but not limited to acute myeloid leukemia (AML), non- Hodgkin's lymphoma (NHL), plasma cell proliferative disorders (e.g., asymptomatic myeloma (smoldering multiple myeloma or indolent myeloma), plasmablastic lymphoma, plasmacytoid dendritic cell neoplasm, plasmacytomas (e.g., plasma cell dyscrasia; solitary myeloma; solitary plasmacytoma; extramedullary plasmacytoma; and multiple plasmacytoma), POEMS syndrome (Crow-Fukase syndrome; Takatsuki disease; PEP syndrome), primary mediastinal large B cell lymphoma (PMBCL), small cell- or a large cell- follicular lymphoma, splenic marginal zone lymphoma (SMZL), systemic amyloid light chain amyloidosis, T cell acute lymphoid leukemia (TALL), T cell lymphoma, transformed follicular lymphoma, Waldenstrom macroglobulinemia, or a combination thereof.

16. The MAPK-ERK pathway inhibitor for use according to claim 14, wherein the genetic disease is selected from the group consisting of achondroplasia, achromatopsia, acute intermittent porphyria, adenosine deaminase deficiency, adenylosuccinate lyase deficiency, Adrenoleukodystrophy, Alagille syndrome, Alexander disease, alpha-1 antitrypsin deficiency, Alstrom syndrome, Alzheimer's disease, Amelogenesis imperfecta, amyotrophic lateral sclerosis, analbuminaemia, atherosclerosis, Barth syndrome, Batten disease, Becker muscular dystrophies, biotinidase deficiency, cataract, chronic granulomatous disease (CGD), Citrullinemia Type 1, cystic fibrosis, diabetes, Di George's syndrome, Duchenne Muscular Dystrophy, epilepsy, Epstein-Barr virus associated malignancies, Fabry's disease, familial hypercholesterolemia, fanconi anemia, glycogen storage disease (GSD), G6PD deficiency, hemophilia A, hemophilia B, hereditary angioedema (HAE), hereditary tyrosinemia, 21-hydroxylase deficiency HIV-1, homocystinuria, Huntington's disease, Hurler's disease, late infantile neuronal ceroid lipofuscinosis type 2 (CLN2), Leber Congenital Amaurosis (LCA), lecithin-cholesterol acyltransferase (LCAT) deficiency, lipoprotein lipase deficiency, lysosomal storage disease, in particular aspartylglucosaminuria, Tay-Sachs disease, I-cell disease, Sphingolipidoses such as Krabbe disease, Gaucher, Niemann-Pick disease, Metachromatic leukodystrophy, Lysosomal acid lipase deficiency, Multiple sulfatase deficiency, mucolipidosis types I (sialidosis type I and II), II (I-cell disease), III (pseudo-Hurler disease) and IV, Galactosialidosis, mucopolysaccharide storage diseases (Hurler disease and variants, Hunter, Sanfilippo Types A,B,C,D, Morquio Types A and B, Maroteaux-Lamy and Sly diseases), Niemann-Pick disease, SAP deficiency, and Salla disease; Menkes disease, metachromatic leukodystrophy, muscular dystrophy, Neuronal Ceroid Lipofuscinosis Type 2 (CLN2), ophthalmic or ocular disease, in particular Primary Open-Angle Glaucoma (POAG), OTC deficiency (OTCD), retinal degenerative diseases; Parkinson's disease, phenylketonuria, polycystic kidney disease, polycythaemia vera, Pompe disease, Sandhoff disease (GM2-gangliosidosis type II), Schindler disease types I and II, severe combined immunodeficiency (SCID), Sickle cell disease (SCD), Siderius X-linked mental retardation syndrome caused by mutations in the PHF8 gene, Spinal muscular atrophy (SMA), Tay-Sachs disease (GM2-gangliosidosis type I), thalassemia, Usher Syndrome, X-linked adrenoleukodystrophy (X-ALD), X-linked severe combined immunodeficiency (X-SCID), or X-linked sideroblastic anemia (XLSA), and Wilson's disease.
